# EUROPEAN PATENT APPLICATION

(11) **EP 4 752 139 A1**
(43) Date of publication of application: **03.06.2026**
(21) Application number: 24845610.5
(22) Date of filing: 23.07.2024
(51) Int. Cl.: C07D 317/22, C07D 319/06, C07D 407/12, C07D 407/14, C07D 493/08, C07D 519/00

(54) **BRANCHED MULTI-HYDROXYL-PROTECTING OLIGOMER, LINKED BODY BONDED THEREWITH, AND BRANCHED MULTI-HYDROXYL OLIGOMER OBTAINED BY DEPROTECTING SAID LINKED BODY**

(30) Priority: 26.07.2023 JP 2023121964
(71) Applicant: Tomoike Bio Ltd., Shatin, N.T., 999077 (HK)
(72) Inventor: MANDAI, Tadakatsu, Kurashiki-shi, Okayama 712-8505 (JP); SENO, Yuki, Kurashiki-shi, Okayama 712-8505 (JP)
(74) Representative: Hasegawa, Kan
(86) International application number: PCT/JP2024/026267
(87) International publication number: WO 2025/023235

(57) **Abstract**

There is provided at low cost a branched multi-hydroxyl-protected oligomer represented by Formula (1):

X - [Y^{a}] ₘ - [Y^{b}] _{m'} - [Y^{c}] ₙ - [Y^{d}] _{n'} (1)

wherein X is a reactive group, Y^{a} to Y^{d} are at least one selected from the group consisting of P, T, G, and A represented by Formula (3); m is 1; m' is an integer of 1 to 3; n is an integer of 0 to 9; and n' is an integer of 0 to 27 (except when the compound represented by Formula (1) is X-G₁G₁, X-G₁G₂, X-G₁A₁, or X-G₁A₂), which can be synthesized with a good yield via few reaction steps by means of a simple method, and is extremely effective in improving water solubility of a target compound.

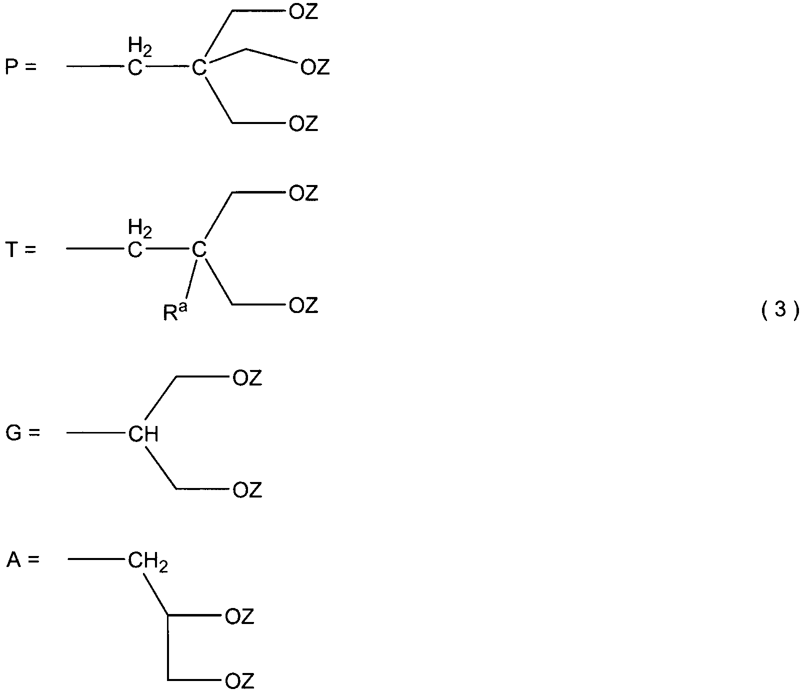

## Description

### Technical Field

The present invention relates to a branched multi-hydroxyl-protected oligomer and a linked body bonded therewith, and a branched multi-hydroxyl oligomer made by deprotecting the linked body.

### Background Art

Some polyphenols such as pterostilbene and resveratrol are known to have antioxidant and anti-inflammatory properties, as well as to improve skin elasticity. They are, however, hardly water-soluble, and thus have problems such as being not mixed evenly and precipitating over time. Glycosylation, in which a sugar component is chemically linked to an aglycone, is known as a method for improving the water solubility of a hardly water-soluble compound. However, this requires combining multiple monosaccharides to achieve sufficient water solubility, and the hydroxyl groups of the monosaccharides must also be protected, sometimes leading to a higher cost.

Another known method for improving water solubility is multihydroxylation, which involves introducing multiple hydroxyl groups. Examples of multihydroxylation include polyethylene glycolation (PEG), polyglycerolation (PGL), and branched oligomerization (BGL). In the light of raw-material availability and cost, these multihydroxylations offer significant advantages over glycosylation. As shown in the formula below, there is only one type of PEG, but linear and semi-linear PGLs are known, and there are multiple types of BGLs depending on the number of branches. 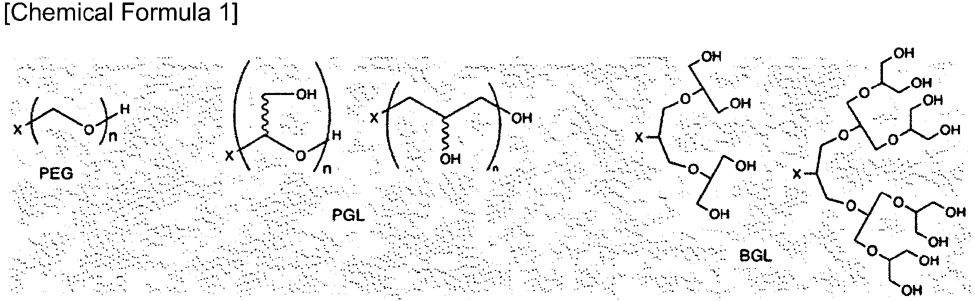 wherein X is a general representation of a functional group linked to a hardly water-soluble target, and n is an integer of 1 or more.

PEG, especially monomers and dimers, is highly toxic, and only oligomers which exceed several dozen in number can contribute to sufficient water solubility. PGL contains numerous uncontrollable asymmetric carbon atoms, making it difficult to maintain reproducibility in compound production, homogeneity, and quality assurance. Furthermore, because both PEG and PGL are produced by polymerization reactions, molecular weight variation is also problematic. Furthermore, glycosylation often results in uncontrollable stereochemistry at the sugar linkage (anomeric position), giving a mixture. In contrast, BGL does not contain any asymmetric carbon atom, and a production method that allows molecular weight control has been established. Furthermore, it is believed that BGL production is highly reproducible, enabling production of quality-assured derivatives. Drawbacks of BGL, however, has drawbacks that include a process for selectively producing 1- and 3- protected glycerol in the first stage of production is laborious (Patent Reference No. 1), and that multiple steps are required for producing oligomers exceeding heptamer, result in a low yield (Non-patent Reference No. 1).

### Citation List

### Patent Literature

Patent Literature No. 1: WO 2020/255741 A1

### Non-patent Literature

Non-patent Literature No. 1: Hisao Nemoto et al., Bioorg. Med. Chem. Lett. 2011, 21, 4724-4727.

### Summary of Invention

### Technical Problem

To solve the above problems, an objective of the present invention is to provide at low cost a branched multi-hydroxyl-protected oligomer, which can be synthesized with a good yield via few reaction steps by means of a simple method, and is extremely effective in improving water solubility of a target compound.

### Solution to Problem

The above problems are resolved by providing a branched multi-hydroxyl-protected oligomer represented by Formula (1):
[Chemical Formula 2]

X-[Y^{a}]ₘ-[Y^{b}]_{m'}-[Y^{c}]n-[Y^{d}]_{n'} (1)

wherein X is at least one selected from the group consisting of groups represented by Formula (2); Y^{a} to Y^{d} are at least one selected from the group consisting of P, T, G, and A represented by Formula (3); m is 1; m' is an integer of 1 to 3; n is an integer of 0 to 9; and n' is an integer of 0 to 27, except when the compound represented by Formula (1) is X-G₁G₁, X-G₁G₂, X-G₁A₁, or X-G₁A₂; 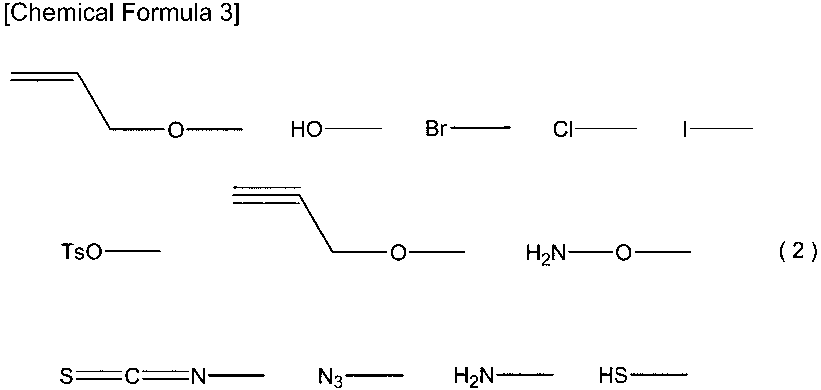 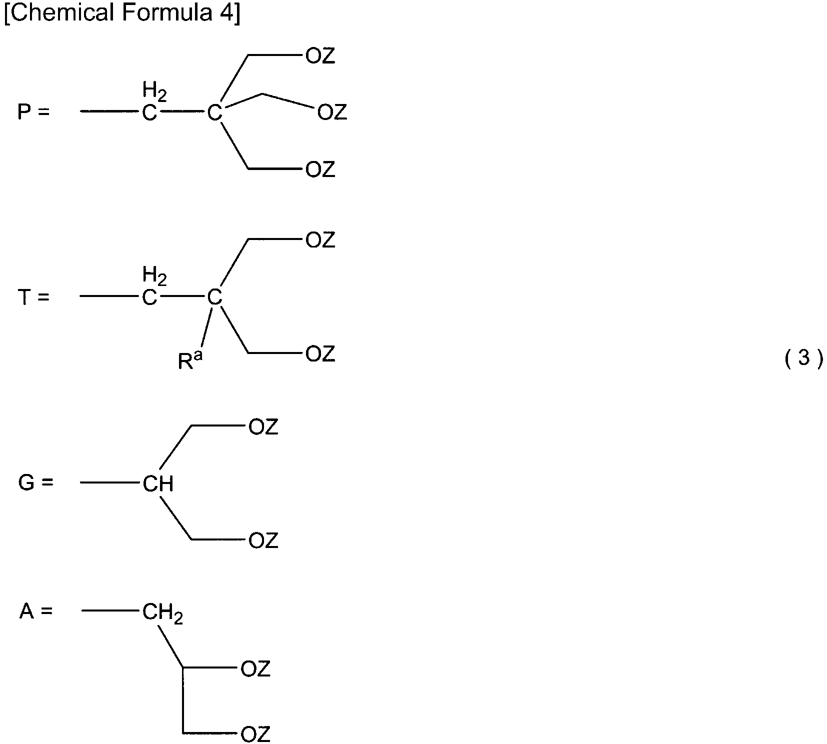
wherein Z is at least one selected from the group consisting of a hydrogen atom, a protecting group and a carbon skeleton, and R^{a} is at least one selected from the group consisting of a hydrogen atom and an alkyl group.

A preferable embodiment is a branched multi-hydroxyl-protected oligomer represented by Formula (1a): [0014] 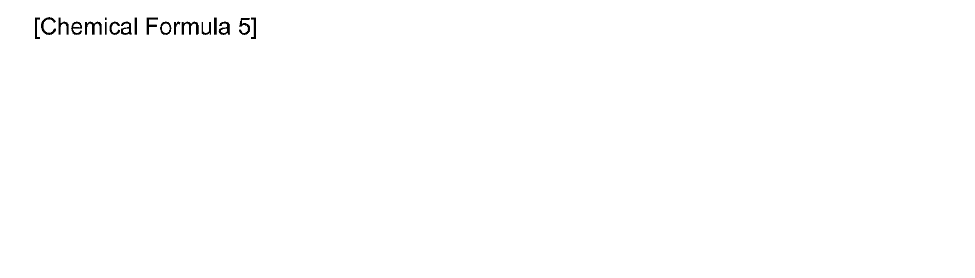 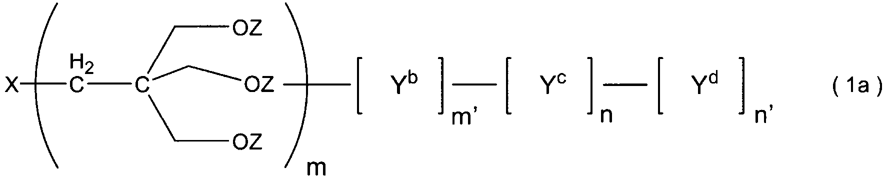
wherein X is at least one selected from the group consisting of groups represented by Formula (2); Y^{b} to Y^{d} are at least one selected from the group consisting of P, T, G, and A represented by Formula (3); Z is at least one selected from the group consisting of a hydrogen atom, a protecting group and a carbon skeleton; m is 1; m' is an integer of 1 to 3; n is an integer of 0 to 9; n' is an integer of 0 to 27; 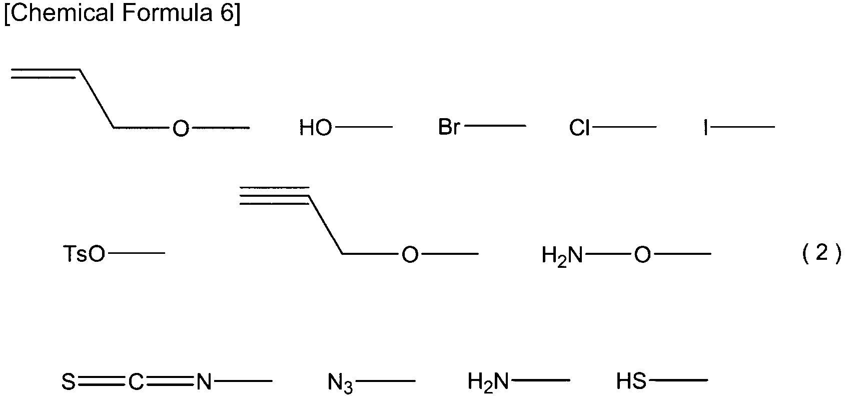 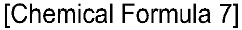 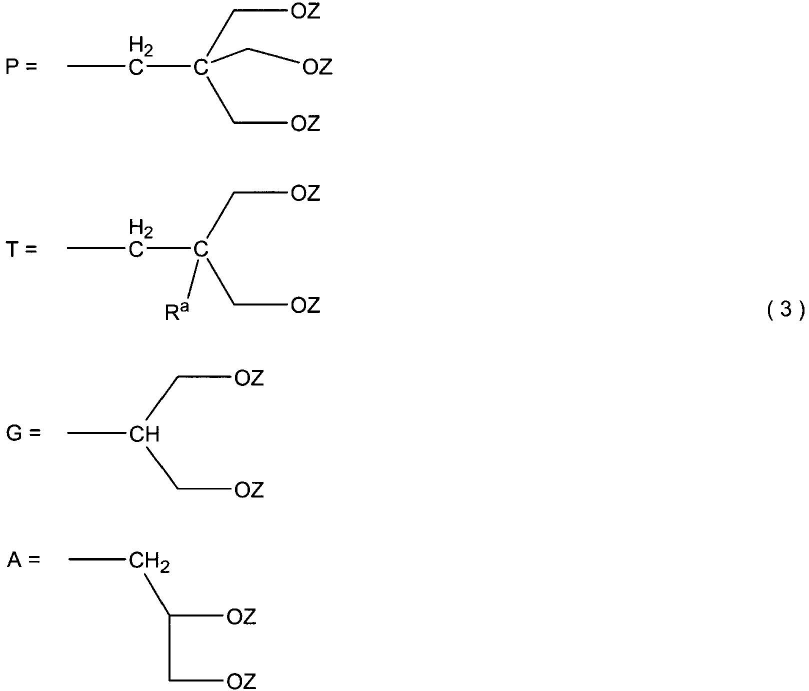
wherein Z is at least one selected from the group consisting of a hydrogen atom, a protecting group and a carbon skeleton; and R^{a} is at least one selected from the group consisting of a hydrogen atom and an alkyl group.

Another preferable embodiment is a branched multi-hydroxyl-protected oligomer represented by Formula (1c): 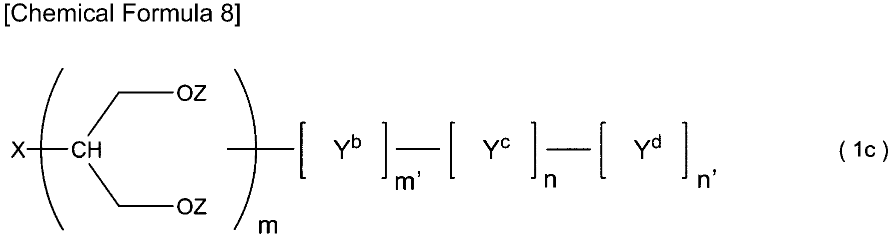
wherein X is at least one selected from the group consisting of groups represented by Formula (2); Y^{b} to Y^{d} are at least one selected from the group consisting of P, T, G, and A represented by Formula (3); Z is at least one selected from the group consisting of a hydrogen atom, a protecting group and a carbon skeleton; m is 1; m' is an integer of 1 to 3; n is an integer of 0 to 9,; n' is an integer of 0 to 27, except when the compound represented by Formula (1c) is X-G₁G₁, X-G₁G₂, X-G₁A₁ or X-G₁A₂; 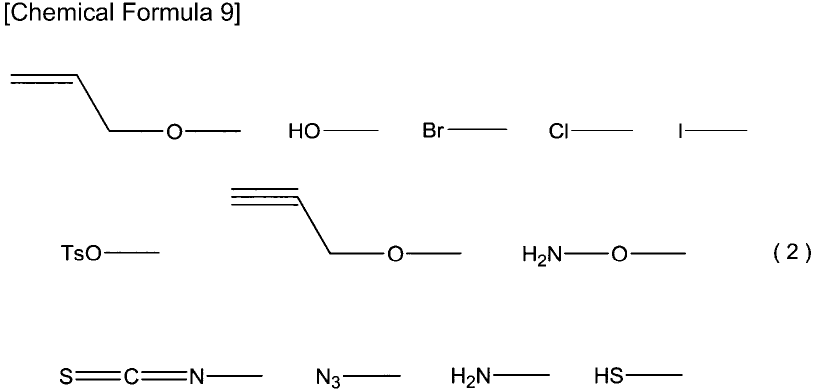 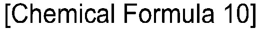 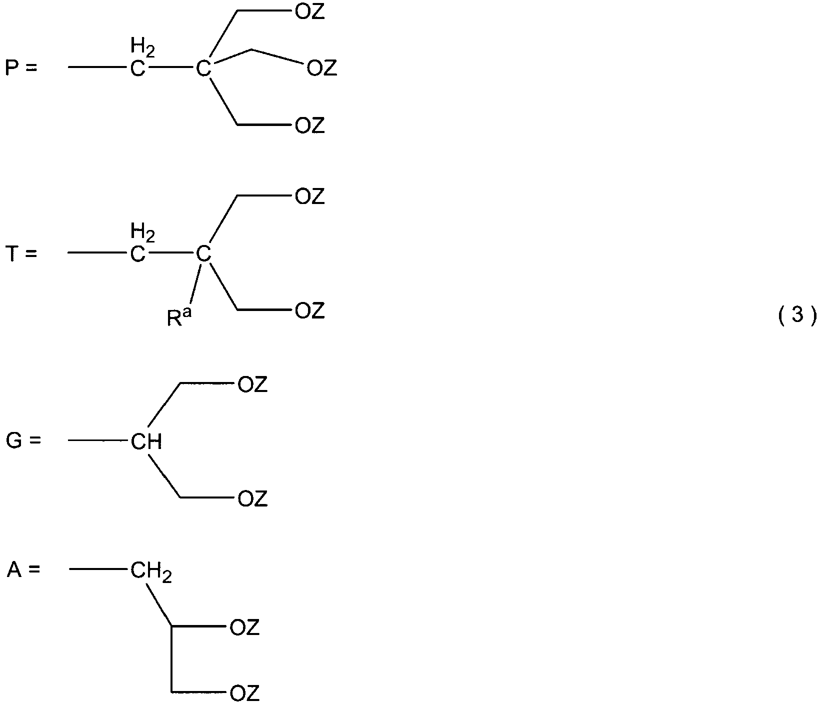
wherein Z is at least one selected from the group consisting of a hydrogen atom, a protecting group and a carbon skeleton; and R^{a} is at least one selected from the group consisting of a hydrogen atom and an alkyl group.

Here, a preferable embodiment is a linked body made by bonding the branched multi-hydroxyl-protected oligomer with a target compound. It is preferable that the branched multi-hydroxyl-protected oligomer is deprotected, and that the target compound is a hardly water-soluble compound. A preferable embodiment is a water-solubility improver made of the branched multi-hydroxyl-protected oligomer.

### Advantageous Effects of Invention

In accordance with the present invention, there can be provided at low cost a branched multi-hydroxyl-protected oligomer, which can be synthesized with a good yield via few reaction steps by means of a simple method, and is extremely effective in improving water solubility of a target compound.

### Brief Description of Drawings

FIG. 1 shows water-solubility evaluation results of pterostilbene, a pterostilbene glycoside and a deprotected pterostilbene linked body.
FIG. 2 shows water-solubility evaluation results of a deprotected pterostilbene linked body.

### Description of Embodiments

A branched multi-hydroxyl-protected oligomer of the present invention is represented by Formula (1):
[Chemical Formula 11]

X-[Y^{a}]ₘ-[Y^{b}]_{m'}-[Y^{c}]ₙ-[Y^{d}]_{n'} (1)

wherein X is at least one selected from the group consisting of groups represented by Formula (2); Y^{a} to Y^{d} are at least one selected from the group consisting of P, T, G, and A represented by Formula (3); m is 1; m' is an integer of 1 to 3; n is an integer of 0 to 9; and n' is an integer of 0 to 27, except when the compound represented by Formula (1) is X-G₁G₁, X-G₁G₂, X-G₁A₁, or X-G₁A₂; 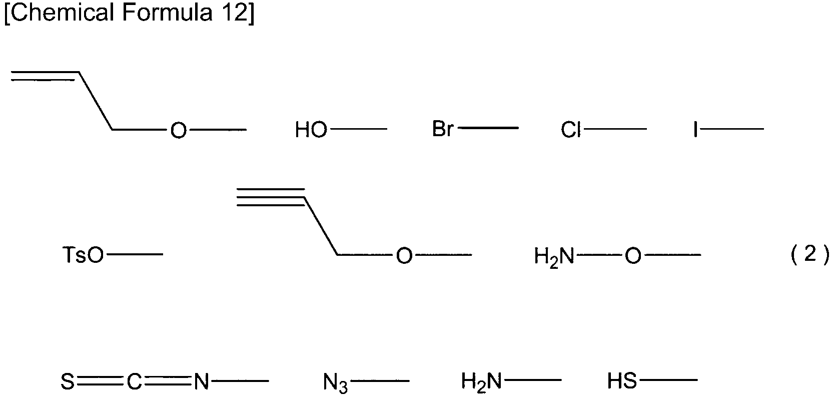 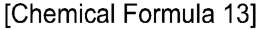 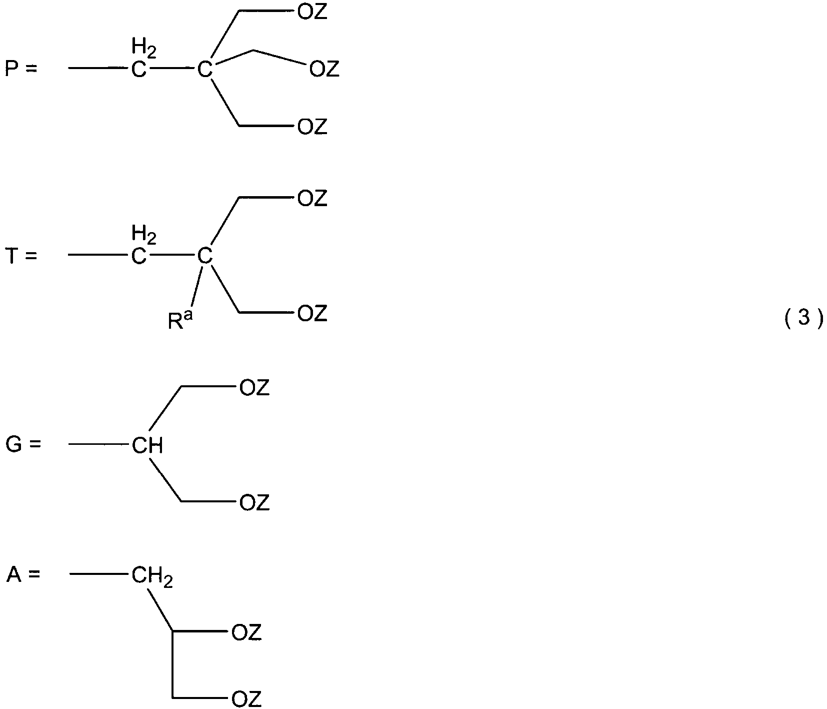
wherein Z is at least one selected from the group consisting of a hydrogen atom, a protecting group and a carbon skeleton, and R^{a} is at least one selected from the group consisting of a hydrogen atom and an alkyl group.

The branched multi-hydroxyl-protected oligomer of the present invention can synthesized with a good yield via few reaction steps by means of a simple method, and thus can be provided at a low cost. Furthermore, as is clear from the water-solubility evaluation results in Examples described below, the branched multi-hydroxyl-protected oligomer of the present invention has the excellent advantage of being extremely effective in improving water solubility of a target compound, which is hardly water-soluble. In contrast, glycosylation may be costly because it is necessary to combine multiple monosaccharides to achieve sufficient water solubility and to protect the hydroxyl groups of these monosaccharides. Furthermore, for a branched oligomer (BGL), the first step in production requires a laborious process to selectively produce glycerin protected at the 1,3-positions, which can be costly. Therefore, it is clear that employing the present invention is very significant.

In Formula (1), X is at least one selected from the group consisting of groups represented by Formula (2), which is a reactive group used for bonding to a target compound. A substituent of the target compound suitably reacts with X, to give a linked body wherein the branched multi-hydroxyl-protected oligomer represented by Formula (1) is bonded to the target compound.

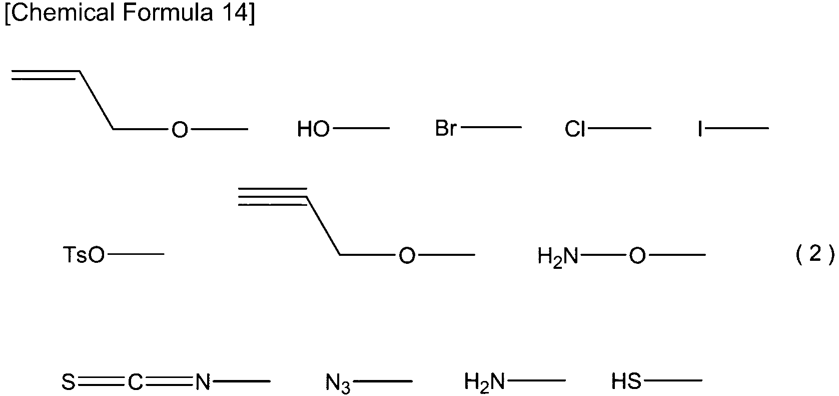

In particular, in the light of good reactivity with the target compound, X is preferably at least one selected from the group consisting of groups represented by Formula (2a):

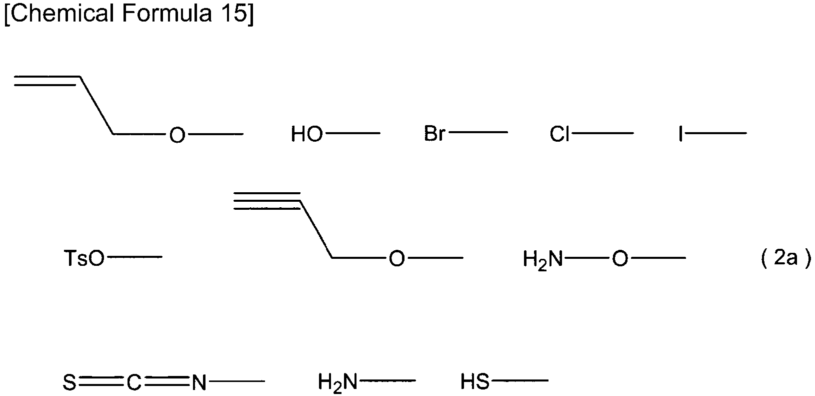

In Formula (1), Y^{a} to Y^{d} are at least one selected from the group consisting of P, T, G, and A represented by Formula (3); m is 1; m' is an integer of 1 to 3; n is an integer of 0 to 9; n' is an integer of 0 to 27. 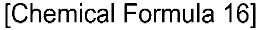 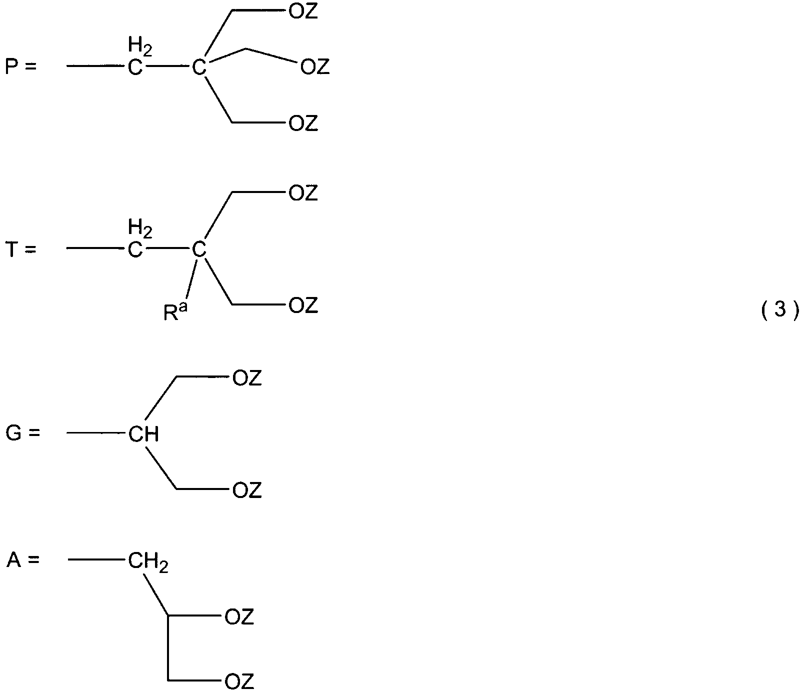 wherein Z is at least one selected from the group consisting of a hydrogen atom, a protecting group and a carbon skeleton; and R^{a} is at least one selected from the group consisting of a hydrogen atom and an alkyl group.

Y^{a} to Y^{d} in Formula (1) is at least one selected from the group consisting of P, T, G, and A represented by Formula (3), and Y^{a} to Y^{d} can be the same or different. Each of Y^{a} to Y^{d} is linked to each other via Z in Formula (3), and Z used for this linkage is a carbon skeleton. That is, Z in -OZ is a carbon skeleton, and each of Y^{a} to Y^{d} is each linked to each other via an ether bond (-O-). The carbon skeleton is preferably a covalent bond, and can be a divalent hydrocarbon group.

In Formula (1), m is 1 and m' is an integer from 1 to 3. Y^{b} can be bonded to the same number of Zs as the number of Zs in Y^{a}, which is at least one selected from the group consisting of P, T, G, and A represented by Formula (3). For example, Y^{b} can be bonded to all of Zs in Y^{a}, or Y^{b} can be bonded to one or more Zs in Y^{a}, with the remaining Z of Y^{a} being at least one selected from the group consisting of a hydrogen atom and a protecting group. When Y^{a} is P, there are three Zs, so the maximum number of m' is 3. In addition, in Formula (1), n is an integer of 0 to 9, and n' is an integer of 0 to 27. Y^{c} can be bonded to the same number of Zs of Y^{b}, which is at least one selected from the group consisting of P, T, G, and A represented by Formula (3), and Y^{d} can be bonded to the same number of Zs of Y^{c}, which is at least one selected from the group consisting of P, T, G, and A represented by Formula (3). For example, Y^{c} can be bonded to all of Zs in Y^{b}, or Y^{d} can be bonded to all of Zs in Y^{c}. Y^{c} can be bonded to one or more Zs in Y^{b}, with the remaining Z in Y^{b} being at least one selected from the group consisting of a hydrogen atom and a protecting group, or Y^{d} can be bonded to one or more Zs in Y^{c}, with the remaining Z in Y^{c} being at least one selected from the group consisting of a hydrogen atom and a protecting group. When m' is 3 and all Y^{b} are P, there are 9 Zs, so the maximum number of n is 9. Similarly, when n is 9 and all Y^{c} are P, there are 27 Zs, so the maximum number of n' is 27. Here, when both n and n' are 0, neither Y^{c} nor Y^{d} is present in the resulting structure.

In Formula (3), Z is at least one selected from the group consisting of a hydrogen atom, a protecting group and a carbon skeleton. As described above, each of Y^{a} to Y^{d} is linked with each other via Z. Z not used in the linkage is preferably at least one selected from the group consisting of a hydrogen atom and a protecting group. From the viewpoint of deprotecting the linked body after obtaining the linked body bound to the target compound, it is a preferred embodiment that the Z not used in the linkage is a protecting group. In particular, it is a preferred embodiment that the terminal of the branched multi-hydroxyl-protected oligomer represented by Formula (1) is a protecting group. There are no particular restrictions to the protecting group as long as it is a substituent that protects a hydroxyl group. Examples include an acyl group, an alkoxymethyl group, an alkoxycarbonyl group, an alkenyl group, an alkylsilyl group, an arylmethyl group, a tetrahydropyranyl group, and a group in which multiple Zs form a cyclic structure. Furthermore, in Formula (3), R^{a} is at least one selected from the group consisting of a hydrogen atom and an alkyl group. The alkyl group is a linear or branched alkyl group having 1 to 10 carbon atoms; examples include methyl, ethyl, n- propyl, isopropyl, n-butyl, isobutyl, sec-butyl, tert-butyl, n-pentyl, isopentyl, neopentyl, tert-pentyl, n-hexyl, isohexyl, 2-ethylhexyl, n-heptyl, n-octyl, n-nonyl, and n-decyl groups. Among these, preferred alkyl groups are methyl and ethyl.

Examples of the acyl group include a linear or branched alkylcarbonyl group having 2 to 10 carbon atoms and an arylcarbonyl group. There are no particular restrictions to the linear or branched alkylcarbonyl group having 2 to 10 carbon atoms as long as it has a structure in which a carbonyl group is bonded to any carbon atom of an alkyl group. Examples of the alkyl group include a linear or branched alkyl group having 1 to 10 carbon atoms, such as a methyl group, an ethyl group, an n-propyl group, an isopropyl group, an n-butyl group, an isobutyl group, a sec-butyl group, a tert-butyl group, an n-pentyl group, an isopentyl group, a neopentyl group, a tert-pentyl group, an n-hexyl group, an isohexyl group, a 2-ethylhexyl group, an n-heptyl group, an n-octyl group, an n-nonyl group and n-decyl group. Specific examples of acyl groups include an acetyl group, a propionyl group, a butyryl group, an isobutyryl group, a benzoyl group, a dodecanoyl group, and a crotonoyl group. Among these, a preferred acyl group is an acetyl group.

There are no particular restrictions to the alkoxymethyl group as long as it has a structure in which an alkoxy group having 1 to 7 carbon atoms is bonded to a methyl group; examples include a methoxymethyl group, an ethoxymethyl group, and a benzyloxymethyl group.

Examples of the alkoxycarbonyl group include linear, branched, and cyclic alkoxycarbonyl groups having 2 to 10 carbon atoms, such as a methoxycarbonyl group, an ethoxycarbonyl group, a 2,2,2-trichloroethoxycarbonyl group, an n-propoxycarbonyl group, an isopropoxycarbonyl group, an allyloxycarbonyl group, an n-butoxycarbonyl group, an isobutoxycarbonyl group, a sec-butoxycarbonyl group, a tert-butoxycarbonyl group, a pentyloxycarbonyl group, a hexyloxycarbonyl group, a heptyloxycarbonyl group, an octyloxycarbonyl group, and a benzyloxycarbonyl group.

The alkenyl group can be a linear or branched alkenyl group having 2 to 6 carbon atoms, such as a vinyl group, an allyl group, a methylvinyl group, a propenyl group, a butenyl group, a pentenyl group, or a hexenyl group.

Examples of the alkylsilyl group include a trimethylsilyl group, a triethylsilyl group, a triisopropylsilyl group, a tert-butyldimethylsilyl group, and a tert-butyldiphenylsilyl group.

There are no particular restrictions to the arylmethyl group as long as it has a structure in which an aryl group is bonded to a methyl group; examples include a benzyl group and a 4-methoxybenzyl group.

When the group in which multiple Zs form a cyclic structure as P, T, G, and A represented by Formula (3) above, structures represented by the following formulas (3a) to (3d), respectively, are preferably employed.

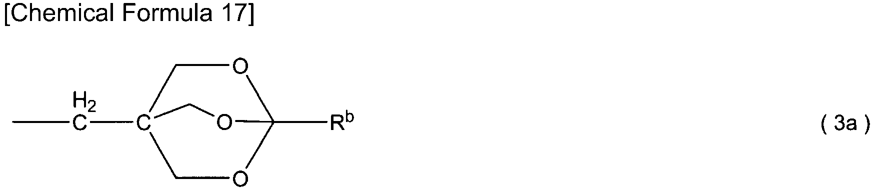

In Formula (3a), R ^{b} is an alkyl group. 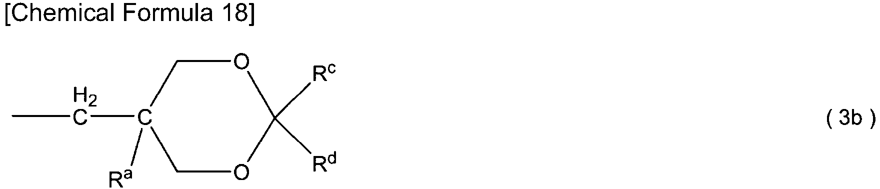 In Formula (3b), R^{a}, R^{c}, and R^{d} are at least one selected from the group consisting of a hydrogen atom and an alkyl group.

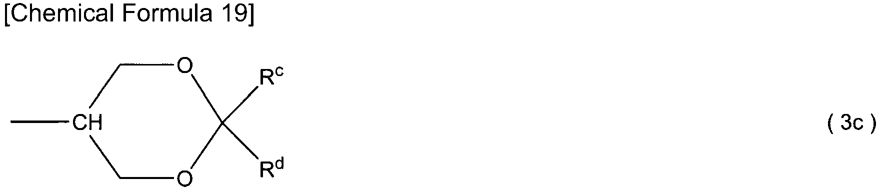

In Formula (3c), R^{c} and R^{d} are at least one selected from the group consisting of a hydrogen atom and an alkyl group.

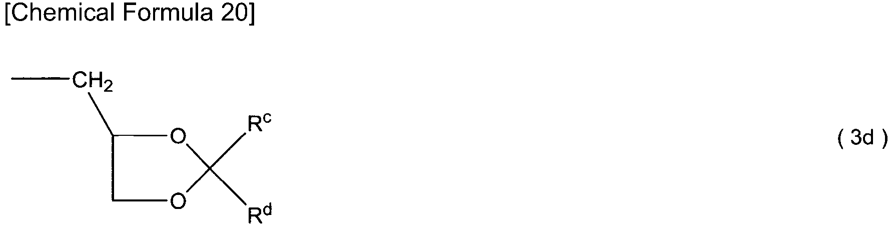

In Formula (3d), R^{c} and R^{d} are at least one selected from the group consisting of a hydrogen atom and an alkyl group.

In Formula (3a), R^{b} is an alkyl group, and the alkyl groups described for R^{a} in Formula (3) can be used as R^{b}. In Formula (3b), R^{a}, R^{c}, and R^{d} are at least one selected from the group consisting of a hydrogen atom and an alkyl group. R^{a} in Formula (3b) is the same substituent as R^{a} in Formula (3), and the alkyl groups described for R^{a} in Formula (3) can be used as well. R^{c} and R^{d} in Formulas (3b), (3c), and (3d) are at least one selected from the group consisting of a hydrogen atom and an alkyl group. In a preferred embodiment, both R^{c} and R^{d} are alkyl groups, and it is also preferred that one is a hydrogen atom and the other is an alkyl group. The alkyl groups used for R^{c} and R^{d} can be the alkyl groups described for R^{a} in Formula (3).

The above-mentioned protecting groups can further have a substituent, and such substituents include halogen atoms such as fluorine, chlorine, bromine, and iodine; alkyl groups such as methyl, ethyl, and n-propyl; alkenyl groups such as vinyl, allyl, methylvinyl, and propenyl; alkynyl groups such as ethynyl, propynyl, and propargyl; aryl groups such as phenyl and naphthyl; alkoxy groups such as methoxy, ethoxy, propoxy, isopropoxy, and butoxy; alkylthio groups such as methylthio, ethylthio, propylthio, and butylthio; arylthio groups such as phenylthio and naphthylthio; acyloxy groups such as acetoxy, propanoyloxy, butanoyloxy, pivaloyloxy, and benzoyloxy; heteroaromatic groups such as pyridyl, thienyl, furyl, imidazolyl, and oxazolyl; an amino group; a hydroxyl group; a cyano group; and a nitro group.

Since m in Formula (1) is 1, the branched multi-hydroxyl-protected oligomer of the present invention is any of the compounds represented by Formulas (1a) to (1d), where Y^{a} is P, T, G, or A. Among these, the branched multi-hydroxyl-protected oligomer represented by Formula (1a) or (1c) below is preferred.

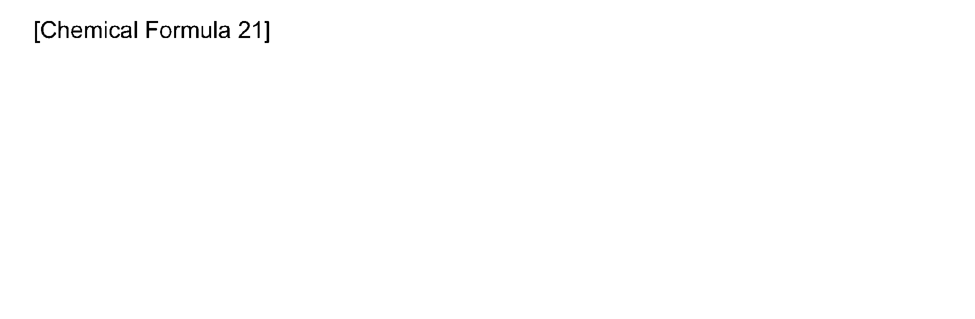

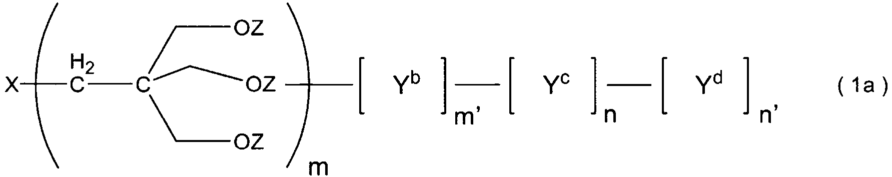

In Formula (1a), X, Y^{b} to Y^{d}, Z, m, m', n and n' are as defined in Formula (1).

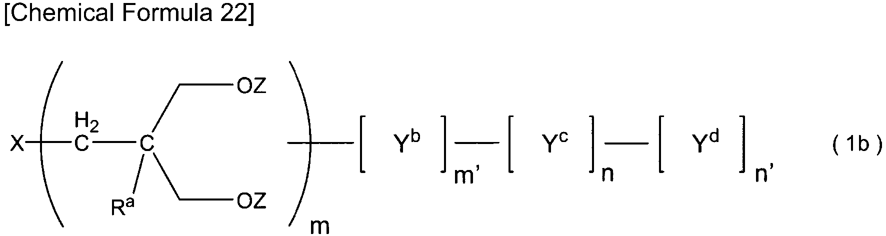

In Formula (1b), X, Y^{b} to Y^{d}, Z, m, m', n and n' are as defined in Formula (1), and R^{a} is as defined in Formula (3).

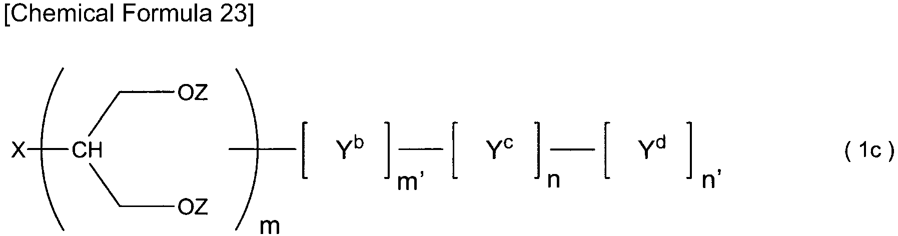

In Formula (1c), X, Y^{b} to Y^{d}, Z, m, m', n, and n' are as defined in Formula (1), except when the compounds represented by Formula (1c) are X-G₁G₁, X-G₁G₂, X-G₁A₁, and X-G₁A₂.

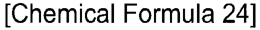

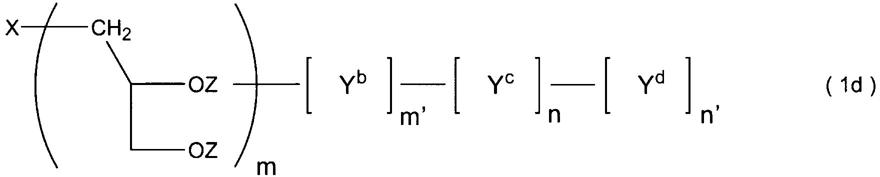

In Formula (1d), X, Y^{b} to Y^{d}, Z, m, m', n, and n' are defined as in Formula (1).

In Formula (1a), for example, a compound represented by Formula (1a') (X-P₁P₁, X-P₁T₁, X-P₁G₁, X-P₁A₁) in which m' is 1, n and n' are 0, and Y^{b} is P, T, G, or A represented by Formula (3) is one of the simplest structures for the branched multi-hydroxyl-protected oligomer of the present invention. However, the branched multi-hydroxyl-protected oligomer of the present invention is not limited to this structure.

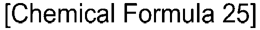

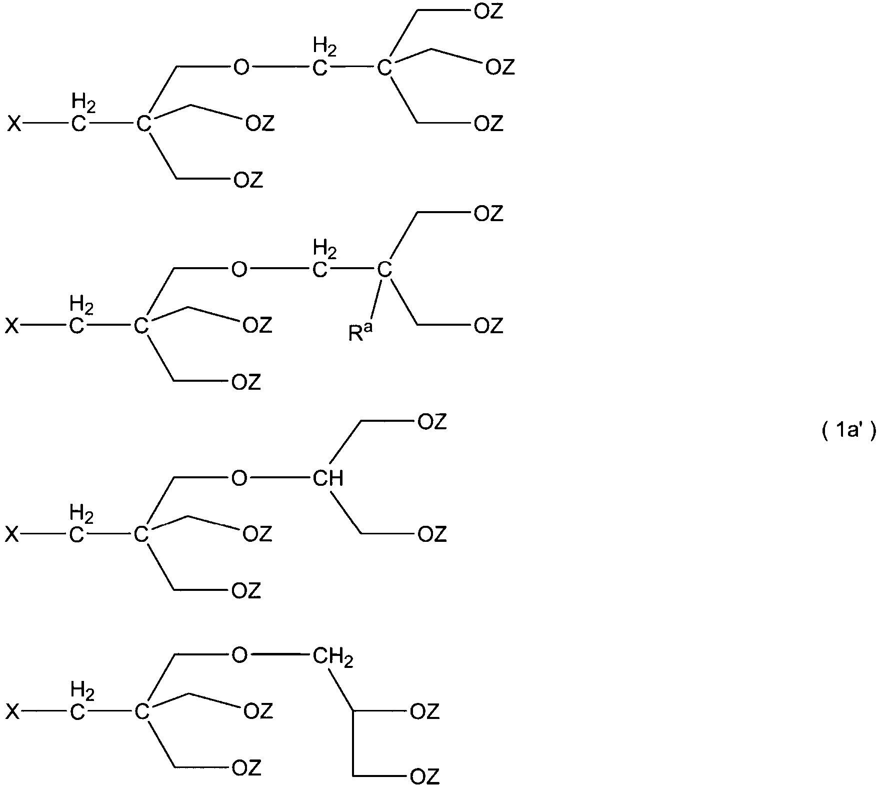

In Formula (1a'), X is as defined in Formula (1), and Z and R^{a} are as defined in Formula (3).

There are no particular restrictions to a method for producing a branched multi-hydroxyl-protected oligomer of the present invention. For example, as shown in the following reaction formula (I), it can be produced by an etherification reaction between a dibromide (when X^{a}=Br) and an alcohol R¹-OH represented by Formula (4). Alternatively, as shown in reaction formula (II), it can be produced by an etherification reaction between a tribromide (when X^{a} =Br) and an alcohol R¹-OH represented by Formula (4). It can also be produced by a linkage reaction between epichlorohydrin (when X^{b}=Cl) and an alcohol R¹-OH represented by Formula (4). These reactions are preferably carried out by well-known methods. The halogen atom in X^{a} is preferably bromine, chlorine, or iodine. The substituent X in the resulting branched multi-hydroxyl-protected oligomer is at least one selected from the group consisting of groups represented by Formula (2), and can be converted to a desired substituent X by appropriate procedures such as tosylation or deallylation.

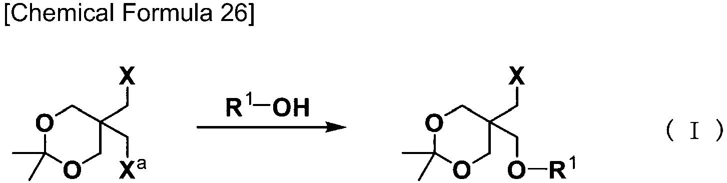

In Formula (I), X is as defined Formula (1), X^{a} is a halogen atom, and R¹ is a group obtained by removing -OH from at least one alcohol R¹-OH selected from the group consisting of Formula (4).

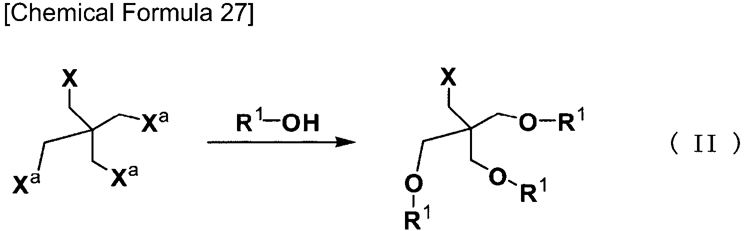

In Formula (II), X is as defined in Formula (1), X^{a} is a halogen atom, and R¹ is a group obtained by removing -OH from at least one alcohol R¹-OH selected from the group consisting of Formula (4).

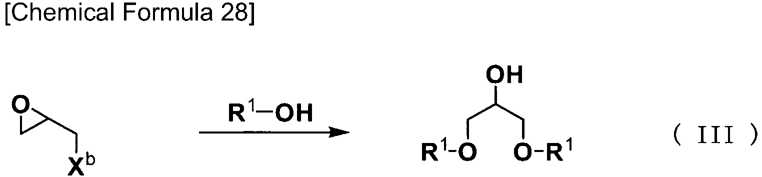

In Formula (III), X is as defined in Formula (1), X^{b} is a halogen atom, and R¹-OH is at least one selected from the group consisting of alcohols represented by Formula (4).

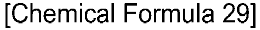

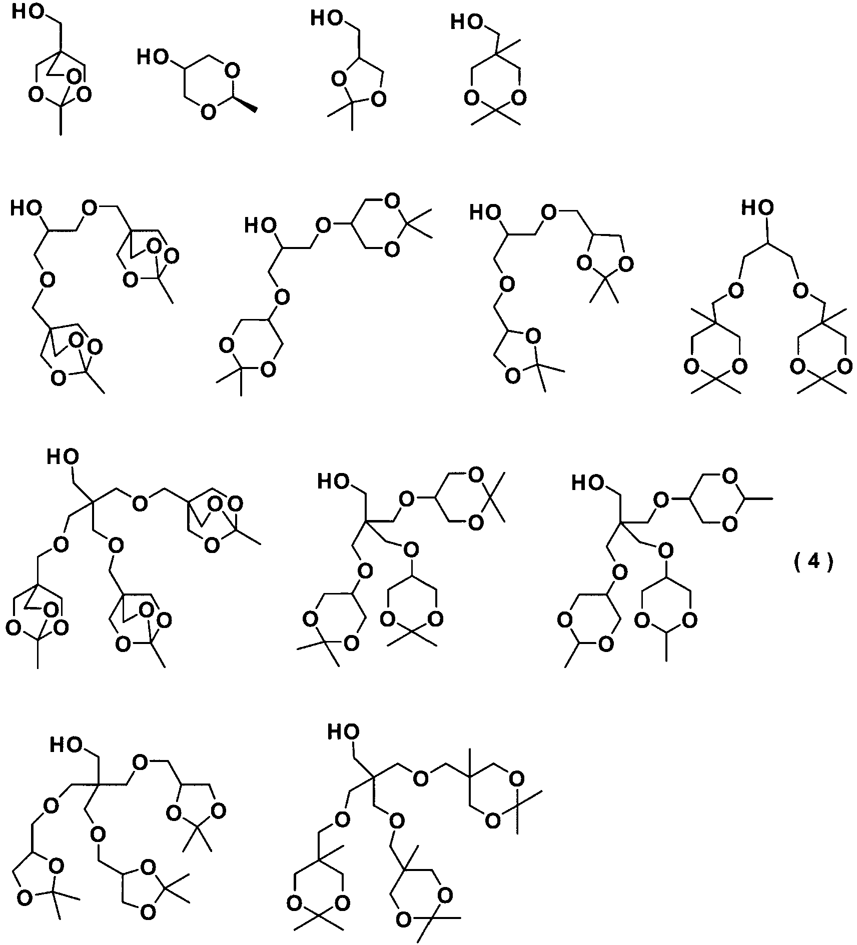

The resulting branched multi-hydroxyl-protected oligomer can be linked to a target compound. For example, when the substituent X in the branched multi-hydroxyl-protected oligomer represented by Formula (1) is -Br or -OTs, it can be reacted with the hydroxyl group of the target compound, pterostilbene, as shown in reaction formula (IV), to provide a linked body. In other words, a linked body formed by linking a branched multi-hydroxyl-protected oligomer to a target compound is a preferred embodiment. The target compound is preferably a hardly water-soluble compound, and preferred examples of such hardly water-soluble compounds include polyphenol compounds having hydroxyl groups, such as pterostilbene and resveratrol.

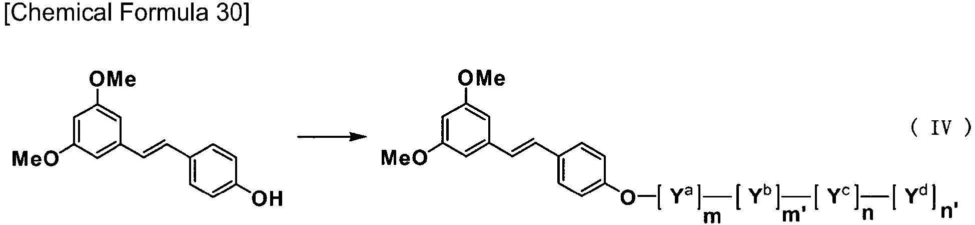

In Formula (IV), Y^{a}, Y^{b}, Y^{c}, Y^{d}, m, m', n and n' are as defined in Formula (1).

The branched multi-hydroxyl-protected oligomer used to bond to a target compound can be a deprotected branched multi-hydroxyl-protected oligomer. However, in the light of efficient reaction, it is preferred to obtain a linked body formed by bonding to the target compound and then to deprotect the linked body. Known methods can be used for deprotection. As shown in Examples described below, the protecting groups in the linked body are deprotected, resulting in generation of numerous hydroxyl groups. As is clear from a water solubility evaluation results in Examples described below, the branched multi-hydroxyl-protected oligomer of the present invention is highly effective in improving water solubility of a target compound, which is a hardly water-soluble compound. A water-solubility improver comprising a branched multi-hydroxyl-protected oligomer is, therefore, a preferred embodiment.

### Examples

The present invention will be more specifically explained below by the use of Examples below. The reagents and solvents used were commercially available and used as received without any purification process. The amounts of the organic solvents used in blending were all expressed in volume ratios. ¹H NMR and ¹³C NMR were measured using a JEOL JNN-ECS400 at 400 MHz and 100 MHz, respectively.

### [Raw material compound]

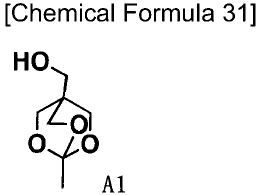

A1:(1-methyl-2,6,7-trioxabicyclo[2.2.2]octan-4-yl)methanol is a known compound , for which a production method is described in T. Jeffrey Dunn, William L. Neumann, Milorad M. Rogic, and Steven R. Woulfe J. Org. Chem., 1990, 55, 6368-6373.

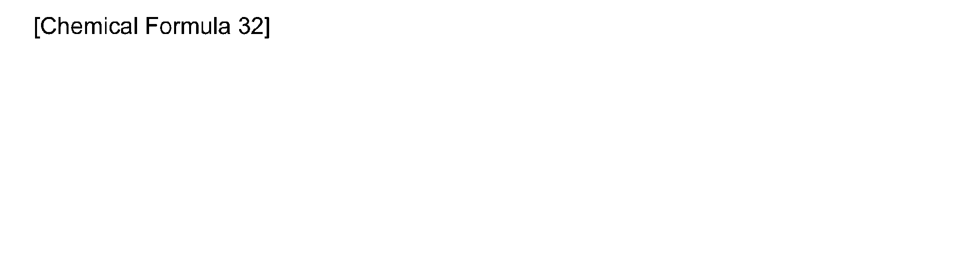

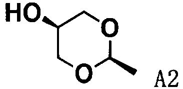

A2:(2s,5s)-2-methyl-1,3-dioxan-5-ol can be obtained a known method described in WO 2020/255741 A1.

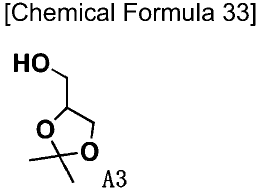

A3: (2,2-dimethyl-1,3-dioxolan-4-yl)methanol is available from TCI (Tokyo Chemical Industry Co., Ltd.).

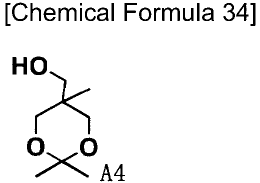

A4: (2,2,5-trimethyl-1,3-dioxan-5-yl)methanol is commercially available from Merck-Aldrich.

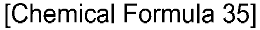

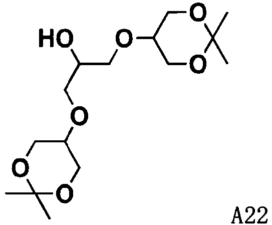

A22:1,3-bis((2,2-dimethyl-1,3-dioxan-5-yl)oxy)propan-2-ol can be synthesized by a known method described in Nemoto, Hisao; Kamiya, Masaki; Nakamoto, Aki; Katagiri, Ayato; Yoshitomi, Kohsuke; Kawamura, Tomoyuki; Hattori, Hatsuhiko. Chem. Lett. 2010, 39, 856-857.

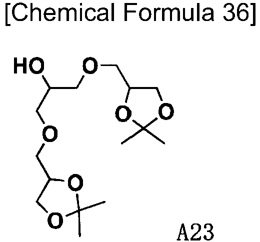

A23:1,3-bis((2,2-dimethyl-1,3-dioxolan-4-yl)methoxy)propan-2-ol is commercially available from Synnovator Product.

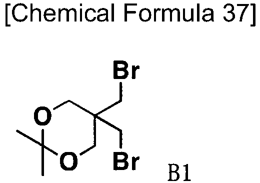

B1:5,5-bis(bromomethyl)-2,2-dimethyl-1,3-dioxane is commercially available from FUJIFILM Wako Pure Chemical Corporation.

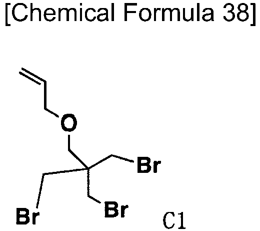

C1:3-(3-bromo-2,2-bis(bromomethyl)propoxy)prop-1-ene is commercially available from HONG KONG CHEMHERE CO LIMITED.

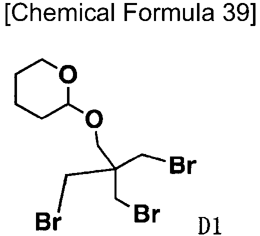

D1:2-(3-bromo-2,2-bis(bromomethyl)propoxy)tetrahydro-2H-pyran can be produced by a known method described in JP 2022-14977 A.

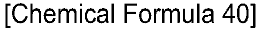

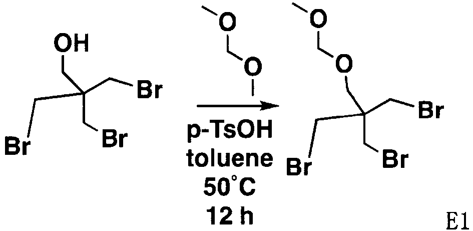

E1:1,3-Dibromo-2-(bromomethyl)-2-((methoxymethoxy)methyl)propane can be prepared in 80% yield by the equilibrium transacetalation of 3-bromo-2,2-bis(bromomethyl)propan-1-ol with 20 equivalents of dimethoxymethane for 12 hours under acid catalysis without solvent.
¹H NMR (CDCl₃, 400 MHz): δ 4.64 (s, 2H), 3.58 (s, 2H), 3.54 (s, 6H), 3.40 (s, 3H)
¹³C NMR (CDCl₃, 100 MHz): δ 97.0 (CH₂), 66.8(CH₂), 55.7(CH₃), 43.6, (C), 34.6 (CH₂ x 3)

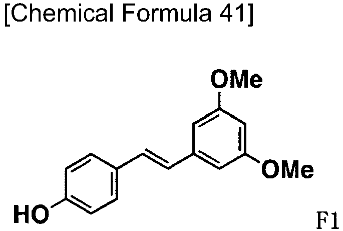

F1:(E)-4-(3,5-dimethoxystyryl)phenol (pterostilbene) is commercially available from TCI (Tokyo Chemical Industry Co., Ltd.).

### Synthesis Example 1

### [Etherification reaction of dibromide B1 with alcohol R¹ -OH]

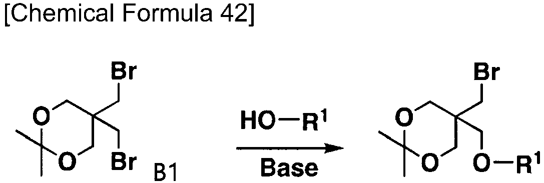

To a solution (2 to 10 mL) of alcohol R¹-OH (10 mmol) in 1,3-dimethylimidazolidin-2-one (DMI) was added sodium hydride (60% in paraffin) (10 to 20 mmol) at room temperature and the mixture was stirred for 5 min. B1 (30 to 40 mmol) was added to the resulting suspension and stirred at 100 °C for 20 hours. The suspension was then cooled to room temperature, deionized water (30 to 40 mL) was added, and an extraction with ethyl acetate/toluene (1/1) (60 to 100 mL) was performed. The resulting organic layer was washed with deionized water (30 to 40 mL, 2 to 3 times) and concentrated under reduced pressure. The residue was purified by silica gel column chromatography (toluene/ethyl acetate = 10/1 to 2/1) to give the target compound as a colorless liquid or solid (3.7 to 7.0 mmol, 37 to 70% yield).

### Synthesis Example 2

### [Etherification reaction of tribromide C1 with alcohol R²-OH]

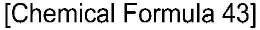

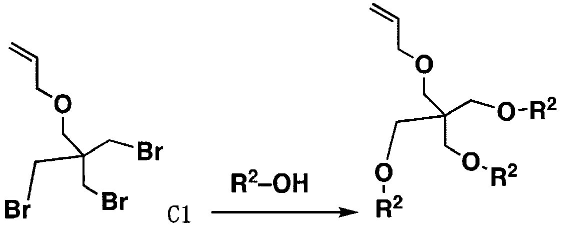

Sodium hydride (60% paraffin) (1.0 equivalent to R²-OH = 40 to 60 mmol) was added to a solution (2 to 10 mL) of alcohol R²-OH (40 to 60 mmol) in 1,3-dimethylimidazolidin-2-one (DMI) at room temperature and the mixture was stirred for 5 min. C1 (30 to 40 mmol) was added to the suspension and stirred at 100 °C overnight. The suspension was then cooled to room temperature, deionized water (30 to 40 mL) was added, and an extraction with ethyl acetate/toluene (1/1) (60 to 100 mL) was performed. The resulting organic layer was washed with deionized water (30 to 40 mL, 2 to 3 times) and concentrated under reduced pressure. DMI (<1 mmHg, boiling point : up to 100 °C) and excess starting alcohol R²-OH (<1 mmHg, boiling points of all products were approximately 140 to 150 °C, 30 to 50 mmol) were distilled from the residue under reduced pressure to be recovered. The resulting residue was purified by silica gel column chromatography (toluene/ethyl acetate = 4/1 to 1/1) to give the target compound as colorless liquid (6.50 to 7.40 mmol, 65 to 74 % yield based on conversion of R²-OH).

### Synthesis Example 3

### [Deallylation reaction of the compound obtained in Synthesis Example 2]

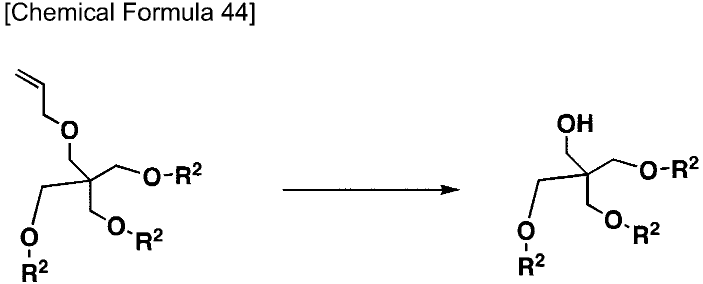

Potassium carbonate (30 to 40 mmol) was added to a solution of allyl ether (10 mmol) in methanol (40 to 60 ml). The resulting suspension was stirred under an argon atmosphere in an argon stream, and tetrakis(triphenylphosphine)palladium (0) (0.1 to 0.2 mmol) was added at room temperature, and the mixture was stirred at 65 °C for 8 to 20 hours. The suspension was filtered through Celite, and the filtrate was concentrated under reduced pressure to give the target compound, which was used in the next reaction without further purification.

### Synthesis Example 4

### [Linking reaction of epichlorohydrin with alcohol R³-OH]

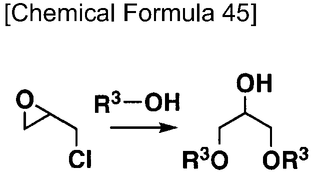

Potassium hydroxide (130 mmol) was added to a solution (30 mL) of alcohol R³-OH (156 mmol) in 1,3-dimethylimidazolidin-2-one (DMI) and the mixture was stirred at 80 °C for 30 min. The resulting suspension was then heated to 55 °C, and a solution (5 mL) of epichlorohydrin (4.6 g, 50 mmol) in 1,3-dimethylimidazolidin-2-one (DMI) was added dropwise. The resulting suspension was stirred at 55 °C for 45 hours and then at 80 °C for 22 hours. lon-exchanged water (50 to 80 mL) was poured into the suspension at room temperature, and an extraction with ethyl acetate (200 to 300 mL) was performed. The resulting organic layer was washed with ion-exchanged water (50 to 80 mL, 2 to 3 times) and concentrated under reduced pressure. After recovering DMI (<1 mmHg, up to 100 °C) and the excess starting alcohol R³-OH (<1 mmHg, 140 to 50 °C, up to 56 mmol), the residue was then distilled under reduced pressure to give the target compound as colorless solid or liquid by distillation (<1 mmHg, 200 to 210 °C) (21.2 to 29.1 mmol, 42.5 to 58.4% yield based on conversion of R³-OH).

### Synthesis Example 5

### [Tosylation reaction of the alcohols obtained in Synthesis Examples 3 and 4]

[Chemical Formula 46] HO-R⁴ **→ TsO-R⁴**

To a pyridine solution (28.0 mmol) of alcohol R⁴-OH (1.4 mmol) were added 4- dimethylaminopyridine (0.28 mmol) and then tosyl chloride (2.8 mmol) at room temperature, and the mixture was stirred for 8 to 20 hours. To the resulting reaction mixture were added saturated aqueous sodium bicarbonate (20 mL) and ethyl acetate (40 mL), and the mixture was stirred for 2 hours. The resulting organic layer was washed with ion-exchanged water (30 to 40 mL, 2 to 3 times) and then concentrated under reduced pressure. The resulting product was used in the next reaction without further purification.

### Synthesis Example 6

### [Etherification reaction of the compound obtained in Synthesis Example 1 or Synthesis Example 5 with the target compound]

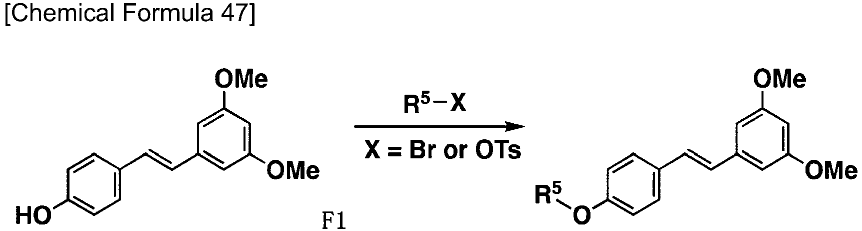

### (1) When X=OTs

Anhydrous potassium carbonate (17.36 mmol) and tosylate R⁵-OTs (7.23 mmol) were added to a solution of pterostilbene F1 (8.68 mmol) in N,N-dimethylformamide (15 mL) at room temperature, and the mixture was stirred at 90 °C for 11 hours. The suspension was then cooled to room temperature, deionized water (40 mL) was added, and an extraction with ethyl acetate/toluene (1/1) (100 mL) was performed. The resulting organic layer was washed with deionized water (40 mL, 2 times) and saturated aqueous sodium bicarbonate (20 mL) in sequence, and then concentrated under reduced pressure. The residue was purified by silica gel column chromatography (toluene / ethyl acetate = 5/1 to 2/1) to obtain the target linked body as colorless liquid (6.07 to 6.13 mmol, 70 to 84% yield).

### (2) When X=Br

Potassium hydroxide (15 to 20 mmol) and bromide R⁵-Br (10 mmol) were added to a DMI solution (10 to 20 mL) of pterostilbene F1 (15 to 20 mmol) at room temperature, and the mixture was stirred at 100 °C for 20 hours. The suspension was then cooled to room temperature, ion-exchanged water (30 to 40 mL) was poured, and an extraction with ethyl acetate / toluene (1/1) (60 to 100 mL) was performed. The resulting organic layer was washed with ion-exchanged water (30 to 40 mL, 2 to 3 times) and concentrated under reduced pressure. The residue was purified by silica gel column chromatography (toluene/ethyl acetate = 10/1 to 3/1) to give a linked body as the target compound as colorless liquids or solid (6.1 to 8.0 mmol, 61 to 80% yield).

### Synthesis Example 7

### [Deprotection reaction of the linked body obtained in Synthesis Example 6]

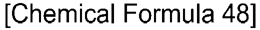

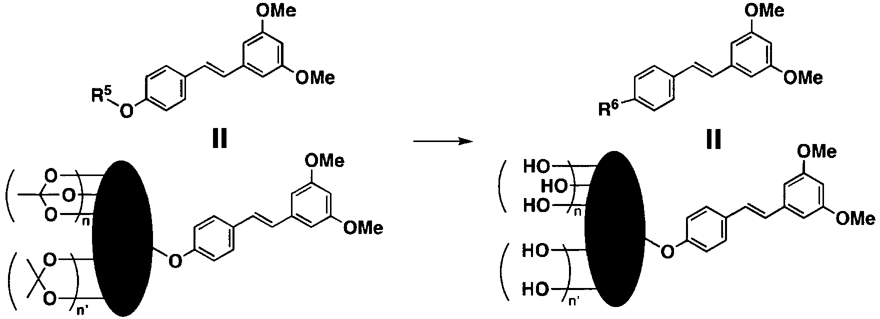

### (1) Reaction conditions when the structure does not contain an orthoester (n=0)

Hydrochloric acid (1 to 3 mol/L, 10 mL) was added to a methanol solution (10 mL) of the compound (10 mmol) obtained in Synthesis Example 6 at room temperature with stirring, and the mixture was stirred for 2 hours. The resulting mixture was then concentrated under reduced pressure. This procedure was repeated 2 to 3 times.

### (2) Reaction conditions when the structure contains an orthoester (n≠ 0)

Hydrochloric acid (1 to 3 mol/L, 10 mL) was added to a methanol solution (10 mL) of the compound (10 mmol) obtained in Synthesis Example 6 at room temperature with stirring, and the mixture was then concentrated under reduced pressure. This procedure was repeated 2 to 3 times. Then, 1.2 to 2.0 equivalents of sodium hydroxide (in terms of the orthoester moiety of the raw material) were added. After confirming by thin-layer chromatography that the product had converged to one point, the mixture was concentrated under reduced pressure.

### (3) Purification method when the target substance has sufficiently high water solubility

The residue was purified using a Diaion resin column (2-propanol / water = 1/4 to 1/1) to obtain the target deprotected linked body (8.5 to 9.8 mmol, 85 to 98% yield). This purification method was used in Examples 1 to 4, 8, 14, and 18.

### (4) Purification method when the target compound has insufficient water solubility

The crude target product was dissolved in 2-propanol to prepare a solution, and sodium bicarbonate was added until the solution reached a pH of 7 or higher. The resulting suspension was filtered, and the filtrate was concentrated under reduced pressure to obtain the target deprotected linked body (8.5 to 9.8 mmol, 85 to 98 % yield). This purification method was used in Examples 5, 6, 10, 11, 13, and 15 to 17.

### Example 1

### [Synthesis of compound G117:P₁ P₁]

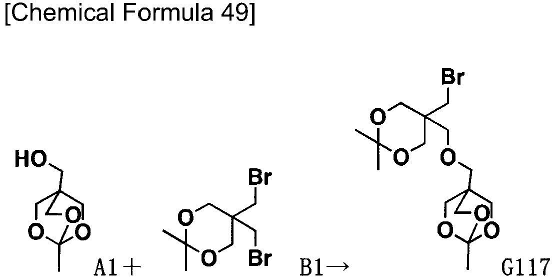

In Synthesis Example 1, A1 was used as R¹-OH to obtain the target compound G117.

Chemical Formula: C₁₅H₂₅BrO₆

4-(((5-(bromomethyl)-2,2-dimethyl-1,3-dioxan-5-yl)methoxy)methyl)-1-methyl-2,6,7-trioxabicyclo[2.2.2]octane
¹H NMR (CDCl₃, 400 MHz): δ 4.00 (s, 6H), 3.73 (d, J = 12.0 Hz, 2H), 3.69 (d, J = 12.0 Hz, 2H),3.47 (s, 2H), 3.42 (s, 2H), 3.23 (s, 2H), 1.46 (s, 3H), 1.41 (s, 3H), 1.40 (s, 3H).
¹³C NMR (CDCl₃, 100 MHz): δ 108.6 (C), 98.7 (C), 71.2 (CH₂), 69.9 (CH₂), 69.4 (CH₂×3), 63.7 (CH₂×2), 38.5 (C), 35.4 (CH₂) 35.1 (C), 23.8 (CH₃), 23.5 (CH₃), 23.4 (CH₃).

### [Synthesis of compound G118]

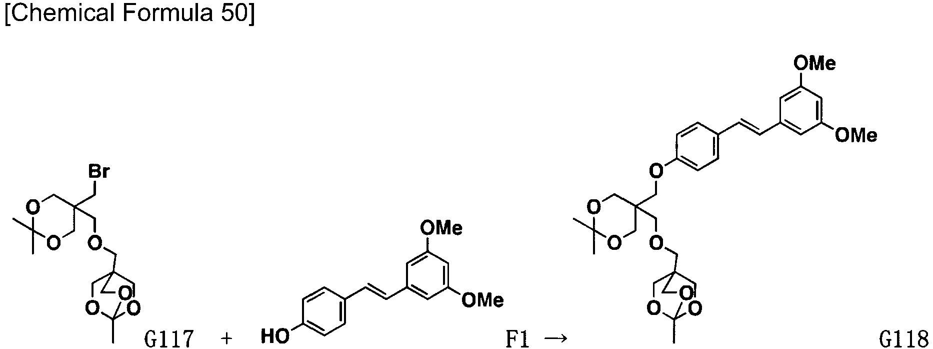

In Synthesis Example 6, item (2), G117 was used as R⁵-X to obtain the target compound, G118.

Chemical Formula: C₃₁H₄₀O₉

(E)-4-(((5-((4-(3,5-dimethoxystyryl)phenoxy)methyl)-2,2-dimethyl-1,3-dioxan-5-yl)methoxy)methyl)-1-methyl-2,6,7-trioxabicyclo[2.2.2]octane
¹H NMR (CDCl₃, 400 MHz): δ 7.44 (d, J = 8.8 Hz, 2H), 7.04 (d, J = 16.0 Hz, 1H), 6.91 (d, J = 16.0 Hz, 1H), 6.88 (d, J = 8.8 Hz, 2H), 6.65 (d, J = 2.4 Hz, 2H), 6.38 (brt, J = 2.4 Hz, 1H), 3.95 (s, 8H), 3.87-3.75 (m, 10H), 3.48 (s, 2H), 3.18 (s, 2H), 1.47-1.40 (m, 9H).
¹³C NMR (CDCl₃, 100 MHz): δ 161.1 (C×2), 158.8 (C), 139.7 (C), 130.4 (C), 128.7 (CH), 128.0 (CH×2), 126.9 (CH), 114.8 (CH×2), 108.6 (C),104.4 (CH×2), 99.8 (CH), 98.6 (C), 71.0 (CH₂), 70.0 (CH₂), 69.5 (CH₂×3), 67.3 (CH₂), 62.6 (CH2×2), 55.5 (CH₃×2), 39.0 (C), 35.2 (C), 24.1 (CH₃), 23.6 (CH₃×2).

### [Synthesis of compound G119]

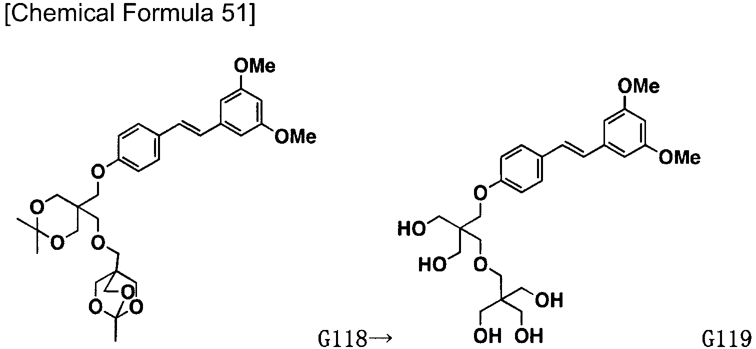

In Synthesis Example 7, item (2), G118 was used as the compound obtained in Synthesis Example 6 to obtain the target compound, G119.

Chemical Formula: C₂₆H₃₆O₉

(E)-2-((3-(4-(3,5-dimethoxystyryl)phenoxy)-2,2-bis(hydroxymethyl)propoxy)methyl)-2-(hydroxymethyl)propane-1,3-diol ¹H NMR (CD₃OD, 400 MHz): δ 7.48 (d, J = 8.8 Hz, 2H), 7.09 (d, J = 16.0 Hz, 1H), 6.95 (d, J = 16.0 Hz, 1H), 6.95 (d, J = 8.8 Hz, 2H), 6.68 (d, J = 2.4 Hz, 2H), 6.37 (brt, J = 2.4 Hz, 1H), 3.98 (s, 2H), 3.80 (s, 6H), 3.70 (s, 4H), 3.57 (s, 6H), 3.52 (2H), 3.43 (2H).
¹³C NMR (CD₃OD, 100 MHz): δ 162.4 (C×2), 160.5 (C), 141.2 (C), 131.5 (C), 129.7 (CH), 128.8 (CH×2), 127.5 (CH), 115.8 (CH×2), 105.3 (CH×2), 100.4 (CH), 71.8 (CH₂), 71.5 (CH₂), 67.9 (CH₂), 63.1 (CH₂×3), 62.4 (CH₂×2), 55.7 (CH₃×2), 47.1(C), 47.0 (C).

### Example 2

### [Synthesis of compound G127 : P₁G₁]

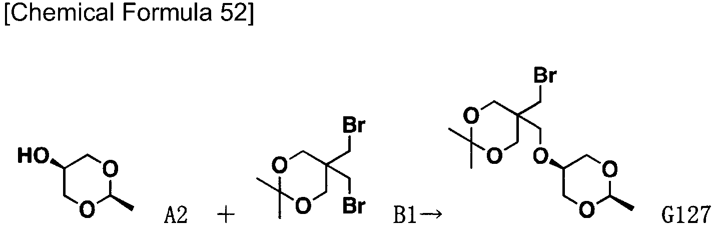

In Synthesis Example 1, A2 was used as R¹-OH to obtain the target compound, G127.

Chemical Formula: C₁₃H₂₃BrO₅

5-(bromomethyl)-2,2-dimethyl-5-((((2s,5s)-2-methyl-1,3-dioxan-5-yl)oxy)methyl)-1,3-dioxane
¹H NMR (CDCl₃, 400 MHz): δ 4.71 (q, J = 5.0 Hz, 1H), 4.18, (brd, J = 13.6 Hz, 2H), 3.86 (d, J = 12.0 Hz, 2H), 3.83 (m, 2H), 3.80 (d, J = 12.0 Hz, 2H), 3.64 (s, 2H), 3.56 (s, 2H), 3.18 (m, 1H), 1.42 (s, 3H), 1.42 (s, 3H), 1.34 (d, J = 5.2 Hz, 3H).
¹³C NMR (CDCl₃, 100 MHz): δ 99.1 (CH), 98.6 (C), 71.3 (CH), 68.4 (CH₂×2), 68.1 (CH2), 63.9 (CH₂×2), 38.5 (C), 36.2 (CH₂), 23.8 (CH₃), 23.7 (CH₃), 21.2 (CH₃).

### [Synthesis of compound G128]

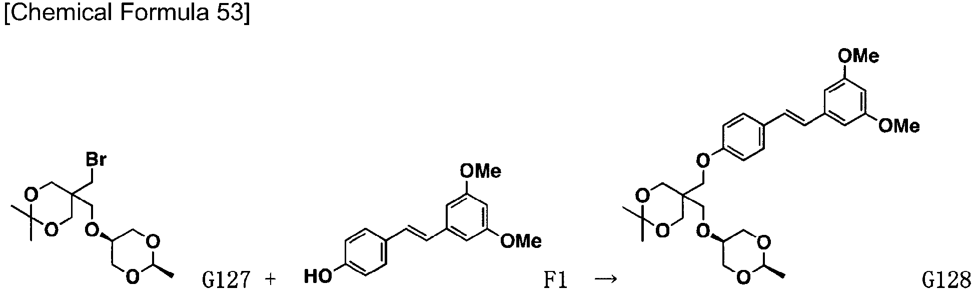

In Synthesis Example 6, item (2), G127 was used as R⁵-Br to obtain the target compound G128.

Chemical Formula: C₂₉H₃₈O₈

5-((4-((E)-3,5-dimethoxystyryl)phenoxy)methyl)-2,2-dimethyl-5-((((2s,5s)-2-methyl-1,3-dioxan-5-yl)oxy)methyl)-1,3-dioxane
¹H NMR (CDCl₃, 400 MHz): δ 7.41 (d, J = 8.8 Hz, 2H), 7.01 (d, J = 16.0 Hz, 1H), 6.90 (d, J = 8.8 Hz, 2H), 6.88 (d, J = 16.0 Hz, 1H), 6.63 (d, J = 2.4 Hz, 2H), 6.35 (brt, J = 2.4 Hz, 1H), 4.64 (q, J = 4.8 Hz, 1H), 4.10 (d, J = 11.6 Hz 2H), 4.05 (s, 2H), 3.90 (s, 4H), 3.78 (s, 6H), 3.72 (d, J = 11.6 Hz, 2H), 3.59 (s, 2H), 3.06 (m, 1H), 1.43 (s, 6H), 1.29 (d, J = 4.8 Hz, 3H).
¹³C NMR (CDCl₃, 100 MHz): δ 160.9 (C×2), 158.8 (C), 139.6 (C), 130.0 (C), 128.6 (CH), 127.7 (CH×2), 126.5 (CH), 114.8 (CH×2), 104.2 (CH×2), 99.6 (CH), 98.9 (CH), 98.2 (C), 71.3 (CH), 68.2 (CH₂×2), 67.6 (CH₂), 67.3 (CH₂), 62.5 (CH2×2), 55.2 (CH₃×2), 38.8 (C), 23.9 (CH₃), 23.6 (CH₃), 21.0 (CH₃).

### [Synthesis of compound G129]

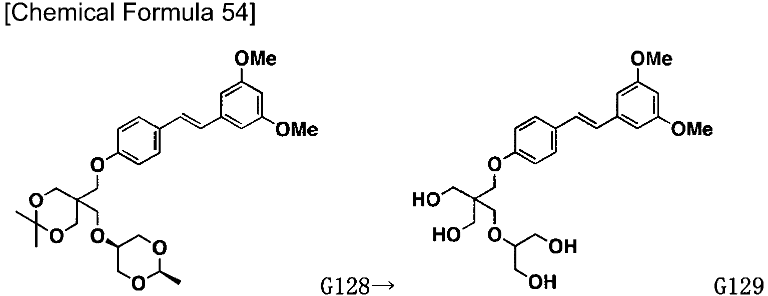

In Synthesis Example 7, item(1), G128 was used as the compound obtained in Synthesis Example 6 to obtain the target compound, G129.

Chemical Formula: C₂₄H₃₂O₃

(E)-2-(((1,3-dihydroxypropan-2-yl)oxy)methyl)-2-((4-(3,5-dimethoxystyryl)phenoxy)methyl)propane-1,3-diol
¹H NMR (CD₃OD, 400 MHz): δ 7.48 (d, J = 8.8 Hz, 2H), 7.09 (d, J = 16.0 Hz, 1H), 6.95 (d, J = 16.0 Hz, 1H), 6.95 (d, J = 8.8 Hz, 2H), 6.68 (d, J = 2.0 Hz, 2H), 6.37 (brt, J = 2.0 Hz, 1H), 4.00 (s, 2H), 3.81 (s, 6H), 3.77-3.68 (m, 7H), 3.68-3.52 (m, 4H).
¹³C NMR (CD₃OD, 100 MHz): δ 162.4 (C×2), 160.4 (C), 141.1 (C), 131.4 (C), 129.7 (CH), 128.8 (CH×2), 127.5 (CH), 115.8 (CH×2), 105.3 (CH×2), 100.4 (CH), 83.1 (CH), 70.1 (CH₂), 67.7 (CH₂), 62.4 (CH₂×4), 55.7 (CH₃×2), 47.0 (C).

### Example 3

### [Synthesis of compound G137 : P₁A₁]

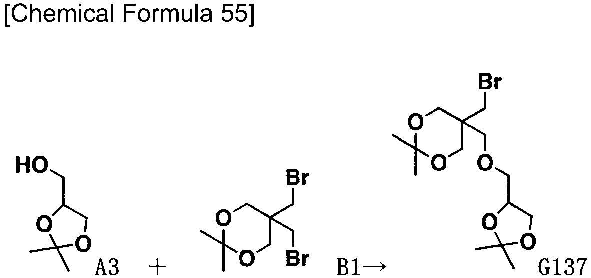

In Synthesis Example 1, A3 was used as R¹-OH to obtain the target compound G137.

Chemical Formula: C₁₄H₂₅BrO₅

5-(bromomethyl)-5-(((2,2-dimethyl-1,3-dioxolan-4-yl)methoxy)methyl)-2,2-dimethyl-1,3-dioxane-methane (1/1)
¹H NMR (CDCl₃, 400 MHz): δ 4.25 (m, 1H), 4.10-3.30 (m, 12H), 1.50-1.20 (m, 12H).
¹³C NMR (CDCl₃, 100 MHz): δ 109.5 (C), 98.7 (C), 74.7 (CH), 72.4 (CH₂), 71.2 (CH₂), 66.8 (CH₂), 63.9 (CH₂×2), 38.6 (C), 36.0 (CH₂), 26.9 (CH₃), 25.6 (CH₃), 24.2 (CH₃), 23.2 (CH₃).

### [Synthesis of compound G138]

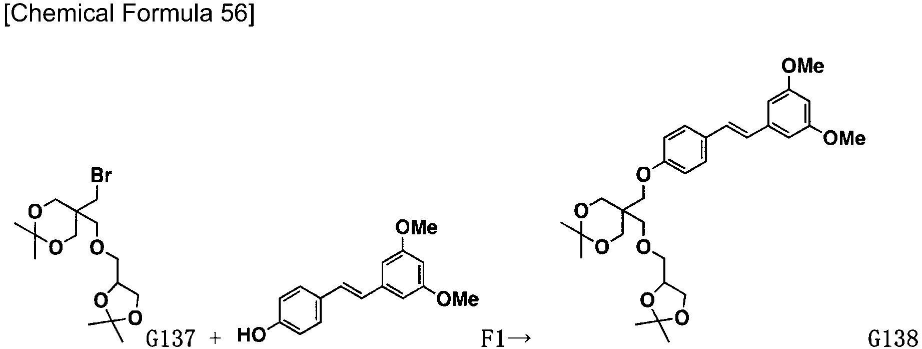

In Synthesis Example 6, item (2), G137 was used as R⁵-X to obtain the target compound G138.

Chemical Formula: C₃₆H₄₀O₈

¹H NMR (CDCl₃, 400 MHz): δ 7.43 (d, J = 8.8 Hz, 2H), 7.03 (d, J = 16.0 Hz, 1H), 6.91 (d, J = 8.8 Hz, 1H), 6.90 (d, J = 16.0 Hz, 2H), 6.65 (d, J = 2.4 Hz, 2H), 6.37 (brt, J = 2.4 Hz, 1H), 4.27-4.16 (m, 1H), 4.02 (s, 2H), 4.01-3.96 (m, 1H), 3.86 (s, 4H), 3.82 (s, 6H), 3.75-3.64 (m, 1H), 3.62-3.40 (m, 4H), 1.44 (s, 3H), 1.43 (s, 3H), 1.39 (s, 3H), 1.33 (s, 3H).
¹³C NMR (CDCl₃, 100 MHz): δ 161.1 (C×2), 158.9 (C), 139.8 (C), 130.2 (C), 128.8 (CH), 127.9 (CH×2), 126.7 (CH), 114.9 (CH×2), 109.4 (C),104.4 (CH×2), 99.7 (CH), 98.5 (C), 74.7 (CH), 72.4 (CH₂), 71.0 (CH₂), 67.3 (CH₂), 66.8 (CH₂), 62.7 (CH₂), 62.7 (CH₂), 55.5 (CH₃×2), 39.0 (C), 26.8 (CH₃), 25.6 (CH₃), 24.6 (CH₃), 23.1 (CH₃).

### [Synthesis of compound G139]

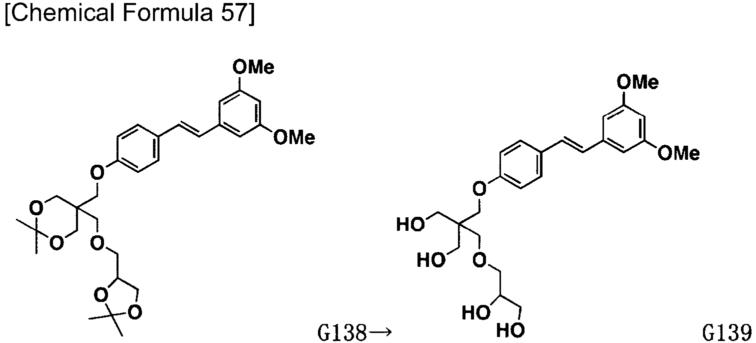

In Synthesis Example 7, item (1), G138 was used as the compound obtained in Synthesis Example 6 to obtain the target compound, G139.

Chemical Formula: C₂₄H₃₂O₈

(E)-3-(3-(4-(3,5-dimethoxystyryl)phenoxy)-2,2-bis(hydroxymethyl)propoxy)propane-1,2-diol
¹H NMR (CD₃OD, 400 MHz): δ 7.42 (d, J = 8.8 Hz, 2H), 7.04 (d, J = 16.4 Hz, 1H), 6.91 (d, J = 8.8 Hz, 2H), 6.89 (d, J = 16.4 Hz, 1H), 6.65 (d, J = 2.0 Hz, 2H), 6.35 (brt, J = 2.0 Hz, 1H), 3.96 (s, 2H), 3.80-3.73 (m, 1H), 3.76 (s, 6H), 3.70 (s, 4H), 3.65-3.41 (m, 6H).
¹³C NMR (CD₃OD, 100 MHz): δ 162.4 (C×2), 160.4 (C), 141.1 (C), 131.4 (C), 129.7 (CH), 128.8 (CH×2), 127.5 (CH), 115.8 (CH×2), 105.3 (CH×2), 100.4 (CH), 72.6 (CH₂), 70.8 (CH), 70.1 (CH₂), 66.5 (CH₂), 63.1 (CH₂), 61.1 (CH₂×2), 54.5 (CH₃×2), 46.8 (C).

### Example 4

### [Synthesis of compound H115 : P₁P₃]

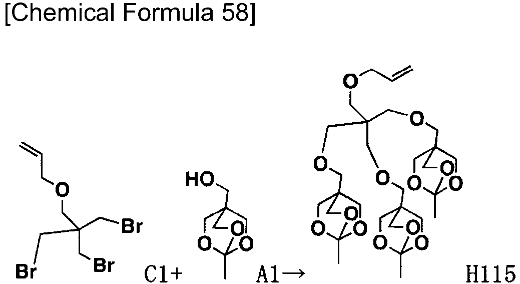

In Synthesis Example 2, A1 was used as R²-OH to obtain the target compound H115.

Chemical Formula: C₂₉H₄₆O₁₃

4,4'-(((2-((allyloxy)methyl)-2-(((1-methyl-2,6,7-trioxabicyclo[2.2.2]octan-4-yl)methoxy)methyl)propane-1,3-diyl)bis(oxy))bis(methylene))bis(1-methyl-2,6,7-trioxabicyclo[2.2.2]octane)
¹H NMR (CDCl₃, 400 MHz): δ 5.82 (ddt, J = 5.6, 10.4, 17.6 Hz, 1H), 5.28-5.14 (m, 2H), 4.00 (s, 18H) 3.90 (ddd, J = 1.2, 1.2, 5.6 Hz, 2H), 3.30-3.11 (m, 14H), 1.46 (s, 9H).
¹³C NMR (CDCl₃, 100 MHz): δ 134.6 (CH), 117.0 (CH₂) 108.6 (C×3), 72.3 (CH2), 70.3 (CH₂×3), 69.8 (CH₂×3), 69.4 (CH₂×9), 68.5 (CH₂), 45.7 (C), 35.2 (Cx3), 23.5 (CH₃×3).

### [Synthesis of compound H116]

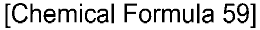

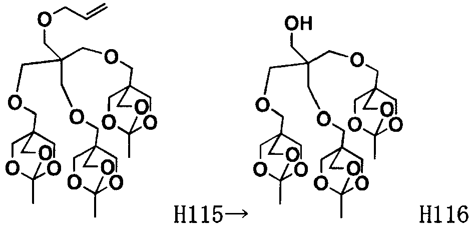

A deallylation was carried out in the same manner as in Synthesis Example 3 to obtain the target compound H116.

Chemical Formula: C₂₆H₄₂O₁₃

3-((1-methyl-2,6,7-trioxabicyclo[2.2.2]octan-4-yl)methoxy)-2,2-bis(((1-methyl-2,6,7-trioxabicyclo[2.2.2]octan-4-yl)methoxy)methyl)propan-1-ol ¹H NMR (CDCl₃, 400 MHz): δ 3.97 (s, 18H), 3.56 (d, J = 5.6 Hz, 2H), 3.30 (s, 6H), 3.16 (s, 6H), 1.98 (t, J = 5.6 Hz, 1H), 1.45 (s, 9H).
¹³C NMR (CDCl₃, 100 MHz): δ 108.7 (C×3), 71.2 (CH₂×2), 70.1 (CH₂×2), 69.5 (CH₂×11), 63.7 (CH₂), 45.7 (C), 35.2 (C×3), 23.5 (CH₃×3).

### [Synthesis of compound H117]

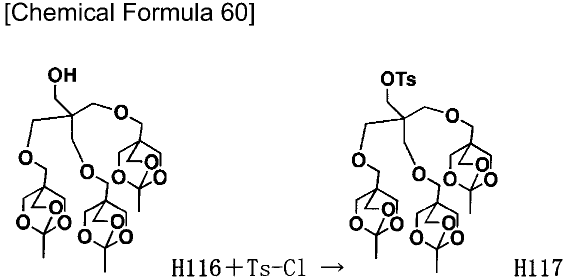

In Synthesis Example 5, H116 was used as R⁴-OH to obtain the target compound, H117. The crude H117 was used as is in the next reaction.

### [Synthesis of compound H118]

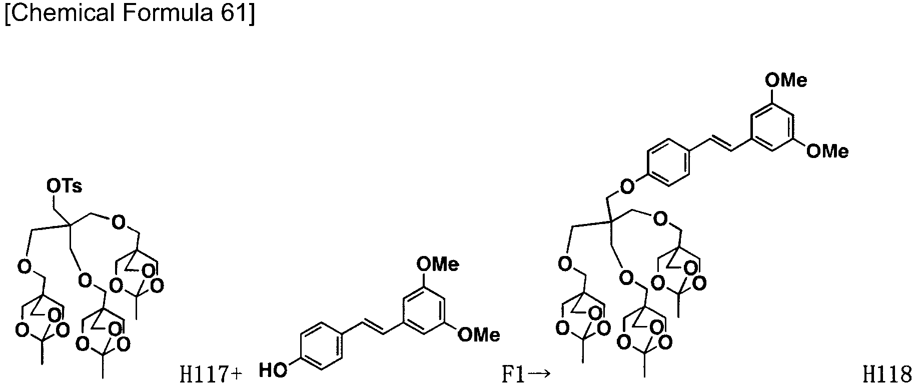

In Synthesis Example 6, item (1), H117 was used as R⁵-OTs to obtain the target compound, H118. The crude H118 was used as is in the next reaction.

### [Synthesis of compound H119]

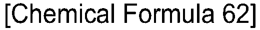

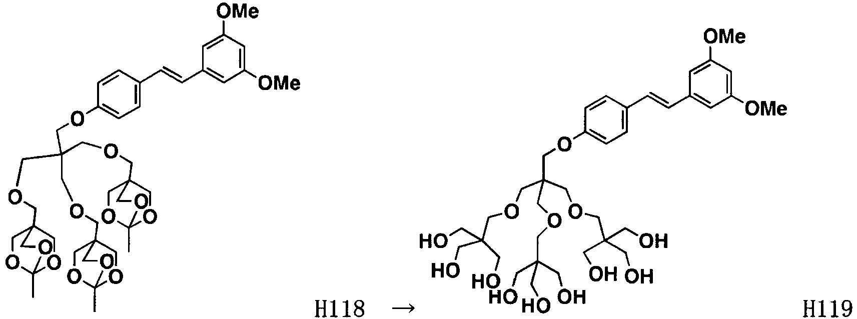

In Synthesis Example 7, item (2), H118 was used as the compound obtained in Synthesis Example 6 to obtain the target compound, H119.

Chemical Formula: C₃₆H₅₆O₁₅

(E)-2,2'-(((2-((4-(3,5-dimethoxystyryl)phenoxy)methyl)-2-((3-hydroxy-2,2-bis(hydroxymethyl)propoxy)methyl)propane-1,3-diyl)bis(oxy))bis(methylene))bis(2-(hydroxymethyl)propane-1,3-diol)
¹H NMR (CD₃OD, 400 MHz): δ 7.47 (d, J = 8.8 Hz, 2H), 7.08 (d, J = 16.0 Hz, 1H), 6.94 (d, J = 16.0 Hz, 1H), 6.94 (d, J = 8.8 Hz, 2H), 6.67 (d, J = 2.0 Hz, 2H), 6.36 (brt, J = 2.0 Hz, 1H), 4.00 (s, 2H), 3.80 (s, 6H), 3.65-3.50 (m, 24H), 3.47-3.40 (m, 6H).
¹³C NMR (CD₃OD, 100 MHz): δ 162.4 (C×2), 160.6 (C), 141.1 (C), 131.3 (C), 129.7 (CH), 128.9 (CH×2), 127.5 (CH), 115.9 (CH×2), 105.3 (CH×2), 100.5 (CH), 72.6 (CH₂×2), 72.0 (CH₂×2), 68.0 (CH₂), 63.5 (CH₂×7), 63.3 (CH₂×2), 63.0 (CH₂×2), 55.8 (CH₃×2), 47.1 (C), 46.9 (C×3).

### Example 5

### [Synthesis of compound H123 : P₁G₃]

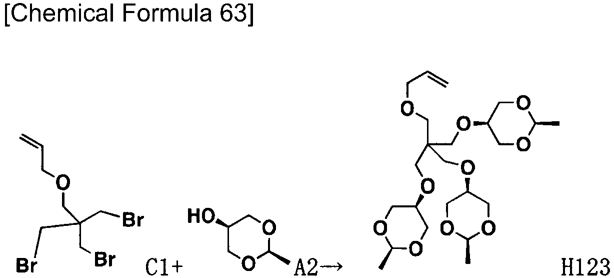

In Synthesis Example 2, A2 was used as R²-OH to obtain the target compound, H123.

Chemical Formula: C₂₃H₄₀O₁₀

(2S,2'S,5s,5's)-5,5'-((2-((allyloxy)methyl)-2-((((2S,5s)-2-methyl-1,3-dioxan-5-yl)oxy)methyl)propane-1,3-diyl)bis(oxy))bis(2-methyl-1,3-dioxane)
¹H NMR (CDCl₃, 400 MHz): δ 5.89 (ddt, J = 5.6, 10.4, 17.2 Hz, 1H), 5.24 (ddd, J = 1.6, 1.6, 17.2 Hz, 1H, one of CH₂), 5.11 (ddd, J = 1.6, 1.6, 10.4 Hz, 1H, one of CH₂), 4.68 (q, J = 4.8 Hz, 3H),4.14 (dd, J = 1.6, 12.4 Hz, 6H), 3.97 (ddd, J = 1.6, 1.6, 5.6 Hz, 2H), 3.77 (dd, J = 1.6, 12.4 Hz, 6H), 3.62-3.57 (m, 8H), 3.19 (m, 3H), 1.31 (d, J = 4.8 Hz, 9H).
¹³C NMR (CDCl₃, 100 MHz): δ 135.5 (CH), 116.1 (CH₂), 99.0 (CH×3), 72.3 (CH2), 71.2 (CH×3), 69.3 (CH2), 68.6 (CH2×6), 67.7 (CH2×3), 45.7 (C), 21.2 (CH₃×3).

### [Synthesis of compound H123']

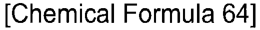

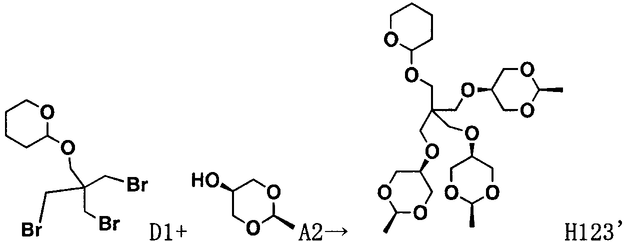

In Synthesis Example 2, D1 was used as tribromide and A2 was used as R²-OH to obtain the target compound, H123'.

Chemical Formula: C₂₅H₄₄O₁₁

¹H NMR (CDCl₃, 400 MHz) d 4.68 (q, J = 4.8 Hz, 3H), 4.62 (t, J = 4.8 Hz, 1H), 4.13 (brd, J = 12.0 Hz, 6H), 3.87 (d, J = 9.2 Hz, 2H), 3.77 (brd, J = 12.0 Hz, 6H), 3.62 (s, 6H), 3.90-3.33 (m, 1H), 3.51 (d, J = 9.2 Hz, 1H), 3.53-3.44 (m, 1H), 3.21-3.19 (m, 3H), 1.75-1.42 (m, 6H), 1.29 (d, J = 4.8 Hz, 9H).
¹³C NMR (CDCl₃, 100 MHz) d 98.8 (CH), 98.7 (CH × 3), 70.9 (CH × 3), 68.4 (CH₂ × 3), 68.2 (CH₂ × 3), 67.5 (CH₂ × 3), 66.4 (CH₂), 61.6 (CH₂), 45.1 (C), 30.4 (CH₂), 25.3 (CH₂), 20.9 (CH₃ × 3), 19.2 (CH₂)

### [Synthesis of compound H123"]

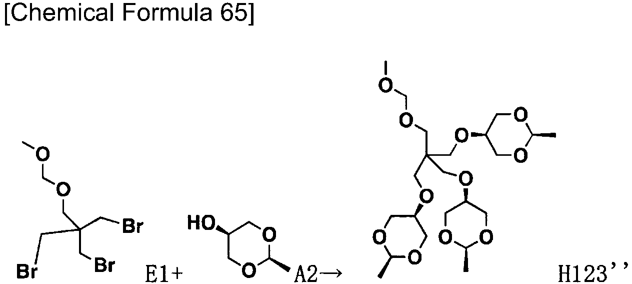

In Synthesis Example 2, E1 was used as tribromide and A2 was used as R²-OH to obtain the target compound, H123".

Chemical Formula: C₂₂H₄₀O₁₁

(2S,2'S,5s,5's)-5,5'-((2-((methoxymethoxy)methyl)-2-((((2S,5s)-2-methyl-1,3-dioxan-5-yl)oxy)methyl)propane-1,3-diyl)bis(oxy))bis(2-methyl-1,3-dioxane) ¹H NMR (CDCl₃, 400 MHz): δ 4.69 (q, J = 5.2 Hz, 3H), 4.62 (s, 2H), 4.14 (brd, J = 12.4 Hz, 6H), 3.78 (brd, J = 12.4 Hz, 6H), 3.68 (s, 2H), 3.60 (s, 6H), 3.35 (s, 3H), 3.23-3.17 (m, 3H), 1.30 (d, J = 5.2 Hz, 9H).
¹³C NMR (CDCl₃, 100 MHz): δ 99.0 (CH×3), 97.1 (CH₂), 71.3 (CH×3), 68.6 (CH₂×6), 67.5 (CH₂×3), 67.0 (CH₂), 55.2 (CH₃), 45.4 (C), 21.2 (CH₃×3).

### [Synthesis of compound H124]

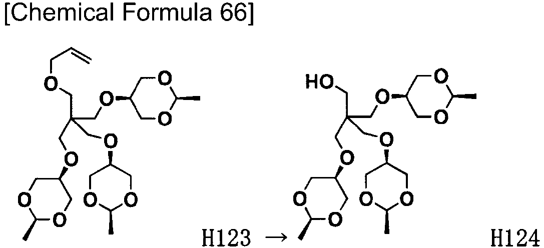

A deallylation was carried out in the same manner as in Synthesis Example 3 to obtain the target compound H124.

Chemical Formula: C₂₀H₃₆O₁₀

3-(((2S,5s)-2-methyl-1,3-dioxan-5-yl)oxy)-2,2-bis((((2S,5s)-2-methyl-1,3-dioxan-5-yl)oxy)methyl)propan-1-ol
¹H NMR (CDCl₃, 400 MHz): δ 4.70 (q, J = 5.2 Hz, 3H), 4.20-4.10 (m, 6H), 3.85-3.74 (m, 8H), 3.64 (s, 6H), 3.26-3.21 (m, 3H), 3.20 (t, J = 5.2 Hz, 1H), 1.31 (d, J = 5.2 Hz, 9H).
¹³C NMR (CDCl₃, 100 MHz): δ 99.0 (CH×3), 71.0 (CH×3), 68.5 (CH₂×3) ,68.3 (CH₂×6), 65.2 (CH₂), 45.4 (C), 21.1 (CH₃×3).

### [Synthesis of compound H127']

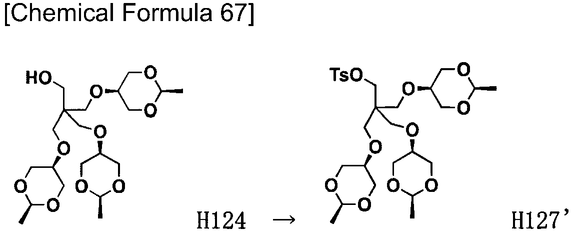

In Synthesis Example 5, item (1), H124 was used as R⁴-OH to obtain the target compound, H127'. The crude H127' was used as is in the next reaction.

### [Synthesis of compound H128']

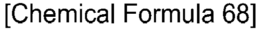

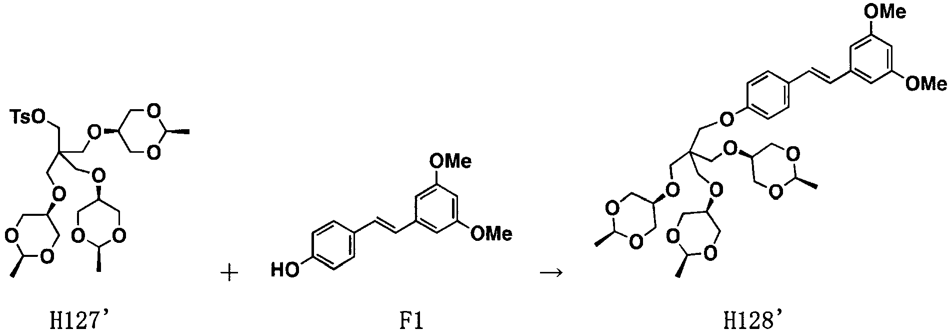

In Synthesis Example 6, item (1), H127' was used as R⁵-OTs to obtain the target compound, H128'.

Chemical Formula: C₃₆H₅₀O₁₂

(2S,2'S,5s,5's)-5,5'-((2-((4-((E)-3,5-dimethoxystyryl)phenoxy)methyl)-2-((((2S,5s)-2-methyl-1,3-dioxan-5-yl)oxy)methyl)propane-1,3-diyl)bis(oxy))bis(2-methyl-1,3-dioxane)
¹H NMR (CDCl₃, 400 MHz): δ 7.43 (d, J = 8.4 Hz, 2H), 7.03 (d, J = 16.0 Hz, 1H), 6.91 (d, J = 8.4 Hz, 2H), 6.89 (d, J = 16.0 Hz, 1H), 6.65 (d, J = 2.4 Hz, 2H), 6.37 (brt, J = 2.4 Hz, 1H), 4.68 (q, J = 5.2 Hz, 3H), 4.18 (s, 2H), 4.17-4.09 (m, 6H), 3.83 (s, 6H), 3.81-3.70 (m, 12H), 3.21 (m, 3H), 1.30 (d, J = 5.2 Hz, 9H). ¹³C NMR (CDCl₃, 100 MHz): δ 161.0 (C×2), 159.0 (C), 139.9 (C), 129.9 (C), 129.1 (CH), 127.8 (CH×2), 126.5 (CH), 115.1 (CH×2), 104.4 (CH×2), 99.8 (CH), 99.1 (CH×3), 71.4 (CH×3), 68.7 (CH₂×6), 67.6 (CH2×3), 67.5 (CH₂), 55.5 (CH₃×2), 45.7 (C), 21.3 (CH₃×3).

### [Synthesis of compound H125]

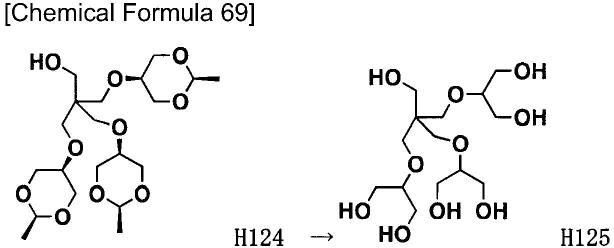

To an aqueous suspension (9.5 mL) of H124 (9.5 mmol) was added amberlyst-15 (Merck-Aldrich, sulfonic acid content 2 mmol/g) (48 mg), and the mixture was stirred at 100 °C overnight. The suspension was filtered, and the filtrate was concentrated under reduced pressure to obtain the target compound, H125. This was used in the next reaction without further purification.

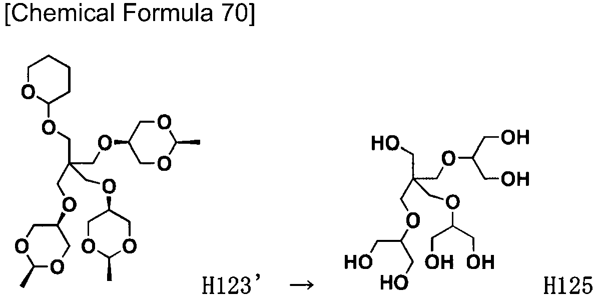

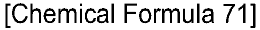

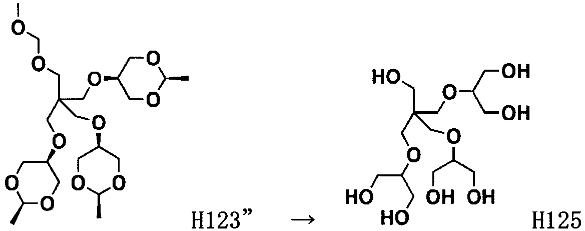

The synthesis of H125 from H123' and the synthesis of H125 from H123" were also carried out according to the above schemes.

### [Synthesis of compound H126]

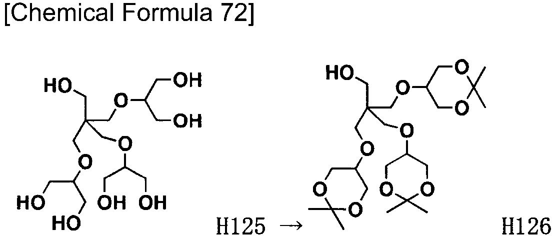

To a suspension of H125 (8.12 mmol) in toluene (20 mL) was added 2,2-dimethoxypropane (73.08 mmol) and amberlyst-15 (Merck-Aldrich, sulfonic acid content 2 mmol/g) (81.2 mg), and the mixture was stirred at 85 °C for 2 hours while distilling off methanol. The resulting suspension was filtered at room temperature, and triethylamine (1 mL) was added to the filtrate, which was then concentrated under reduced pressure. The residue was purified by silica gel column chromatography (toluene/ethyl acetate = 1/1 to 1/4) to give the target compound H126 as colorless liquid (5.28 mmol, 52.8% yield by three steps from H122).

Chemical Formula: C₂₃H₄₂O₁₀

3-((2,2-dimethyl-1,3-dioxan-5-yl)oxy)-2,2-bis(((2,2-dimethyl-1,3-dioxan-5-yl)oxy)methyl)propan-1-ol
¹H NMR (CDCl₃, 400 MHz): δ 3.95 (dd, J = 4.0, 12.0 Hz, 6H), 3.74 (dd, 6.0, 12.0 Hz, 6H), 3.69 (brd, J = 4.0 Hz, 2H), 3.51 (s, 6H), 3.4-3.30 (m, 3H), 2.99 (brt, 1H), 1.41 (s, 9H), 1.40 (s, 9H).
¹³C NMR (CDCl₃, 100 MHz): δ 98.3 (C×3), 70.9 (CH×3), 68.4 (CH₂×3), 64.5 (CH₂), 62.6 (CH₂×6), 45.2 (C), 23.9 (CH₃×3), 23.5 (CH₃×3).

### [Synthesis of compound H127]

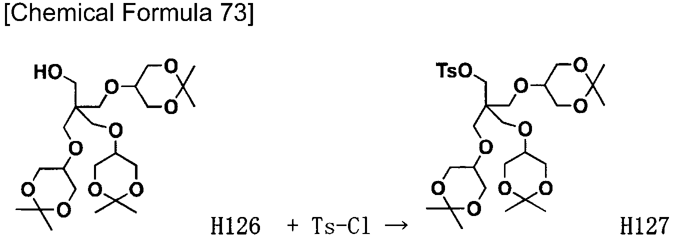

In Synthesis Example 5, H126 was used as R⁴-OH to obtain the target compound, H127. The crude H127 was used as is in the next reaction.

### [Synthesis of compound H128]

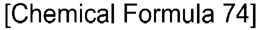

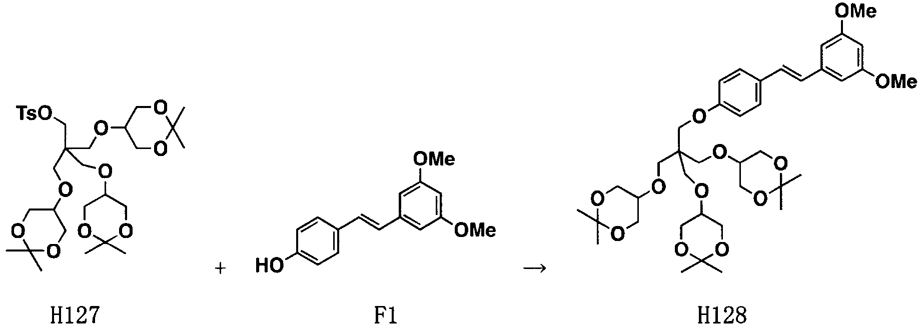

In Synthesis Example 6, item (1), H127 was used as R⁵-OTs to obtain the target compound, H128.

Chemical Formula: C₃₉H₅₆O₁₂

(E)-5,5'-((2-((4-(3,5-dimethoxystyryl)phenoxy)methyl)-2-(((2,2-dimethyl-1,3-dioxan-5-yl)oxy)methyl)propane-1,3-diyl)bis(oxy))bis(2,2-dimethyl-1,3-dioxane) ¹H NMR (CDCl₃, 400 MHz): δ 7.42 (d, J = 8.8 Hz, 2H), 7.03 (d, J = 16.4 Hz, 1H), 6.90 (d, J = 16.4 Hz, 1H), 6.89 (d, J = 8.8 Hz, 2H), 6.65 (d, J = 2.4 Hz, 2H), 6.38 (brt, J = 2.4 Hz, 1H), 3.98 (s, 2H), 3.93 (dd, J = 4.4, 12.0 Hz, 6H), 3.83 (s, 6H), 3.68 (dd, J = 6.4, 12.0 Hz, 6H), 3.57 (s, 6H), 3.44-3.34 (m, 3H), 1.40 (s, 9H), 1.38 (s, 9H).
¹³C NMR (CDCl₃, 100 MHz): δ 161.1 (C×2), 159.1 (C), 139.8 (C), 130.1 (C), 128.9 (CH), 127.9 (CH×2), 126.6 (CH), 115.0 (CH×2), 104.4 (CH×2), 99.7 (CH), 98.3 (C×3), 71.0 (CH×3), 67.2 (CH2×3), 66.7 (CH₂), 62.7 (CH₂×6), 55.5 (CH₃×2), 45.4 (C), 24.6 (CH₃×3), 22.9 (CH₃×3).

### [Synthesis of compound H129]

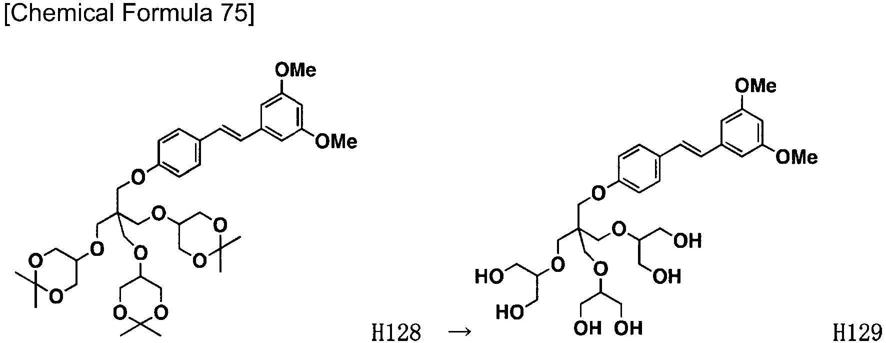

In Synthesis Example 7, item (1), H128 was used as the compound obtained in Synthesis Example 6 to obtain the target compound, H129.

Chemical Formula: C₃₀H₄₄O₁₂

(E)-2,2'-((2-(((1,3-dihydroxypropan-2-yl)oxy)methyl)-2-((4-(3,5-dimethoxystyryl)phenoxy)methyl)propane-1,3-diyl)bis(oxy))bis(propane-1,3-diol) ¹H NMR (CD₃OD, 400 MHz): δ 7.46 (d, J = 8.8 Hz, 2H), 7.08 (d, J = 16.0 Hz, 1H), 6.94 (d, J = 8.8 Hz, 2H), 6.93 (d, J = 16.0 Hz, 1H), 6.67 (d, J = 2.0 Hz, 2H), 6.36 (brt, J = 2.0 Hz, 1H), 4.07 (s, 2H), 3.80 (s, 6H), 3.76-3.69 (m, 6H), 3.67-3.52 (m, 12H), 3.44-3.35 (m, 3H).
¹³C NMR (CD₃OD, 100 MHz): δ 162.5 (C×2), 160.5 (C), 141.2 (C), 131.5 (C), 129.7 (CH), 128.8 (CH×2), 127.5 (CH), 115.9 (CH×2), 105.3 (CH×2), 100.4 (CH), 83.0 (CH×3), 69.6 (CH2×3), 68.0 (CH₂), 62.4 (CH₂×6), 55.7 (CH₃×2), 46.9 (C).

### Example 6

### [Synthesis of compound H135 : P₁A₃]

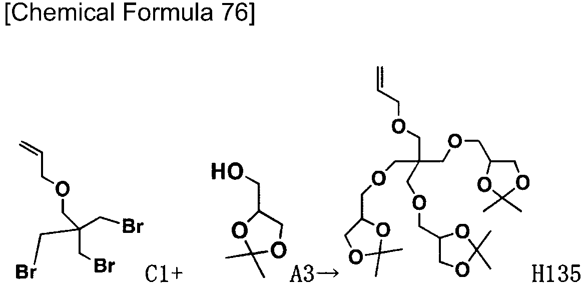

In Synthesis Example 2, A3 was used as R²-OH to obtain the target compound, H135.

Chemical Formula: C₂₆H₄₆O₁₀

4,4'-(((2-((allyloxy)methyl)-2-(((2,2-dimethyl-1,3-dioxolan-4-yl)methoxy)methyl)propane-1,3-diyl)bis(oxy))bis(methylene))bis(2,2-dimethyl-1,3-dioxolane)--methane (1/1)
¹H NMR (CDCl₃, 400 MHz): δ 5.93-5.80 (m, 1H), 5.28-5.10 (m, 2H), 4.26-4.17 (m, 3H), 4.03 (dd, J = 6.4, 8.0 Hz, 3H), 3.95-3.90 (m, 2H), 3.75 (dd, J = 6.4, 8.0 Hz, 3H), 3.53-3.37 (m, 14H), 1.41 (s, 9H), 1.36 (s, 9H).
¹³C NMR (CDCl₃, 100 MHz): δ 135.0 (CH), 116.1 (CH₂) 109.1 (C×3), 74.5 (CH×4), 72.2 (CH₂×3), 70.2 (CH₂×3), 68.8 (CH₂), 66.8 (CH₂×3), 45.6 (C), 26.7 (CH₃×3), 25.5 (CH₃×3).

### [Synthesis of compound H136]

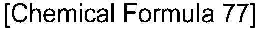

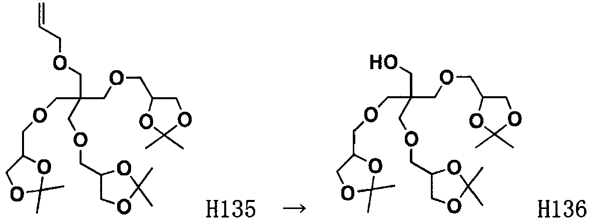

A deallylation reaction was carried out in the same manner as in Synthesis Example 3 to obtain the target compound H136.

Chemical Formula: C₂₃H₄₂O₁₀

3-((2,2-dimethyl-1,3-dioxolan-4-yl)methoxy)-2,2-bis(((2,2-dimethyl-1,3-dioxolan-4-yl)methoxy)methyl)propan-1-ol
¹H NMR (CDCl₃, 400 MHz): δ 4.28-4.14 (m, 3H), 4.10-3.98 (m, 4H), 3.78-3.60 (m, 4H), 3.54-3.40 (m, 12H), 3.25-2.84 (m, 1H), 1.40 (s, 6H), 1.40 (s, 3H), 1.34 (s, 9H).
¹³C NMR (CDCl₃, 100 MHz): δ 109.5 (C×2), 109.4 (C), 74.7 (CH×3), 72.5 (CH2×2), 71.9 (CH2), 70.2 (CH2), 66.9 (CH2), 66.7 (CH2×2), 65.4 (CH2), 45.5 (C), 26.9 (CH₃×3), 25.5 (CH₃×3).

### [Synthesis of compound H137]

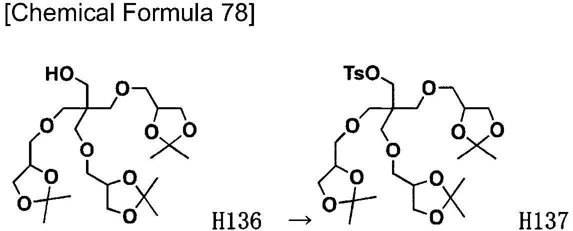

In Synthesis Example 5, H136 was used as R⁴-OH to obtain the target compound, H137. The crude H137 was used as is in the next reaction.

### [Synthesis of compound H138]

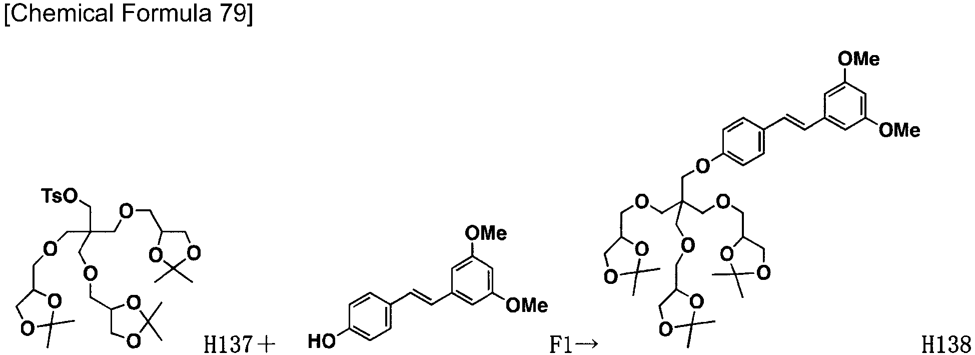

In Synthesis Example 6, item (1), H137 was used as R⁵-OTs to obtain the target compound, H138.

Chemical Formula: C₃₉H₅₆O₁₂

(E)-4,4'-(((2-((4-(3,5-dimethoxystyryl)phenoxy)methyl)-2-(((2,2-dimethyl-1,3-dioxolan-4-yl)methoxy)methyl)propane-1,3-diyl)bis(oxy))bis(methylene))bis(2,2-dimethyl-1,3-dioxolane)
¹H NMR (CDCl₃, 400 MHz): δ 7.42 (d, J = 8.8 Hz, 2H), 7.03 (d, J = 16.0 Hz, 1H), 6.90 (d, J = 16.0 Hz, 1H), 6.89 (d, J = 8.8 Hz, 2H), 6.65 (d, J = 2.4 Hz, 2H), 6.38 (brt, J = 2.4 Hz, 1H), 4.27-4.13 (m, 3H), 4.12-3.98 (m, 4H), 3.97 (s, 2H), 3.83 (s, 6H), 3.78-3.65 (m, 4H), 3.57 (s, 6H), 3.54-3.34 (m, 4H), 1.40 (s, 9H), 1.34 (s, 9H).
¹³C NMR (CDCl₃, 100 MHz): δ 161.1 (C×2), 159.1 (C), 139.8 (C), 130.0 (C), 128.9 (CH), 127.9 (CH×2), 126.6 (CH), 114.9 (CH×2), 109.4 (C×3), 104.4 (CH×2), 99.7 (CH), 74.7 (CH×3), 72.5 (CH₂×3), 70.2 (CH₂×3), 67.0 (CH2), 66.9 (CH2×3), 55.5 (CH₃×2), 45.7 (C), 26.9 (CH₃×3), 25.6 (CH₃×3).

### [Synthesis of compound H139]

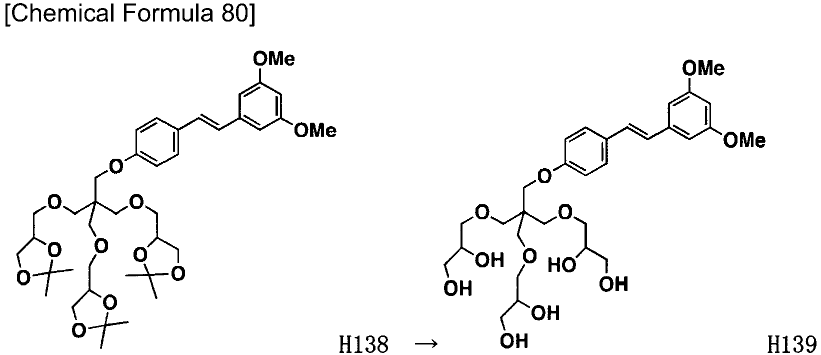

In Synthesis Example 7, item (1), H138 was used as the compound obtained in Synthesis Example 6 to obtain the target compound, H139.

Chemical Formula: C₃₀H₄₄O₁₂

(E)-3,3'-((2-((2,3-dihydroxypropoxy)methyl)-2-((4-(3,5-dimethoxystyryl)phenoxy)methyl)propane-1,3-diyl)bis(oxy))bis(propane-1,2-diol) ¹H NMR (CD₃OD, 400 MHz): δ 7.47 (d, J = 8.8 Hz, 2H), 7.08 (d, J = 16.0 Hz, 1H), 6.93 (d, J = 16.0 Hz, 1H), 6.93 (d, J = 8.8 Hz, 2H), 6.68 (d, J = 2.4 Hz, 2H), 6.36 (brt, J = 2.4 Hz, 1H), 4.02 (s, 2H), 3.80 (s, 6H), 3.79-3.70 (m, 4H), 3.68-3.98 (m, 17).
¹³C NMR (CD₃OD, 100 MHz): δ 162.5 (C×2), 160.5 (C), 141.2 (C), 131.5 (C), 129.7 (CH), 128.8 (CH×2), 127.5 (CH), 115.9 (CH×2), 105.2 (CH×2), 100.4 (CH), 74.0 (CH₂×2), 73.9 (CH2), 72.2 (CH₂×3), 71.1 (CH×3), 68.2 (CH₂), 64.5(CH₂×2), 64.4 (CH₂), 55.7 (CH₃×2), 46.7 (C).

### Example 7

### [Synthesis of compound H145 : P₁T₃]

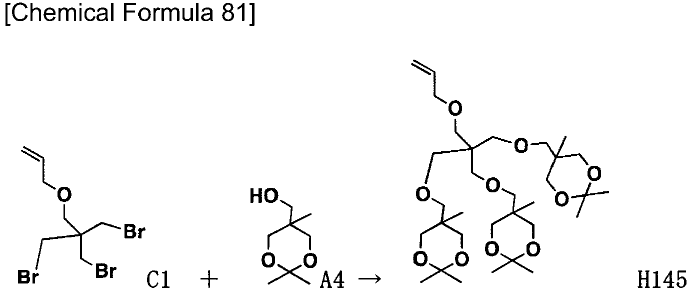

In Synthesis Example 2, A4 was used as R²-OH to obtain the target compound, H145.

Chemical Formula: C₃₂H₅₈O₁₀

5,5'-(((2-((allyloxy)methyl)-2-(((2,2,5-trimethyl-1,3-dioxan-5-yl)methoxy)methyl)propane-1,3-diyl)bis(oxy))bis(methylene))bis(2,2,5-trimethyl-1,3-dioxane)
¹H NMR (CDCl₃, 400 MHz): δ 5.87 (ddt, J = 5.6, 10.4, 17.6 Hz, 1H), 5.26 (ddd, J = 1.6, 3.2, 17.6 Hz, 1H, one of CH₂), 5.14 (ddd, J = 1.6, 3.2, 10.6 Hz, 1H, one of CH₂), 3.93 (ddd, J = 1.6, 1.6, 5.6 Hz, 2H), 3.75-3.59 (m, 6H), 3.55-3.29 (m, 20H), 1.42 (s, 9H), 1.39 (s, 9H), 0.89 (s, 9H).
¹³C NMR (CDCl₃, 100 MHz): δ 135.3 (CH), 116.4 (CH₂) 97.9 (C×3), 74.4 (CH₂×3), 72.5 (CH₂), 70.4 (CH₂×3), 69.6 (CH₂), 66.8 (CH₂×6), 46.2 (C), 34.7 (C×3), 25.6 (CH₃×3), 22.2 (CH₃×3), 18.7 (C×3).

### [Synthesis of compound H146]

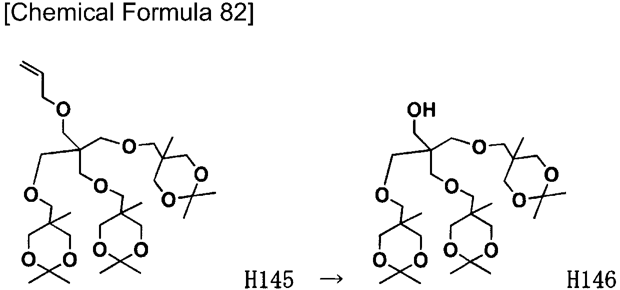

A deallylation was carried out in the same manner as in Synthesis Example 3 to obtain the target compound H146.

Chemical Formula: C₂₉H₅₄O₁₀

3-((2,2,5-trimethyl-1,3-dioxan-5-yl)methoxy)-2,2-bis(((2,2,5-trimethyl-1,3-dioxan-5-yl)methoxy)methyl)propan-1-ol
¹H NMR (CDCl₃, 400 MHz): δ 3.80-3.64 (m, 8H), 3.60-3.45 (m, 10H), 3.41-3.44 (m, 8H), 2.80 (t, J = 6.4 Hz, 1H),1.43 (s, 9H), 1.40 (s, 9H), 0.90 (s, 3H), 0.88 (s, 6H).
¹³C NMR (CDCl₃, 100 MHz): δ 98.0 (C×2), 97.9 (C), 74.8 (CH₂×2), 74.4 (CH2),72.0 (CH2×2), 70.2 (CH2), 66.8 (CH2×2), 66.7 (CH2×4), 66.3 (CH₂), 45.8 (C), 34.7 (C×3), 26.4 (CH₃×2), 25.9 (CH₃), 22.0 (CH₃), 21.5 (CH₃×2) 18.7 (CH₃), 18.5 (CH₃×2).

### Example 8

### [Synthesis of compound H216 : G₁P₂]

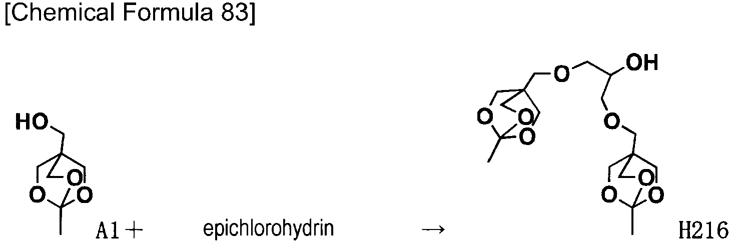

In Synthesis Example 4, A1 was used as R³-OH to obtain the target compound, H216.

Chemical Formula: C₁₇H₂₈O₉

1,3-bis((1-methyl-2,6,7-trioxabicyclo[2.2.2]octan-4-yl)methoxy)propan-2-ol ¹H NMR (CDCl₃, 400 MHz): δ 4.00 (s, 12H), 3.93-3.84 (m, 1H), 3.42 (dd, J = 4.4, 9.6 Hz, 2H), 3.38 (dd, J = 6.0, 10.0 Hz, 2H), 3.27 (d, J = 10.0 Hz, 2H), 3.24 (d, J = 9.6 Hz, 2H), 2.21 (m, 1H), 1.46 (s, 6H).

### [Synthesis of compound H217]

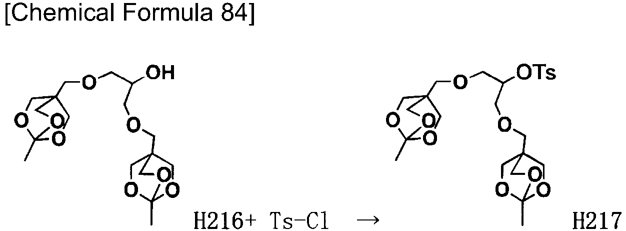

In Synthesis Example 5, H216 was used as R⁴-OH to obtain the target compound, H217. The crude H217 was used as is in the next reaction.

### [Synthesis of compound H218]

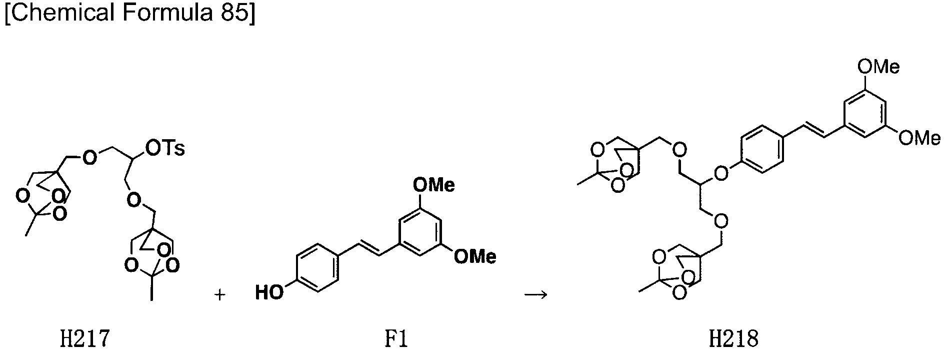

In Synthesis Example 6, item (1), H217 was used as R⁵-OTs to obtain the target compound, H218.

Chemical Formula: C₃₃H₄₂O₁₁

¹H NMR (CDCl₃, 400 MHz): δ 7.44 (d, J = 8.8 Hz, 2H), 7.03 (d, J = 16.0 Hz, 1H), 6.92 (d, J =16.4 Hz, 2H), 6.89 (d, J = 8.8 Hz, 1H), 6.65 (d, J = 2.4 Hz, 2H), 6.39 (brt, J = 2.4 Hz, 1H), 4.50-4.42 (m, 1H), 4.05-3.90 (m, 14H), 3.83 (s, 6H), 3.60-3.53 (m, 2H), 3.24 (s, 4H), 1.45 (s, 6H).
¹³C NMR (CDCl₃, 100 MHz): δ 161.1 (C×2), 157.7 (C), 139.7 (C), 131.1 (C), 128.5 (CH), 128.0 (CH×2), 127.4 (CH), 116.4 (CH×2), 108.7 (C×2), 104.5 (CH×2), 100.0 (CH), 71.0 (CH₂×2), 70.4 (CH₂×2), 69.5 (CH₂×7), 55.5 (CH₃×2), 35.2 (C×2), 23.6 (CH₃×2).

### [Synthesis of compound H219]

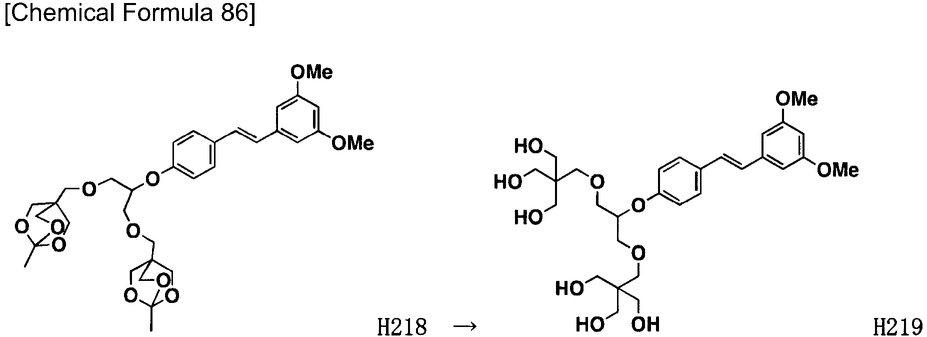

In Synthesis Example 7, item (2), H218 was used as the compound obtained in Synthesis Example 6 to obtain the target compound, H219.

Chemical Formula: C₂₉H₄₂O₁₁

(E)-2,2'-(((2-(4-(3,5-dimethoxystyryl)phenoxy)propane-1,3-diyl)bis(oxy))bis(methylene))bis(2-(hydroxymethyl)propane-1,3-diol)
¹H NMR (CD₃OD, 400 MHz): δ 7.43 (d, J = 8.8 Hz, 2H), 7.04 (d, J = 16.4 Hz, 1H), 6.98 (d, J = 8.8 Hz, 2H), 6.87 (d, J = 16.4 Hz, 1H), 6.65 (d, J = 2.0 Hz, 2H), 6.35 (brt, J = 2.0 Hz, 1H), 4.65-4.57 (m, 1H), 3.77 (s, 6H), 3.72-3.63 (m, 2H), 3.64-3.52 (m, 16H), 3.52-3.43 (m, 2H).
¹³C NMR (CD₃OD, 100 MHz): δ 162.4 (C×2), 159.3 (C), 141.0 (C), 131.9 (C), 129.5 (CH), 128.9 (CH×2), 127.8 (CH), 117.5 (CH×2), 105.3 (CH×2), 100.5 (CH), 77.7 (CH), 72.6 (CH₂), 71.9 (CH₂). 63.2 (CH₂×8), 55.8 (CH₂×2), 46.9 (C×2).

### Example 9

### [Synthesis of compound H246 : G₁T₂]

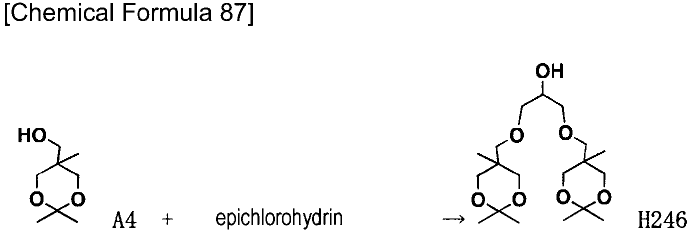

In Synthesis Example 4, A4 was used as R³-OH to obtain the target compound, H246.

Chemical Formula: C₁₉H₃₆O₇

1,3-bis((2,2,5-trimethyl-1,3-dioxan-5-yl)methoxy)propan-2-ol ¹H NMR (CDCl₃, 400 MHz): δ 4.03-3.93 (m, 1H), 3.80-3.30 (m, 16H), 2.57 (d, J = 4.8 Hz, 1H), 1.43 (s, 6H), 1.40 (s, 6H), 0.86 (s, 6H).
¹³C NMR (CDCl₃, 100 MHz): δ 98.1 (C×2), 74.3 (CH₂×2), 72.8 (CH₂×2), 69.6 (CH), 66.6 (CH₂×4), 34.6 (C×2), 27.0 (CH₃×2), 20.8 (CH₃×2), 18.4 (CH₃×2).

### Example 10

### [Synthesis of compound J117 : P₁P₁P₃]

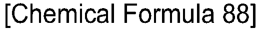

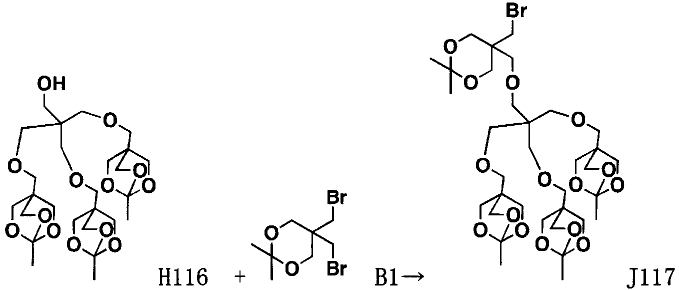

In Synthesis Example 1, H116 was used as R¹-OH to obtain the target compound, J117.

Chemical Formula: C₃₄H₅₅BrO₁₅

4,4'-(((2-(((5-(bromomethyl)-2,2-dimethyl-1,3-dioxan-5-yl)methoxy)methyl)-2-(((1-methyl-2,6,7-trioxabicyclo[2.2.2]octan-4-yl)methoxy)methyl)propane-1,3-diyl)bis(oxy))bis(methylene))bis(1-methyl-2,6,7-trioxabicyclo[2.2.2]octane)
¹H NMR (CDCl₃, 400 MHz): δ 3.97 (s, 18H), 3.70 (s, 4H), 3.50 (s, 2H), 3.36 (s, 2H), 3.28 (s, 2H), 3.23 (s, 6H), 3.13 (s, 6H), 1.45 (s, 9H), 1.41 (s, 6H).
¹³C NMR (CDCl₃, 100 MHz): δ 108.7 (C×3), 98.9 (C), 70.9 (CH₂), 70.1 (CH₂×2), 70.0 (CH₂×3), 69.7 (CH₂), 69.5 (CH₂×10), 64.0 (CH₂×2), 46.0 (C), 38.7 (C), 35.7 (CH₃), 35.3 (C×3), 24.0 (CH₃), 23.6 (CH₃×3), 23.5 (CH₃).

### [Synthesis of compound J118]

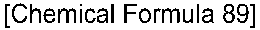

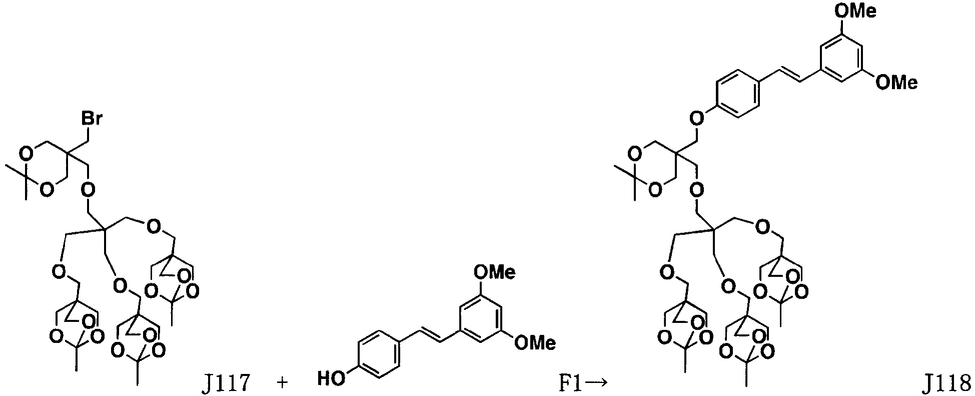

In Synthesis Example 6, item (2), J117 was used as R⁵-X to obtain the target compound, J118. The crude J118 was used as is in the next reaction without purifying by silica gel column chromatography.

### [Synthesis of compound J119]

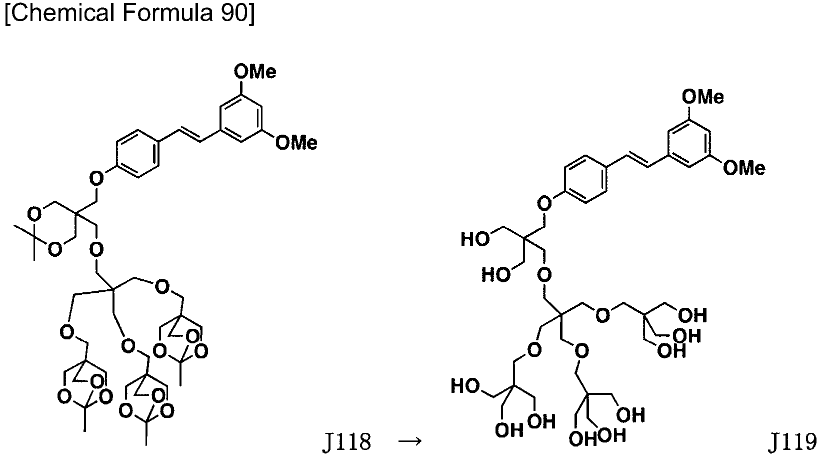

In Synthesis Example 7, item (2), J118 was used as the compound obtained in Synthesis Example 6 to obtain the target compound, J119.

Chemical Formula: C₄₁H₆₆O₁₈

(E)-2,2'-(((2-((3-(4-(3,5-dimethoxystyryl)phenoxy)-2,2-bis(hydroxymethyl)propoxy)methyl)-2-((3-hydroxy-2,2-bis(hydroxymethyl)propoxy)methyl)propane-1,3-diyl)bis(oxy))bis(methylene))bis(2-(hydroxymethyl)propane-1,3-diol)
¹H NMR (CD₃OD, 400 MHz): δ 7.48 (d, J = 8.8 Hz, 2H), 7.08 (d, J = 16.0 Hz, 1H), 6.95 (d, J = 8.8 Hz, 2H), 6.94 (d, J = 16.0 Hz, 1H), 6.67 (d, J = 2.4 Hz, 2H), 6.36 (brt, J = 2.4 Hz, 1H), 3.98 (s, 2H), 3.80 (s, 6H), 3.71 (s, 4H), 3.60-3.55 (m, 18H), 3.38-3.34 (m, 16H).
¹³C NMR (CD₃OD, 100 MHz): δ 162.4 (C×2), 160.6 (C), 141.1 (C), 131.3 (C), 129.7 (CH), 128.9 (CH×2), 127.5 (CH), 115.7 (CH×2), 105.2 (CH×2), 100.5 (CH), 72.6 (CH₂×2), 72.1 (CH₂×2), 71.6 (CH₂), 68.5 (CH₂), 63.5 (CH₂×10), 63.3 (CH₂×2), 63.0 (CH₂×2), 55.8 (CH₃×2), 47.1 (C×2), 46.9 (C×3).

### Example 11

### [Synthesis of compound J127: P₁P₁G₃]

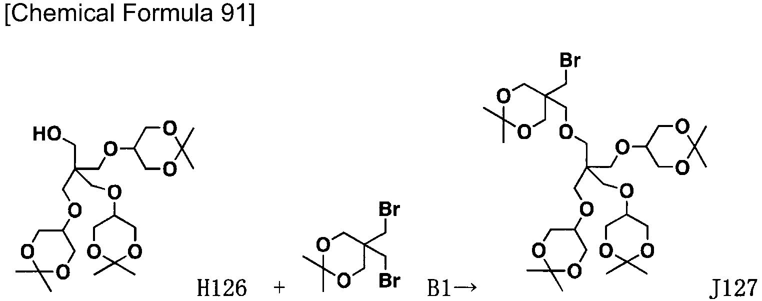

In Synthesis Example 1, H126 was used as R¹-OH to obtain the target compound, J127.

Chemical Formula: C₃₁H₅₅BrO₁₂

5,5'-((2-(((5-(bromomethyl)-2,2-dimethyl-1,3-dioxan-5-yl)methoxy)methyl)-2-(((2,2-dimethyl-1,3-dioxan-5-yl)oxy)methyl)propane-1,3-diyl)bis(oxy))bis(2,2-dimethyl-1,3-dioxane)
¹H NMR (CDCl₃, 400 MHz): δ 4.00-3.88 (m, 6H), 3.80-3.64 (m, 10H), 3.56 (s, 2H), 3.46 (m, 13H), 1.42 (s, 9H), 1.40 (s, 6H), 1.39 (s, 9H).
¹³C NMR (CDCl₃, 100 MHz): δ 98.7 (C), 98.2 (C×3), 70.9 (CH×3), 69.8 (CH₂), 67.4 (CH₂×2), 63.9 (CH₂×2), 62.6 (CH₂×8), 45.6 (C), 38.6 (C), 36.1 (CH₂), 24.6 (CH₃), 24.5 (CH₃×3), 23.0 (CH₃×3), 22.8 (CH₃).

### [Synthesis of compound J128]

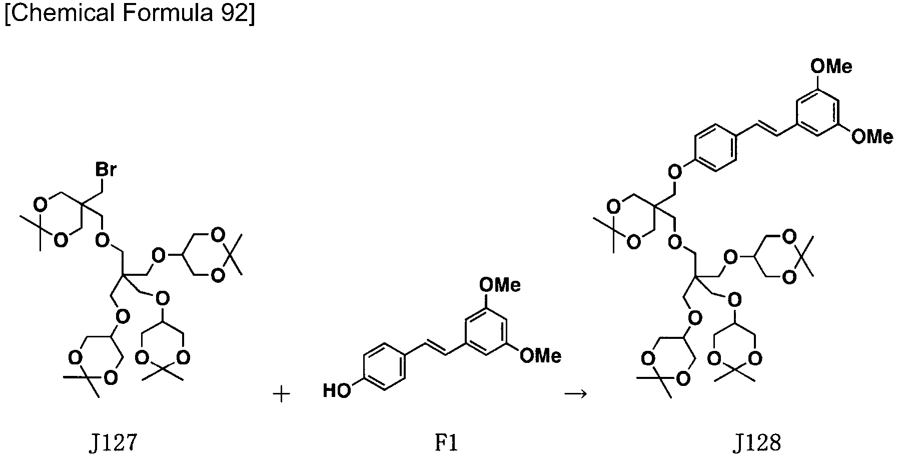

In Synthesis Example 6, item (2), J127 was used as R⁵-X to obtain the target compound, J128.

Chemical Formula: C₄₇H₇₀O₁₅

(E)-5,5'-((2-(((5-((4-(3,5-dimethoxystyryl)phenoxy)methyl)-2,2-dimethyl-1,3-dioxan-5-yl)methoxy)methyl)-2-(((2,2-dimethyl-1,3-dioxan-5-yl)oxy)methyl)propane-1,3-diyl)bis(oxy))bis(2,2-dimethyl-1,3-dioxane)
¹H NMR (CDCl₃, 400 MHz): δ 7.43 (d, J = 8.8 Hz, 2H), 7.02 (d, J = 16.0 Hz, 1H), 6.89 (d, J = 8.8 Hz, 2H), 6.89 (d, J = 16.0 Hz, 1H), 6.63 (brd, J = 2.4 Hz, 2H), 6.38 (brt, J = 2.4 Hz, 1H), 3.99 (s, 2H), 3.88-3.80 (m, 8H), 3.79 (s, 6H), 3.74-3.58 (m, 8H), 3.46-3.30 (m, 10H), 3.25 (m, 3H), 1.43 (s, 3H), 1.41 (s, 3H), 1.38 (s, 9H), 1.35 (s, 9H).
¹³C NMR (CDCl₃, 100 MHz): δ 160.9 (C×2), 158.8 (C), 139.6 (C), 130.0 (C), 128.6 (CH), 127.7 (CH×2), 126.6 (CH), 114.7 (CH×2), 104.2 (CH×2), 99.5 (CH), 98.3 (C), 97.9 (C×3), 70.7 (CH×3), 69.6 (CH₂), 67.8 (CH₂), 67.2 (CH₂×2), 67.0 (CH₂), 62.6 (CH₂), 62.5 (CH₂×2) 62.4 (CH₂×6), 55.2 (CH₃×2), 45.5 (C), 38.8 (C), 24.2(CH₃), 24.0 (CH₃×3), 23.1 (CH₃×3), 23.0 (CH₃).

### [Synthesis of compound J129]

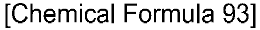

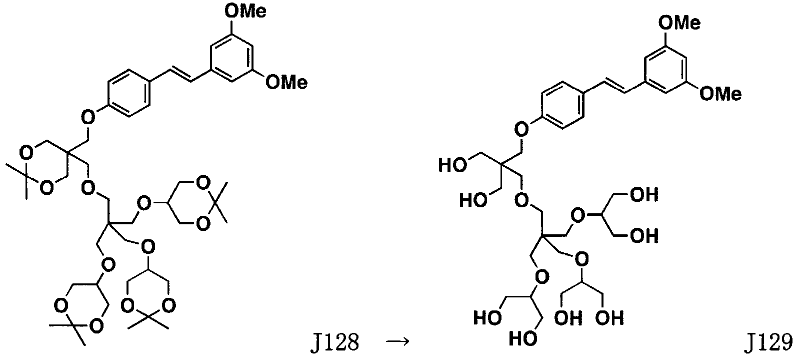

In Synthesis Example 7, item (1), J128 was used as the compound obtained in Synthesis Example 6 to obtain the target compound, J129.

Chemical Formula: C₃₅H₅₄O₁₅
(E)-2,2'-((2-(((1,3-dihydroxypropan-2-yl)oxy)methyl)-2-((3-(4-(3,5-dimethoxystyryl)phenoxy)-2,2-bis(hydroxymethyl)propoxy)methyl)propane-1,3-diyl)bis(oxy))bis(propane-1,3-diol)
¹H NMR (CD₃OD, 400 MHz): δ 7.46 (d, J = 8.8 Hz, 2H), 7.08 (d, J = 16.4 Hz, 1H), 6.94 (d, J = 8.8 Hz, 2H), 6.93 (d, J = 16.4 Hz, 1H), 6.67 (d, J = 2.4 Hz, 2H), 6.36 (brt, J = 2.4 Hz, 1H), 3.97 (s, 2H), 3.78 (s, 6H), 3.70 (s, 4H), 3.78-3.44 (m, 22H), 3.42-3.33 (m, 3H).
¹³C NMR (CD₃OD, 100 MHz): δ 162.4 (C×2), 160.4 (C), 141.1 (C), 131.4 (C), 129.7 (CH), 128.8 (CH×2), 127.5 (CH), 115.7 (CH×2), 105.2 (CH×2), 100.4 (CH), 82.9 (CH×3), 70.0 (CH₂), 69.8 (CH₂×2), 69.7 (CH₂), 67.7 (CH₂), 62.5 (CH₂×2), 62.3 (CH₂×7), 55.7 (CH₃×2), 47.1 (C), 46.9 (C).

### Example 12

### [Synthesis of compound J127': P₁P₁G₃]

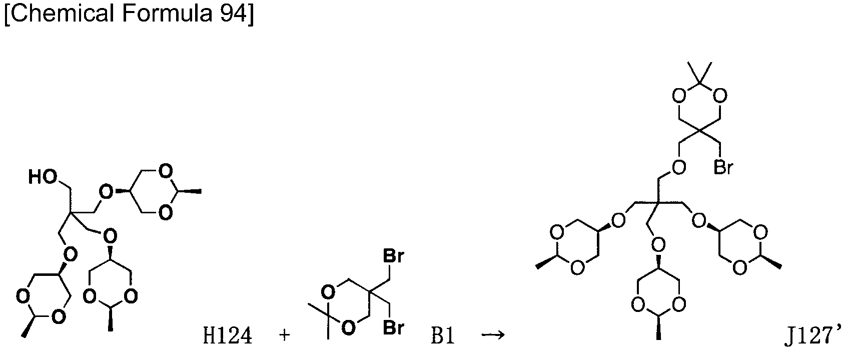

In Synthesis Example 1, H124 was used as R¹-OH to obtain the target compound, J127'.

Chemical Formula: C₂₈H₄₉BrO₁₂

(2S,2'S,5s,5's)-5,5'-((2-(((5-(bromomethyl)-2,2-dimethyl-1,3-dioxan-5-yl)methoxy)methyl)-2-((((2S,5s)-2-methyl-1,3-dioxan-5-yl)oxy)methyl)propane-1,3-diyl)bis(oxy))bis(2-methyl-1,3-dioxane)
¹H NMR (CDCl₃, 400 MHz): δ 4.71 (q, J = 5.2 Hz, 3H), 4.18-4.08 (m, 6H), 3.83-3.68 (m, 10H), 3.60 (s, 2H), 3.59 (s, 8H), 3.39 (s, 2H), 3.20 (m, 3H), 1.40 (s, 3H), 1.40 (s, 3H), 1.34-1.28 (m, 9H).
¹³C NMR (CDCl₃, 100 MHz): δ 98.8 (CH×3), 98.4 (C), 71.1 (CH×3), 70.8 (CH₂), 70.2 (CH₂), 68.4 (CH₂×7), 67.6 (CH₂×2), 63.8 (CH₂×2), 45.7 (C), 38.4 (C), 36.2 (CH₂), 25.0 (CH₃), 22.2 (CH₃), 21.1 (CH₃×3).

### [Synthesis of compound J128']

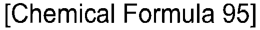

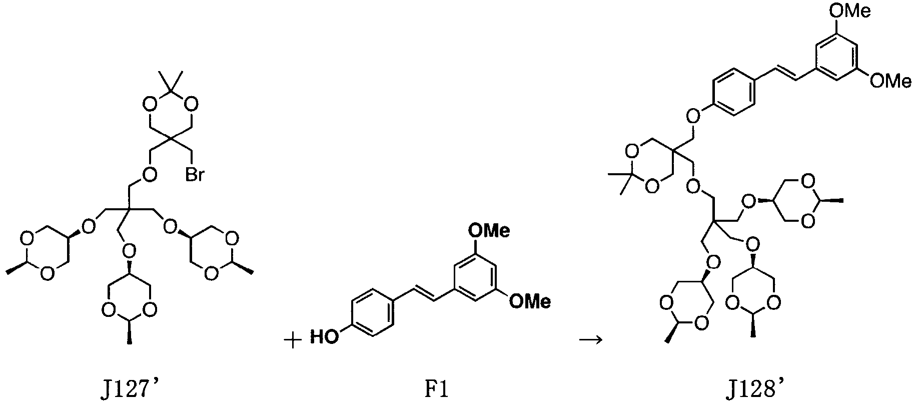

In Synthesis Example 6, item (2), J127' was used as R⁵-X to obtain the target compound, J128'.

Chemical Formula: C₄₄H₆₄O₁₅

(2S,2'S,5s,5's)-5,5'-((2-(((5-((4-((E)-3,5-dimethoxystyryl)phenoxy)methyl)-2,2-dimethyl-1,3-dioxan-5-yl)methoxy)methyl)-2-((((2S,5s)-2-methyl-1,3-dioxan-5-yl)oxy)methyl)propane-1,3-diyl)bis(oxy))bis(2-methyl-1,3-dioxane)
¹H NMR (CDCl₃, 400 MHz): δ 7.42 (d, J = 8.8 Hz, 2H), 7.04 (d, J = 16.0 Hz, 1H), 6.91 (d, J = 16.0 Hz, 1H), 6.89 (d, J = 8.8 Hz, 2H), 6.66 (d, J = 2.4 Hz, 2H), 6.38 (brt, J = 2.4 Hz, 1H), 4.64 (q, J = 5.2 Hz, 3H), 4.12-4.04 (m, 6H), 4.03 (s, 2H), 3.83 (s, 10H), 3.72-3.64 (m, 6H), 3.56 (s, 2H), 3.54 (s, 6H), 3.46 (s, 2H), 3.11-3.04 (m, 3H), 1.44 (s, 3H), 1.42 (s, 3H), 1.29 (d, J = 5.2 Hz, 9H).
¹³C NMR (CDCl₃, 100 MHz): δ 161.0 (C×2), 159.0 (C), 139.8 (C), 130.3 (C), 129.2 (CH), 127.9 (CH×2), 126.6 (CH), 114.8 (CH×2), 104.4 (CH×2), 99.7 (CH), 99.0 (CH×3), 98.5 (C), 71.3 (CH×3), 70.5 (CH₂), 70.3 (CH₂), 68.6 (CH₂×8), 67.8 (CH2×3), 67.1 (CH₂), 62.9 (CH₂×2), 55.5 (CH₃×2), 25.5 (CH₃), 22.2 (CH₃), 21.2 (CH₃×3).

### Example 13

### [Synthesis of compound J137: P₁P₁A₃]

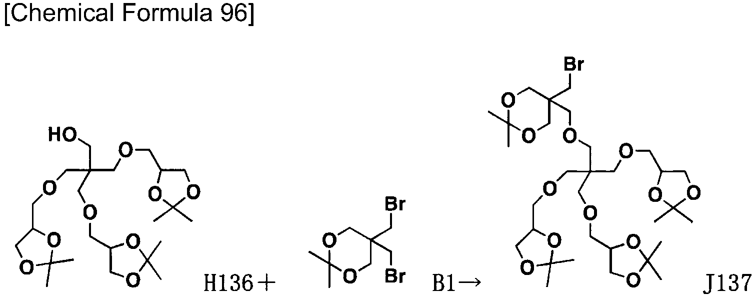

In Synthesis Example 1, H136 was used as R¹-OH to obtain the target compound, J137.

Chemical Formula: C₃₁H₅₅BrO₁₂

5-(bromomethyl)-5-((3-((2,2-dimethyl-1,3-dioxolan-4-yl)methoxy)-2,2-bis(((2,2-dimethyl-1,3-dioxolan-4-yl)methoxy)methyl)propoxy)methyl)-2,2-dimethyl-1,3-dioxane--methane (1/1)
¹H NMR (CDCl₃, 400 MHz): δ 4.23 (quin, J = 6.0 Hz, 3H), 4.03 (dd, J = 6.4 Hz, 3H), 3.84-3.64 (m, 8H), 3.62-3.30 (m, 17H), 1.41 (s, 16H), 1.36 (s, 8H).
¹³C NMR (CDCl₃, 100 MHz): δ 109.3 (C×3), 98.6 (C), 74.7 (CH×3), 72.4 (CH2×2), 70.9 (CH₂), 70.4 (CH₂×2), 70.2 (CH₂), 66.9 (CH₂×4), 63.9 (CH₂×2), 63.7 (CH₂), 45.9 (C), 38.6 (C), 36.0 (CH₂), 26.8 (CH₃×3), 25.6 (CH₃×3), 24.6 (CH₃), 22.7 (CH₃).

### [Synthesis of compound J138]

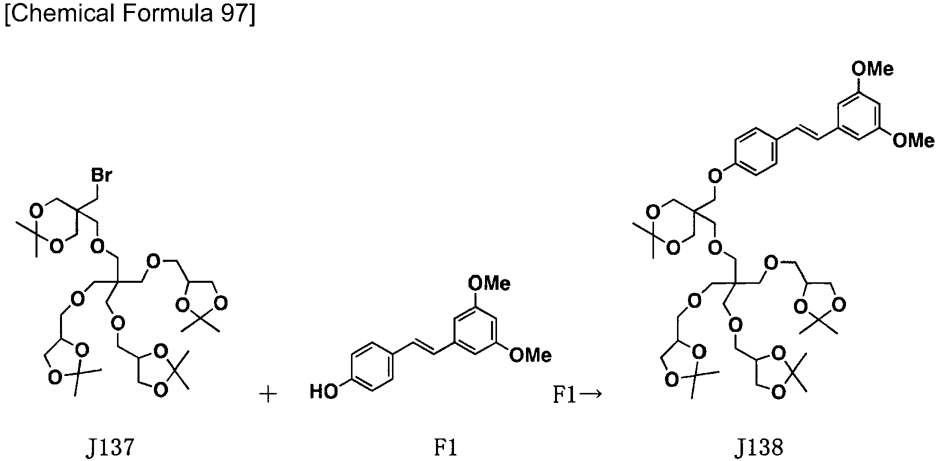

In Synthesis Example 6, item (2), J137 was used as R⁵-X to obtain the target compound, J138.

Chemical Formula: C₄₇H₇₀O₁₅

(E)-5-((4-(3,5-dimethoxystyryl)phenoxy)methyl)-5-((3-((2,2-dimethyl-1,3-dioxolan-4-yl)methoxy)-2,2-bis(((2,2-dimethyl-1,3-dioxolan-4-yl)methoxy)methyl)propoxy)methyl)-2,2-dimethyl-1,3-dioxane ¹H NMR (CDCl₃, 400 MHz): δ 7.43 (d, J = 8.8 Hz, 2H), 7.03 (d, J = 16.0 Hz,
1H), 6.91 (d, J = 16.0 Hz, 1H), 6.90 (d, J = 8.8 Hz, 2H), 6.63 (brd, J = 2.4 Hz, 2H), 6.38 (brt, J = 2.4 Hz, 1H), 4.20-4.12 (m, 3H), 4.02 (s, 2H), 4.02-3.95 (m, 3H), 3.88-3.79 (m, 10H), 3.73-3.65 (m, 3H), 3.48-3.30 (m, 16H), 1.45 (s, 3H), 1.42 (s, 3H), 1.39 (s, 9H), 1.34 (s, 9H).
¹³C NMR (CDCl₃, 100 MHz): δ 161.1 (C×2), 159.0 (C), 139.8 (C), 130.2 (C), 128.8 (CH), 127.9 (CH×2), 126.7 (CH), 114.8 (CH×2), 109.4 (C×3), 104.2 (CH×2), 99.8 (CH), 98.5 (C), 74.7 (CH×3), 72.4 (CH₂×3), 70.8 (CH₂), 70.5 (CH₂×3), 70.2 (CH₂), 67.3 (CH₂), 66.9 (CH₂×4), 62.8 (CH₂), 55.5 (CH₃×2), 46.0 (C), 39.0 (C), 26.9 (CH₃×3), 25.6 (CH₃×3), 25.3 (CH₃), 22.5 (CH₃).

### [Synthesis of compound J139]

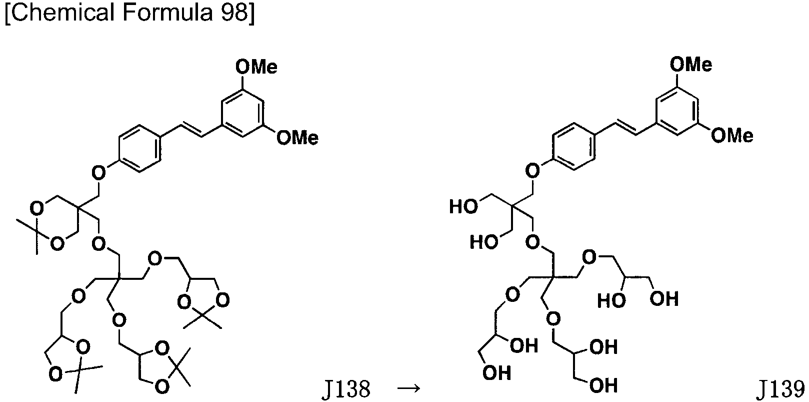

In Synthesis Example 7, item (1), J138 was used as the compound obtained in Synthesis Example 6 to obtain the target compound, J139.

Chemical Formula: C₃₅H₅₄O₁₅

(E)-3,3'-((2-((2,3-dihydroxypropoxy)methyl)-2-((3-(4-(3,5-dimethoxystyryl)phenoxy)-2,2-bis(hydroxymethyl)propoxy)methyl)propane-1,3-diyl)bis(oxy))bis(propane-1,2-diol)
¹H NMR (CD₃OD, 400 MHz): δ 7.47 (d, J = 8.8 Hz, 2H), 7.08 (d, J = 16.0 Hz, 1H), 6.94 (d, J = 8.8 Hz, 2H), 6.93 (d, J = 16.0 Hz, 1H), 6.67 (d, J = 2.4 Hz, 2H), 6.36 (brt, J = 2.4 Hz, 1H), 3.97 (s, 2H), 3.79 (s, 6H), 3.76-3.65 (m, 6H), 3.64-3.33 (m, 23H).
¹³C NMR (CD₃OD, 100 MHz): δ 162.4 (C×2), 160.5 (C), 141.1 (C), 131.4 (C), 129.7 (CH), 128.9 (CH×2), 127.5 (CH), 115.7 (CH×2), 105.2 (CH×2), 100.4 (CH), 73.9 (CH₂×4), 72.0 (CH×3), 71.7 (CH₂×2), 71.5 (CH₂), 67.8 (CH₂), 64.5 (CH₂×4), 62.6 (CH₂×2), 55.8 (CH₃×2), 46.8 (C), 46.8 (C).

### Example 14

### [Synthesis of compound J147: P₁P₁T₃]

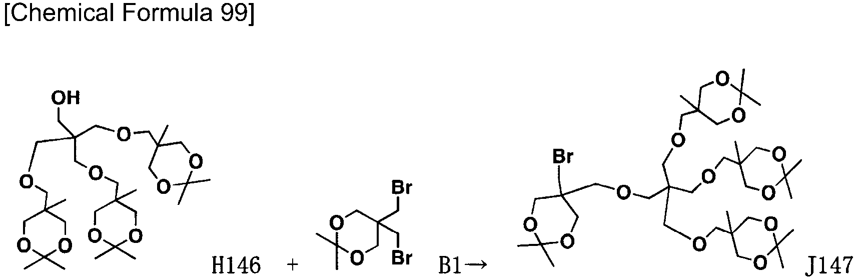

In Synthesis Example 1, H146 was used as R¹-OH to obtain the target compound, J147.

Chemical Formula: C₃₇H₆₇BrO₁₂

5,5'-(((2-(((5-(bromomethyl)-2,2-dimethyl-1,3-dioxan-5-yl)methoxy)methyl)-2-(((2,2,5-trimethyl-1,3-dioxan-5-yl)methoxy)methyl)propane-1,3-diyl)bis(oxy))bis(methylene))bis(2,2,5-trimethyl-1,3-dioxane)
¹H NMR (CDCl₃, 400 MHz): δ 3.73-3.63 (m, 12H), 3.57-3.49 (m, 10H),3.47 (s, 6H), 3.39 (s, 6H), 1.42 (s, 12H), 1.39 (s, 12H), 0.87 (s, 9H).
¹³C NMR (CDCl₃, 100 MHz): δ 98.0 (C×4), 74.6 (CH₂×4), 71.8 (CH₂×4), 66.6 (CH₂×8), 65.9 (CH₂), 63.3 (CH₂), 45.7 (C), 35.1 (CH₂), 34.6 (C×3), 26.4 (CH₃×4), 21.3 (CH₃×4), 18.5 (CH₃×3).

### [Synthesis of compound J148]

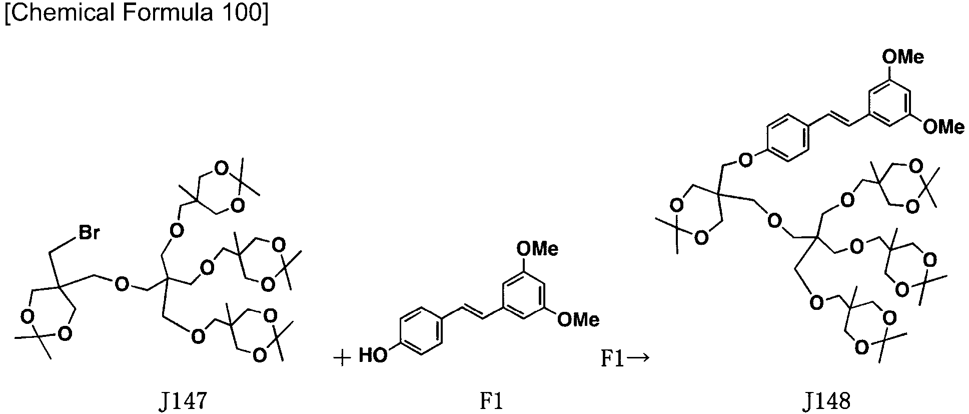

In Synthesis Example 6, item (2), J147 was used as R⁵-X to obtain the target compound, J148.

Chemical Formula: C₅₃H₈₂O₁₅

(E)-5,5'-(((2-(((5-((4-(3,5-dimethoxystyryl)phenoxy)methyl)-2,2-dimethyl-1,3-dioxan-5-yl)methoxy)methyl)-2-(((2,2,5-trimethyl-1,3-dioxan-5-yl)methoxy)methyl)propane-1,3-diyl)bis(oxy))bis(methylene))bis(2,2,5-trimethyl-1,3-dioxane)
¹H NMR (CDCl₃, 400 MHz): δ 7.43 (d, J = 8.4 Hz, 2H), 7.03 (d, J = 16.0 Hz, 1H), 6.91 (d, J = 8.4 Hz, 2H), 6.90 (d, J = 16.0 Hz, 1H), 6.63 (d, J = 2.4 Hz, 2H), 6.38 (brt, J = 2.4 Hz, 1H), 4.05 (s, 2H), 3.91-3.78 (m, 4H), 3.83 (s, 6H), 3.66 (d, J = 11.6 Hz, 6H), 3.48 (d, J = 11.6 Hz, 6H), 3.42 (s, 2H), 3.39 (s, 2H), 3.35 (s, 6H), 3.27 (s, 6H), 1.45 (s, 3H), 1.42 (s, 3H), 1.41 (s, 9H), 1.38 (s, 9H), 0.85 (s, 9H).
¹³C NMR (CDCl₃, 100 MHz): δ 161.1 (C×2), 159.1 (C), 139.8 (C), 130.1 (C), 128.9 (CH), 127.9 (CH×2), 126.7 (CH), 114.9 (CH×2), 104.2 (CH×2), 99.8 (CH), 98.5 (C), 97.9 (C×3), 74.4 (CH₂×3), 71.0 (CH₂), 70.7 (CH₂), 70.5 (CH₂×3), 67.4 (CH2), 66.7 (CH₂×6), 62.8 (CH₂×2), 55.5 (CH₃×2), 46.5 (C), 39.0 (C), 34.7 (C×3), 25.9 (CH₃×3), 25.7 (CH₃), 22.2 (CH₃), 22.0 (CH₃×3), 18.6 (CH₃×3).

### [Synthesis of compound J149]

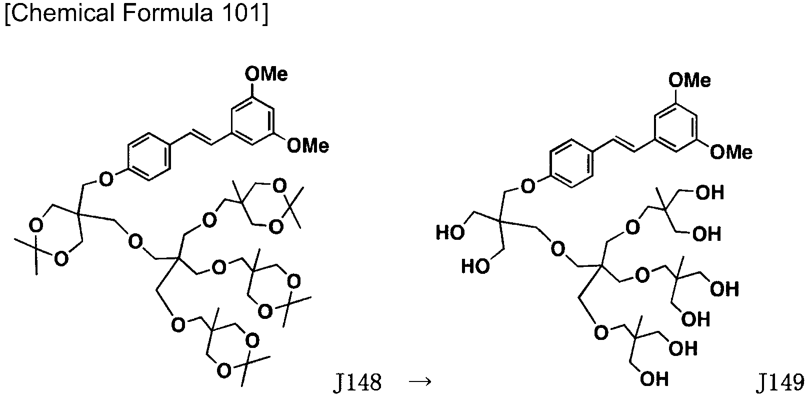

In Synthesis Example 7, item (1), J148 was used as the compound obtained in Synthesis Example 6 to obtain the target compound, J149.

Chemical Formula: C₄₁H₆₆O₁₅

(E)-2,2'-(((2-((3-(4-(3,5-dimethoxystyryl)phenoxy)-2,2-bis(hydroxymethyl)propoxy)methyl)-2-((3-hydroxy-2-(hydroxymethyl)-2-methylpropoxy)methyl)propane-1,3-diyl)bis(oxy))bis(methylene))bis(2-methylpropane-1,3-diol)
¹H NMR (CD₃OD, 400 MHz): δ 7.48 (d, J = 8.8 Hz, 2H), 7.08 (d, J = 16.0 Hz, 1H), 6.94 (d, J = 16.0 Hz, 1H), 6.94 (d, J = 8.8 Hz, 2H), 6.67 (d, J = 2.0 Hz, 2H), 6.36 (brt, J = 2.0 Hz, 1H), 3.98 (s, 2H), 3.79 (s, 6H), 3.70 (s, 4H), 3.51-3.33 (m, 22H), 3.21 (s, 6H), 0.84 (s, 9H).
¹³C NMR (CD₃OD, 100 MHz): δ 162.4 (C×2), 160.5 (C), 141.1 (C), 131.4 (C), 129.7 (CH), 128.9 (CH×2), 127.6 (CH), 115.8 (CH×2), 105.3 (CH×2), 100.4 (CH), 75.5 (CH₂×3), 71.9 (CH₂), 71.8 (CH₂×2), 71.4 (CH₂), 67.8 (CH₂), 66.5 (CH₂×7), 62.5 (CH₂×2), 55.7 (CH₃×2), 47.1 (C), 47.0 (C), 42.6 (C×3), 17.2 (CH₃×3).

### Example 15

### [Synthesis of compound J217: P₁G₁P₂]

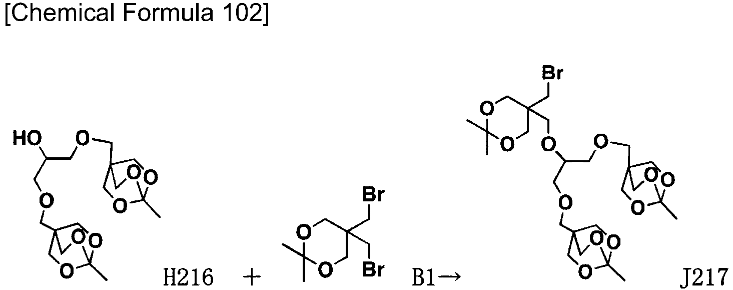

In Synthesis Example1, H216 was used as R¹-OH to obtain the target compound, J217.

Chemical Formula: C₂₅H₄₁BrO₁₁

1,3-bis((1-methyl-2,6,7-trioxabicyclo[2.2.2]octan-4-yl)methoxy)propan-2-ol ¹H NMR (CDCl₃, 400 MHz): δ 3.99 (s, 12H), 3.71 (dd, J = 12.0, 18.8 Hz, 4H), 3.56 (s, 2H), 3.53 (quin, J = 4.8 Hz, 1H), 3.50 (s, 2H), 3.40 (s, 2H), 3.39 (s, 2H), 3.21 (s, 4H), 1.46 (s, 6H), 1.41 (s, 3H), 1.40 (s, 3H).
¹³C NMR (CDCl₃, 100 MHz): δ 108.7 (C×2), 98.8 (C), 78.3 (CH), 71.3 (CH₂×2), 70.1 (CH₂×2), 69.5 (CH₂×6), 69.4 (CH₂), 63.9 (CH₂×2), 38.5 (C), 35.7 (CH₂), 35.1 (C×2), 24.2 (CH₃), 23.6 (CH₃×2), 23.3 (CH₃).

### [Synthesis of compound J218]

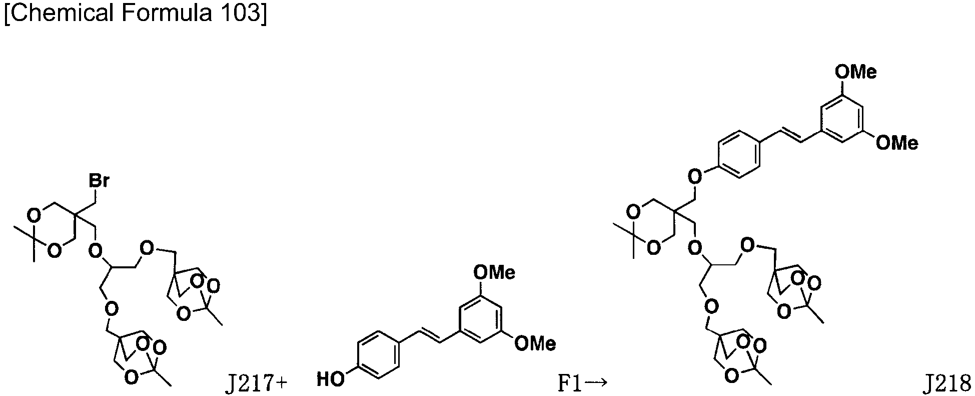

In Synthesis Example 6, item (2), J217 was used as R⁵-X to obtain the target compound, J218.

Chemical Formula: C₄₁H₅₆O₁₄

¹H NMR (CDCl₃, 400 MHz): δ 7.43 (d, J = 8.8 Hz, 2H), 7.04 (d, J = 16.0 Hz, 1H), 6.91 (d, J = 8.8 Hz, 2H), 6.90 (d, J = 16.0 Hz, 1H), 6.65 (d, J = 2.4 Hz, 2H), 6.37 (brt, J = 2.4 Hz, 1H), 4.05 (s, 2H), 3.92-3.78 (m, 10H), 3.70-3.63 (m, 6H), 3.60 (quin, J = 5.2 Hz, 1H), 3.54-3.43 (m, 8H), 3.43-3.34 (m, 4H),
1.44 (s, 3H), 1.42 (s, 3H), 1.41 (s, 6H), 1.38 (s, 6H), 0.83 (s, 6H).
¹³C NMR (CDCl₃, 100 MHz): δ 161.0 (C×2), 159.0 (C), 139.8 (C), 130.0 (C), 128.9 (CH), 127.8 (CH×2), 126.6 (CH), 114.9 (CH×2), 104.4 (CH×2), 99.7 (CH), 98.4 (C), 97.9 (C×2), 78.7 (CH), 74.4 (CH₂×2), 71.3 (CH₂×2), 69.8 (CH₂), 67.3 (CH2), 66.6 (CH₂×2), 66.6 (CH₂×2), 62.7 (CH₂×2), 55.5 (CH₃×2), 38.9 (C), 34.5 (C×2), 26.5 (CH₃×2), 25.2 (CH₃), 22.6 (CH₃), 21.3 (CH₃×2), 18.4 (CH₃×2).

### [Synthesis of compound J219]

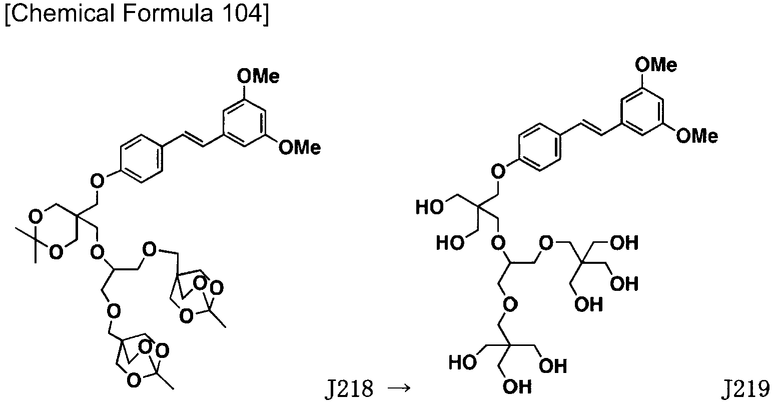

In Synthesis Example 7, item (2), J218 was used as the compound obtained in Synthesis Example 6 to obtain the target compound, J219.

Chemical Formula: C₃₄H₅₂O₁₄

(E)-2,2'-(((2-(3-(4-(3,5-dimethoxystyryl)phenoxy)-2,2-bis(hydroxymethyl)propoxy)propane-1,3-diyl)bis(oxy))bis(methylene))bis(2-(hydroxymethyl)propane-1,3-diol)
¹H NMR (CD₃OD, 400 MHz): δ 7.48 (d, J = 8.8 Hz, 2H), 7.09 (d, J = 16.4 Hz, 1H), 6.95 (d, J = 8.8 Hz, 2H), 6.94 (d, J = 16.4 Hz, 1H), 6.67 (d, J = 2.0 Hz, 2H), 6.36 (brt, J = 2.0 Hz, 1H), 3.99 (s, 2H), 3.80 (s, 6H), 3.76-3.72 (m, 2H), 3.72-3.69 (m, 4H), 3.62-3.54 (m, 13H), 3.54-3.48 (m, 4H),3.47-3.42 (m, 4H).
¹³C NMR (CD₃OD, 100 MHz): δ 162.4 (C×2), 160.4 (C), 141.1 (C), 131.4 (C), 129.7 (CH), 128.8 (CH×2), 127.5 (CH), 115.8 (CH×2), 105.3 (CH×2), 100.4 (CH), 79.6 (CH), 72.3 (CH₂×2), 72.1 (CH₂×2), 63.1 (CH₂×8), 62.7 (CH₂×2), 55.8 (CH₃×2), 46.8 (C×2), 46.8 (C).

### Example 16

### [Synthesis of compound J227: P₁G₁G₂]

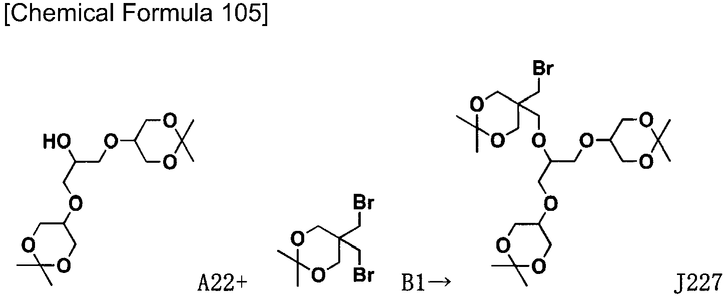

In Synthesis Example 1, A22 was used as R¹-OH to obtain the target compound, J227.

Chemical Formula: C₂₃H₄₁BrO₉

5,5'-((2-((5-(bromomethyl)-2,2-dimethyl-1,3-dioxan-5-yl)methoxy)propane-1,3-diyl)bis(oxy))bis(2,2-dimethyl-1,3-dioxane)
¹H NMR (CDCl₃, 400 MHz): δ 3.96 (dd, J = 4.0, 12.0 Hz, 4H), 3.82-3.68 (m, 8H), 3.63-3.50 (m, 9H), 3.44 (m, 2H), 1.44-1.39 (m, 18H).
¹³C NMR (CDCl₃, 100 MHz): δ 98.5 (C), 98.2 (C×2), 78.8 (CH), 71.0 (CH×2), 69.9 (CH), 68.7 (CH₂×2), 63.8 (CH₂×2), 62.6 (CH₂×2), 62.5 (CH₂×2), 38.5 (C), 36.0 (CH₂), 24.2 (CH₃×2), 24.2 (CH₃), 23.1 (CH₃), 23.1 (CH₃×2).

### [Synthesis of compound J228]

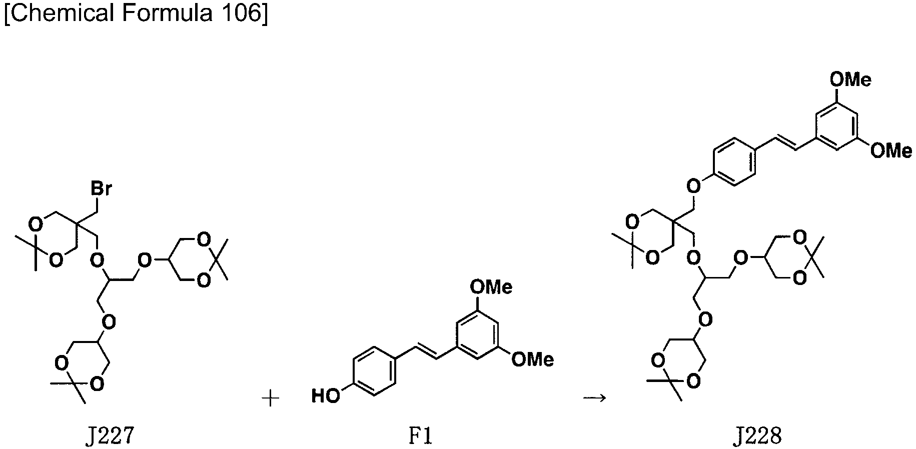

In Synthesis Example 6, item (2), J227 was used as R⁵-X to obtain the target compound, J228.

Chemical Formula: C₃₉H₅₆O₁₂

(*E*)-5,5'-((2-((5-((4-(3,5-dimethoxystyryl)phenoxy)methyl)-2,2-dimethyl-1,3-dioxan-5-yl)methoxy)propane-1,3-diyl)bis(oxy))bis(2,2-dimethyl-1,3-dioxane)
¹H NMR (CDCl₃, 400 MHz): *δ* 7.43 (d, *J* = 8.8 Hz, 2H), 7.03 (d, *J* = 16.4 Hz, 1H), 6.91 (d, *J* = 8.8 Hz, 1H), 6.90 (d, *J* = 16.4 Hz, 2H), 6.64 (d, *J* = 2.4 Hz, 2H), 6.37 (brt, *J* = 2.4 Hz, 1H), 4.02 (s, 2H), 3.92-3.76 (m, 14H), 3.72-3.60 (m, 6H), 3.58-3.44 (m, 5H), 3.40-3.31 (m, 2H), 1.44 (s, 3H), 1.42 (s, 3H), 1.39 (s, 6H), 1.36 (s, 6H).
¹³C NMR (CDCl₃, 100 MHz): *δ* 161.0 (C×2), 158.9 (C), 139.8 (C), 130.2 (C), 128.8 (CH), 127.9 (CH×2), 126.7 (CH), 114.8 (CH×2), 104.4 (CH×2), 99.7 (CH), 98.5 (C), 98.3 (C×2), 79.1 (CH), 71.1 (CH×2), 69.6 (CH₂), 68.6 (CH₂×2), 67.1 (CH2), 62.7 (CH₂×2), 62.7 (CH₂×2), 62.6 (CH₂×2), 55.4 (CH₃×2), 39.0 (C), 24.7 (CH₃), 24.2 (CH₃×2), 23.2 (CH₃×2), 23.1 (CH₃).

### [Synthesis of compound J229]

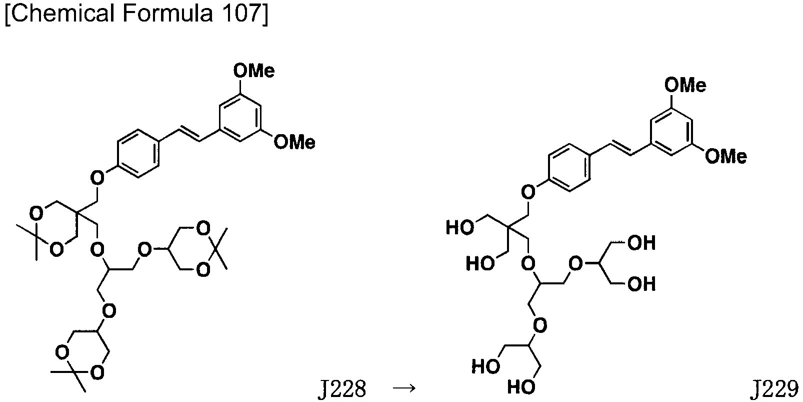

In Synthesis Example 7, item (1), J228 was used as the compound obtained in Synthesis Example 6 to obtain the target compound, J229.

Chemical Formula: C₃₀H₄₄O₁₂

(E)-2,2'-((2-(3-(4-(3,5-dimethoxystyryl)phenoxy)-2,2-bis(hydroxymethyl)propoxy)propane-1,3-diyl)bis(oxy))bis(propane-1,3-diol)
¹H NMR (CD₃OD, 400 MHz): δ 7.42 (d, J = 8.4 Hz, 2H), 7.08 (d, J = 16.0 Hz, 1H), 6.95 (d, J = 16.0 Hz, 1H), 6.94 (d, J = 8.4 Hz, 2H), 6.67 (d, J = 2.0 Hz, 2H), 6.36 (brt, J = 2.0 Hz, 1H), 3.98 (s, 2H), 3.79 (s, 6H), 3.78-3.37 (m, 21H).
¹³C NMR (CD₃OD, 100 MHz): δ 162.3 (C×2), 160.3 (C), 140.9 (C), 131.3 (C), 129.6 (CH), 128.8 (CH×2), 127.4 (CH), 115.7 (CH×2), 105.3 (CH×2), 100.4 (CH), 82.9 (CH×2), 80.4 (CH), 70.5 (CH₂×2), 70.3 (CH₂), 67.6 (CH₂), 63.1 (CH₂×3), 62.4 (CH₂×3), 55.7 (CH₃×2), 47.8 (C).

### Example 17

### [Synthesis of compound J237: P₁G₁A₂]

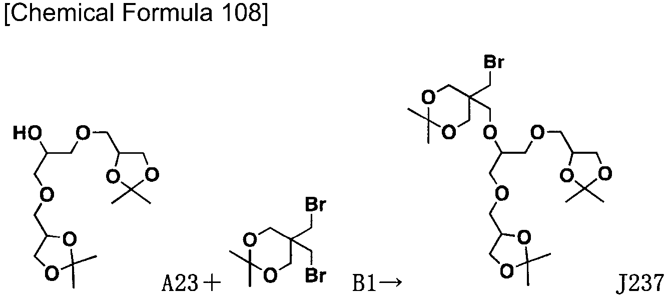

In Synthesis Example 1, A23 was used as R¹-OH to obtain the target compound, J237.

Chemical Formula: C₂₃H₄₁BrO₉

5-(((1,3-bis((2,2-dimethyl-1,3-dioxolan-4-yl)methoxy)propan-2-yl)oxy)methyl)-5-(bromomethyl)-2,2-dimethyl-1,3-dioxane
¹H NMR (CDCl₃, 400 MHz): δ 4.30-4.20 (s, 2H), 4.09-4.01 (m, 2H), 3.83-3.43 (m, 19H), 1.42 (s, 6H), 1.41 (s, 6H), 1.36 (s, 6H).
¹³C NMR (CDCl₃, 100 MHz): δ 109.5 (C×2), 98.7 (C), 78.8 (CH), 74.8 (CH₂×2), 72.6 (CH₂×2), 71.7 (CH₂), 71.5 (CH₂), 70.0 (CH₂), 66.9 (CH₂×2), 63.9 (CH₂×2), 38.7 (C), 36.2 (CH₂), 26.9 (CH₃×2), 25.6 (CH₃×2), 24.4 (CH₃), 23.1 (CH₃).

### [Synthesis of compound J238]

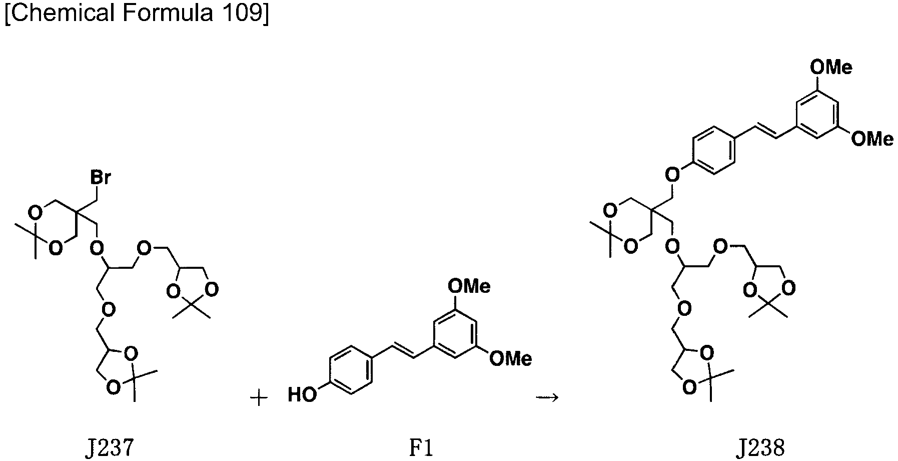

In Synthesis Example 6, item (2), J237 was used as R⁵-X to obtain the target compound, J238.

Chemical Formula: C₃₉H₅₆O₁₂

(E)-5-(((1,3-bis((2,2-dimethyl-1,3-dioxolan-4-yl)methoxy)propan-2-yl)oxy)methyl)-5-((4-(3,5-dimethoxystyryl)phenoxy)methyl)-2,2-dimethyl-1,3-dioxane
¹H NMR (CDCl₃, 400 MHz): δ 7.43 (d, J = 8.8 Hz, 2H), 7.03 (d, J = 16.8 Hz, 1H), 6.91 (d, J = 8.8 Hz, 2H), 6.90 (d, J = 16.8 Hz, 1H), 6.64 (d, J = 2.4 Hz, 2H), 6.37 (brt, J = 2.4 Hz, 1H), 4.24-4.13 (m, 2H), 4.03 (s, 2H), 4.01-3.94 (m, 2H), 3.88-3.77 (m, 12H), 3.72-3.62 (m, 3H), 3.62-3.36 (m, 8H), 1.44 (s, 3H), 1.43 (s, 3H), 1.40 (s, 6H), 1.34 (s,6H).
¹³C NMR (CDCl₃, 100 MHz): δ 161.0 (C×2), 159.0 (C), 139.7 (C), 130.1 (C), 128.8 (CH), 127.8 (CH×2), 126.7 (CH), 114.8 (CH×2), 109.4 (C×2), 104.4 (CH×2), 99.7 (CH), 98.4 (C), 78.8 (CH), 74.7 (CH×2), 72.6 (CH₂×2), 71.6 (CH₂), 71.4 (CH₂), 69.6 (CH₂), 67.1 (CH₂), 66.7 (CH₂×4), 62.7 (CH₂×2), 55.5 (CH₃×2), 39.0 (C), 26.9 (CH₃×2), 25.5 (CH₃×2), 24.9 (CH₃), 22.9 (CH₃).

### [Synthesis of compound J239]

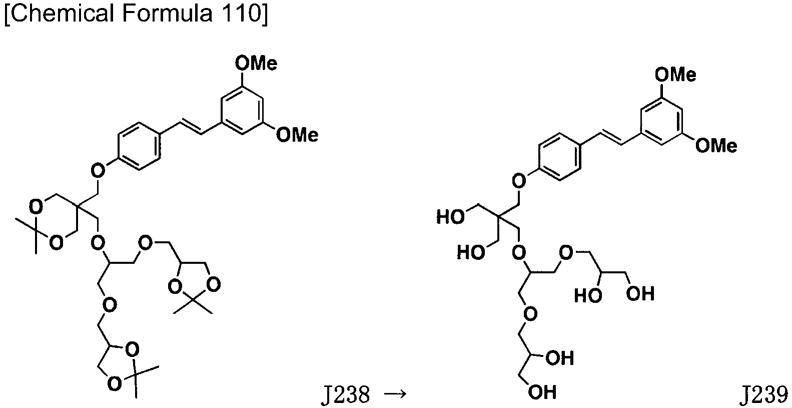

In Synthesis Example 7, item (1), J238 was used as the compound obtained in Synthesis Example 6 to obtain the target compound, J239.

Chemical Formula: C₃₀H₄₄O₁₂

(E)-3,3'-((2-(3-(4-(3,5-dimethoxystyryl)phenoxy)-2,2-bis(hydroxymethyl)propoxy)propane-1,3-diyl)bis(oxy))bis(propane-1,2-diol)
¹H NMR (CD₃OD, 400 MHz): δ 7.46 (d, J = 8.8 Hz, 2H), 7.07 (d, J = 16.0 Hz, 1H), 6.94 (d, J = 8.8 Hz, 2H), 6.93 (d, J = 16.0 Hz, 1H), 6.67 (d, J = 2.0 Hz, 2H), 6.36 (brt, J = 2.0 Hz, 1H), 3.98 (s, 2H), 3.79 (s, 6H), 3.77-3.66 (m, 6H), 3.65-3.40 (m, 15H).
¹³C NMR (CD₃OD, 100 MHz): δ 162.4 (C×2), 160.4 (C), 141.1 (C), 131.4 (C), 129.7 (CH), 128.8 (CH×2), 127.5 (CH), 115.8 (CH×2), 105.3 (CH×2), 100.5 (CH), 79.9 (CH×2), 73.8 (CH₂×2), 72.1 (CH₂×2), 70.6 (CH), 67.7 (CH₂), 64.4 (CH₂×3), 62.6 (CH₂×2), 55.8 (CH₃×2), 46.8 (C).

### Example 18

### [Synthesis of compound J247: P₁G₁T₂]

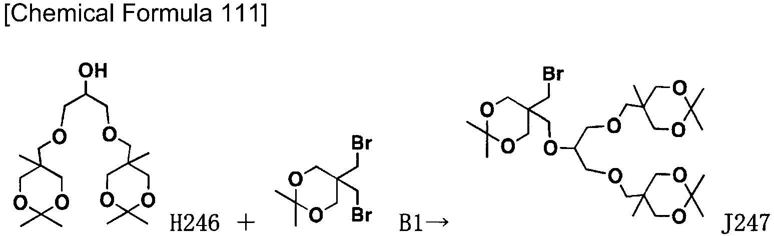

In Synthesis Example 1, H246 was used as R¹-OH to obtain the target compound, J247.

Chemical Formula: C₂₇H₄₉BrO₉

5,5'-(((2-((5-(bromomethyl)-2,2-dimethyl-1,3-dioxan-5-yl)methoxy)propane-1,3-diyl)bis(oxy))bis(methylene))bis(2,2,5-trimethyl-1,3-dioxane)
¹H NMR (CDCl₃, 400 MHz): δ 3.81 (d, J = 12.4 Hz, 2H), 3.72 (d, J = 12.4 Hz, 2H), 3.73-3.39 (m, 21H), 1.43 (s, 6H), 1.40 (s, 6H), 1.39 (s, 6H), 0.87 (s, 6H). ¹³C NMR (CDCl₃, 100 MHz): δ 98.3 (C), 97.7 (C×2), 78.5 (CH), 74.2 (CH₂×2), 71.5 (CH₂×2), 69.8 (CH₂), 66.4 (CH₂×4), 63.7 (CH₂×2), 38.4 (C), 36.1 (CH₂), 34.3 (C×2), 26.4 (CH₃×2), 24.6 (CH₃), 22.6 (CH₃), 21.1 (CH₃×2), 18.2 (CH₃×2).

### [Synthesis of compound J248]

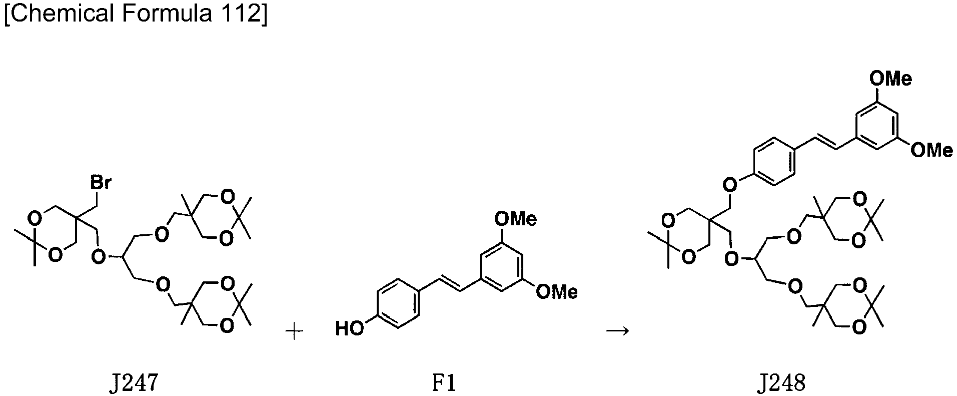

In Synthesis Example 6, item (2), J247 was used as R⁵-X to obtain the target compound, J248.

Chemical Formula: C₄₃H₆₄O₁₂

¹H NMR (CDCl₃, 400 MHz): δ 7.43 (d, J = 8.8 Hz, 2H), 7.04 (d, J = 16.0 Hz, 1H), 6.91 (d, J = 8.8 Hz, 2H), 6.90 (d, J = 16.0 Hz, 1H), 6.65 (d, J = 2.4 Hz, 2H), 6.37 (brt, J = 2.4 Hz, 1H), 4.05 (s, 2H), 3.92-3.78 (m, 10H), 3.70-3.63 (m, 6H), 3.60 (quin, J = 5.2 Hz, 1H), 3.54-3.43 (m, 8H), 3.43-3.34 (m, 4H), 1.44 (s, 3H), 1.42 (s, 3H), 1.41 (s, 6H), 1.38 (s, 6H), 0.83 (s, 6H).
¹³C NMR (CDCl₃, 100 MHz): δ 161.0 (C×2), 159.0 (C), 139.8 (C), 130.0 (C), 128.9 (CH), 127.8 (CH×2), 126.6 (CH), 114.9 (CH×2), 104.4 (CH×2), 99.7 (CH), 98.4 (C), 97.9 (C×2), 78.7 (CH), 74.4 (CH₂×2), 71.3 (CH₂×2), 69.8 (CH₂), 67.3 (CH₂), 66.6 (CH₂×2), 66.6 (CH₂×2), 62.7 (CH₂×2), 55.5 (CH₃×2), 38.9 (C), 34.5 (C×2), 26.5 (CH₃×2), 25.2 (CH₃), 22.6 (CH₃), 21.3 (CH₃×2), 18.4 (CH₃×2).

### [Synthesis of compound J249]

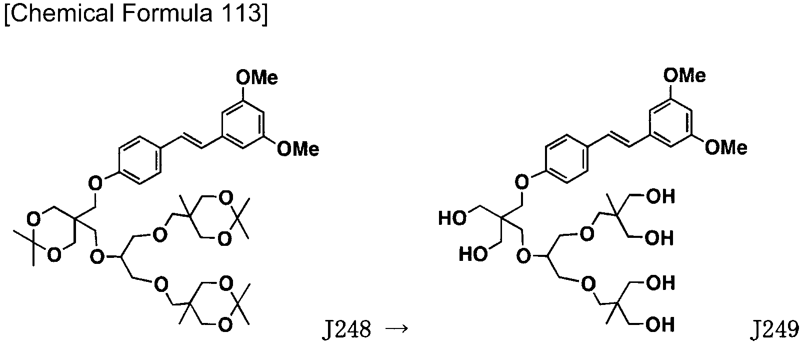

In Synthesis Example 7, item (1), J248 was used as the compound obtained in Synthesis Example 6 to obtain the target compound, J249.

Chemical Formula: C₃₄H₅₂O₁₂

(E)-2,2'-(((2-(3-(4-(3,5-dimethoxystyryl)phenoxy)-2,2-bis(hydroxymethyl)propoxy)propane-1,3-diyl)bis(oxy))bis(methylene))bis(2-methylpropane-1,3-diol)
¹H NMR (CD₃OD, 400 MHz): δ 7.46 (d, J = 8.8 Hz, 2H), 7.07 (d, J = 16.4 Hz, 1H), 6.94 (d, J = 8.8 Hz, 2H), 6.93 (d, J = 16.4 Hz, 1H), 6.67 (d, J = 2.0 Hz, 2H), 6.36 (brt, J = 2.0 Hz, 1H), 3.99 (s, 2H), 3.79 (s, 6H), 3.76-3.69 (m, 6H), , 3.66-3.54 (m, 1H), 3.51-3.46 (m, 4H), 3.46-3.41 (m, 8H), 3.33-3.30 (m, 4H), 0.85 (s, 6H).
¹³C NMR (CD₃OD, 100 MHz): δ 162.4 (C×2), 160.5 (C), 141.1 (C), 131.4 (C), 129.7 (CH), 128.8 (CH×2), 127.5 (CH), 115.8 (CH×2), 105.3 (CH×2), 100.4 (CH), 79.8 (CH), 75.4 (CH₂×2), 72.3 (CH₂×2), 70.7 (CH₂), 67.7 (CH₂), 66.4 (CH₂×3), 66.3 (CH₂×3), 55.7 (CH₃×2), 46.8 (C), 42.5 (C×2), 17.1 (CH₃×2).

### Example 19

### [Evaluation of water solubility]

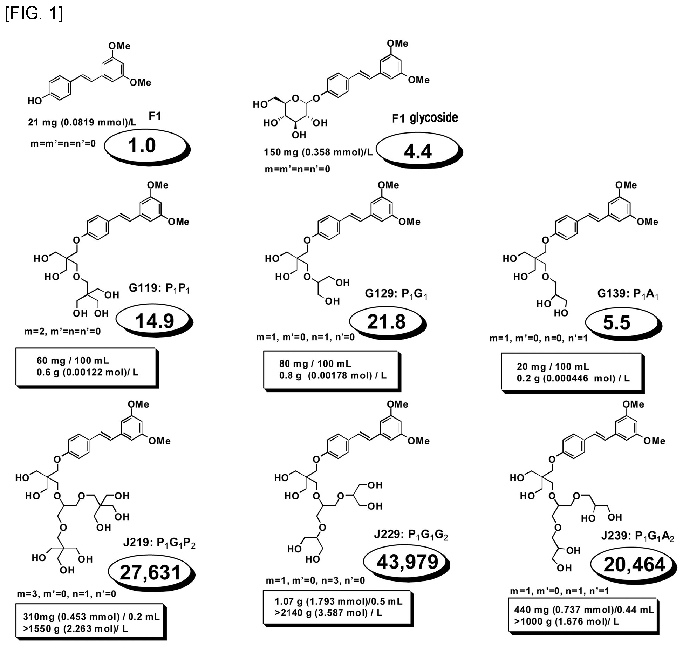

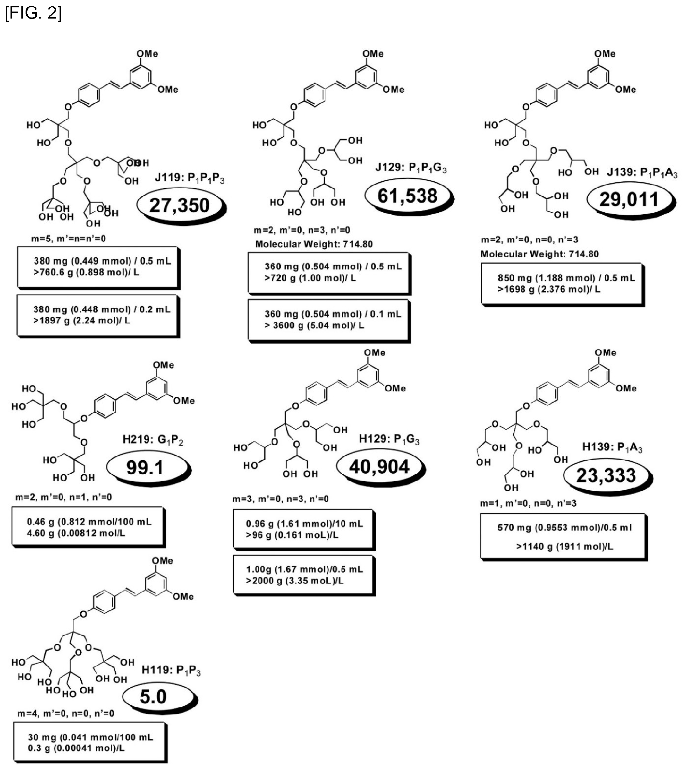

The deprotected pterostilbene linked body obtained in Examples were evaluated for water solubility using the following method. The results are summarized in FIGs 1 and 2. In FIGs 1 and 2, the numbers in the ellipses for each compound represent a level of water solubility as a molar ratio, with the water solubility of F1 (pterostilbene) (21 mg (0.0819 mmol) dissolved in 1 liter) being set at 1.0. The water solubility of F1 (pterostilbene) was determined using the value described in Sarah J. Bethune et al., Cryst. Growth Des. 2011, 11, 2817-2823. The water solubility of F1 glycoside (pterostilbene monoglycoside) was determined using the value described in Jose L. Gonzalez-Alfonso et al., Molecules 2018, 23, 1271.

### [Compounds G119, G129, G139, H119 and H219]

Into a round-bottom flask was weighed 1.0 g of the compound and 100 mL of ion-exchanged water in sequence and the mixture was heated with a heat gun for about 1 minute, taking care not to boil the mixture, and then cooled to room temperature and allowed to stand. After 24 hours, the residue was removed by filtration, and the filtrate was concentrated under reduced pressure. A water solubility of each compound was calculated from the weight of the compound recovered from the filtrate.

### [Compounds H129, H139, J119, J129, J139, J219, J229 and J239]

In a round-bottom flask, a solution was prepared the amounts of the compound and ion-exchanged water shown in Figure 1, and a water solubility of the compound was calculated. Because the compound was extremely water-soluble, a water solubility threshold could not be measured. However, compared to the hardly water-soluble F1, its water solubility was 2×10^4 times or more, that is, extremely higher.

## Claims

1. A branched multi-hydroxyl-protected oligomer represented by Formula (1):
[Chemical Formula 1]
X- [Ya] ₘ - [Y^{b}] _{m'} - [Y^{c}] ₙ - [Y^{d}] _{n'} (1)
wherein X is at least one selected from the group consisting of groups represented by Formula (2); Y^{a} to Y^{d} are at least one selected from the group consisting of P, T, G, and A represented by Formula (3); m is 1; m' is an integer of 1 to 3; n is an integer of 0 to 9; and n' is an integer of 0 to 27, except when the compound represented by Formula (1) is X-G₁G₁, X-G₁G₂, X-G₁A₁, or X-G₁A₂; 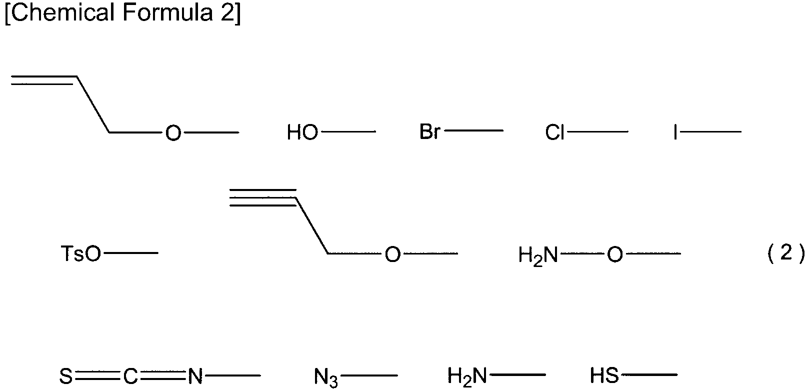 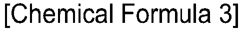 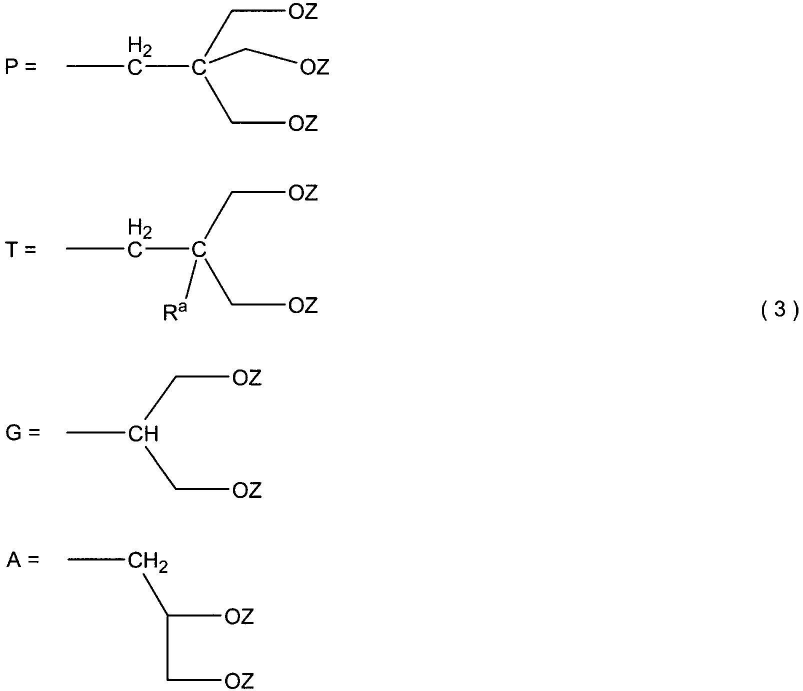
wherein Z is at least one selected from the group consisting of a hydrogen atom, a protecting group and a carbon skeleton, and R^{a} is at least one selected from the group consisting of a hydrogen atom and an alkyl group.

2. A branched multi-hydroxyl-protected oligomer represented by Formula (1a): 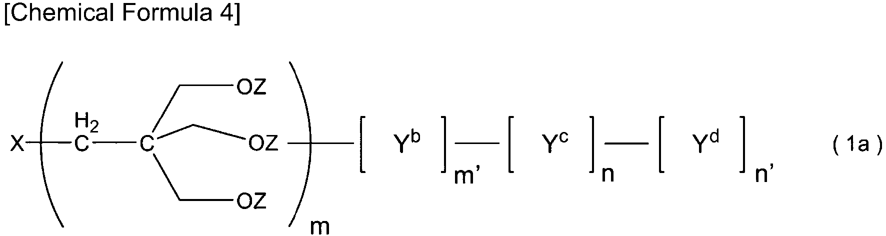
wherein X is at least one selected from the group consisting of groups represented by Formula (2); Y^{b} to Y^{d} are at least one selected from the group consisting of P, T, G, and A represented by Formula (3); Z is at least one selected from the group consisting of a hydrogen atom, a protecting group and a carbon skeleton; m is 1; m' is an integer of 1 to 3; n is an integer of 0 to 9; n' is an integer of 0 to 27; 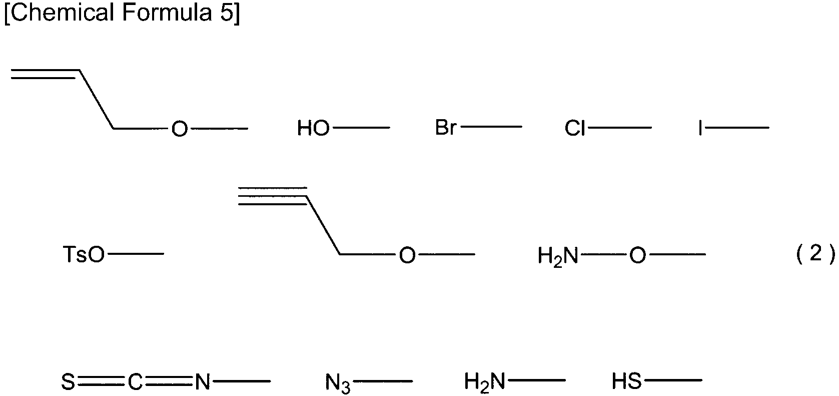 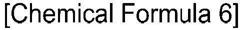 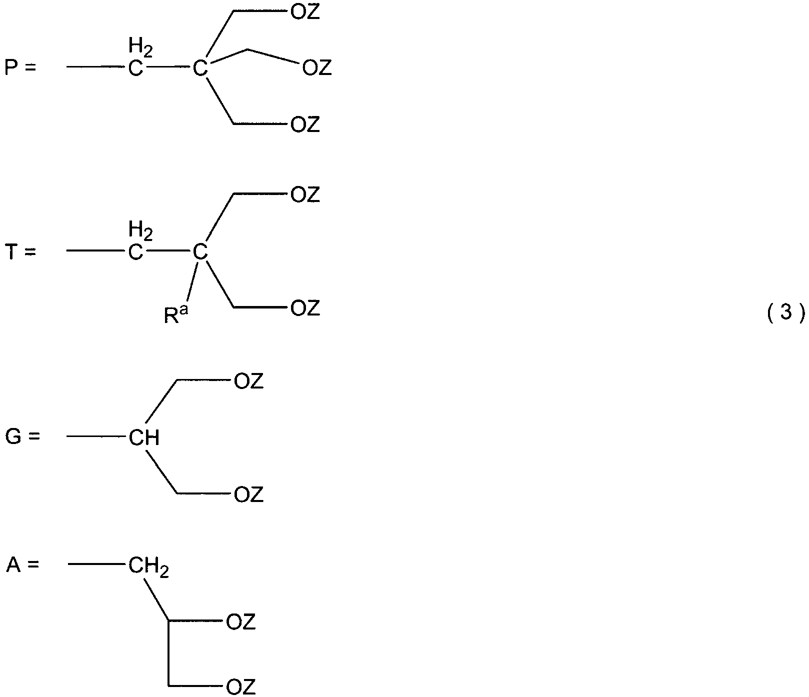
wherein Z is at least one selected from the group consisting of a hydrogen atom, a protecting group and a carbon skeleton; and R^{a} is at least one selected from the group consisting of a hydrogen atom and an alkyl group.

3. A branched multi-hydroxyl-protected oligomer represented by Formula (1c): 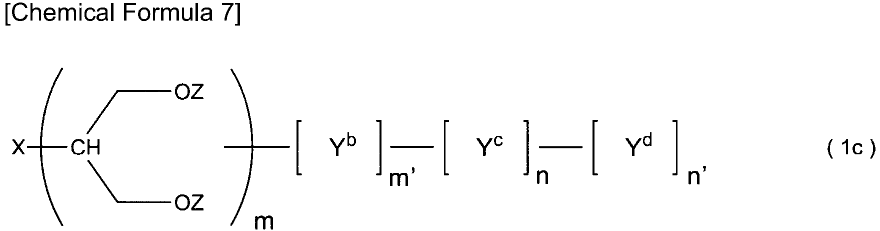
wherein X is at least one selected from the group consisting of groups represented by Formula (2); Y^{b} to Y^{d} are at least one selected from the group consisting of P, T, G, and A represented by Formula (3); Z is at least one selected from the group consisting of a hydrogen atom, a protecting group and a carbon skeleton; m is 1; m' is an integer of 1 to 3; n is an integer of 0 to 9; n' is an integer of 0 to 27, except when the compound represented by Formula (1c) is X-G₁G₁, X-G₁G₂, X-G₁A₁ or X-G₁A₂; 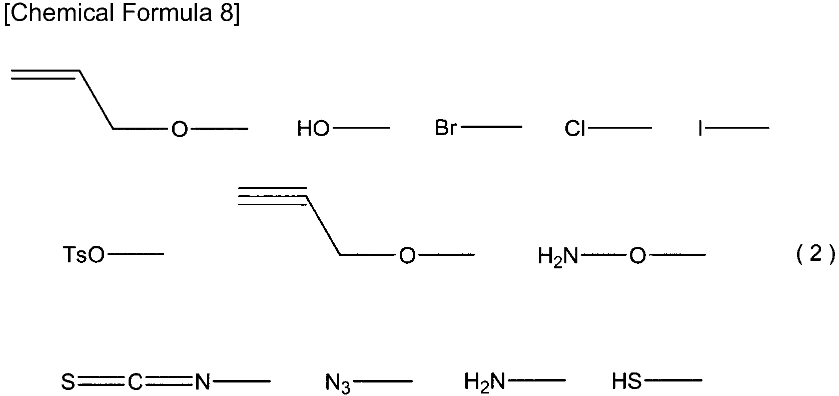 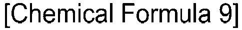 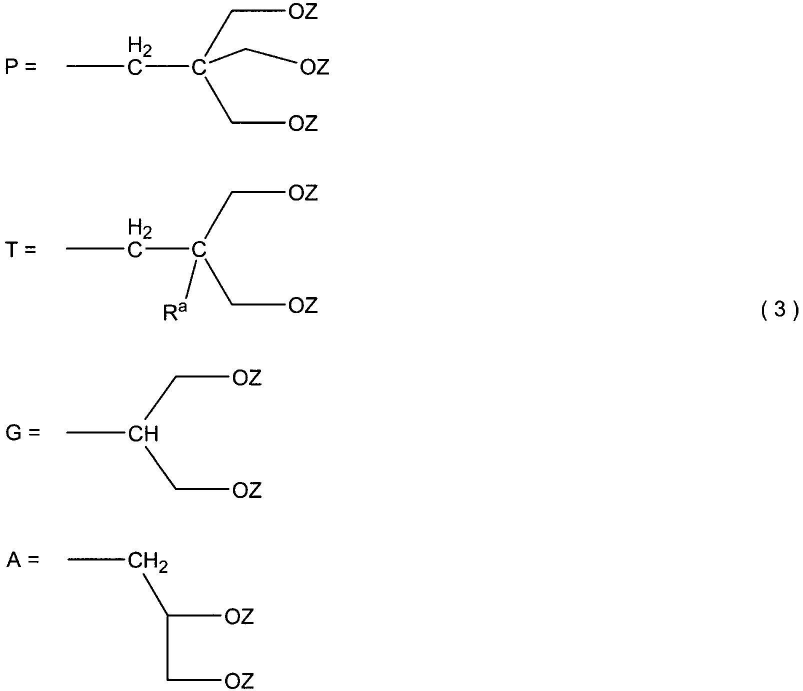
wherein Z is at least one selected from the group consisting of a hydrogen atom, a protecting group and a carbon skeleton; and R^{a} is at least one selected from the group consisting of a hydrogen atom and an alkyl group.

4. A linked body made by bonding the branched multi-hydroxyl-protected oligomer according to any one of Claims 1 to 3 with a target compound.

5. A linked body made by deprotecting the branched multi-hydroxyl-protected oligomer according to Claim 4.

6. A linked body wherein the target compound according to Claim 4 is a hardly water-soluble compound.

7. A water-solubility improver made of the branched multi-hydroxyl-protected oligomer according to any one of Claims 1 to 3.
